# EUROPEAN PATENT SPECIFICATION

(11) **EP 4 142 497 B1**
(45) Date of publication and mention of the grant of the patent: **09.07.2025**
(21) Application number: 21718577.6
(22) Date of filing: 15.04.2021
(51) Int. Cl.: A01N 37/36, A01P 3/00, A01N 37/50, C07C 251/60, C07C 255/64, C07D 205/04, C07D 261/08, C07D 271/06, C07D 277/28, C07D 305/06

(54) **USE OF STROBILURIN TYPE COMPOUNDS FOR COMBATING PHYTOPATHOGENIC FUNGI CONTAINING AN AMINO ACID SUBSTITUTION F129L IN THE MITOCHONDRIAL CYTOCHROME B PROTEIN CONFERRING RESISTANCE TO QO INHIBITORS (I)**
VERWENDUNG VON VERBINDUNGEN VOM STROBILURINTYP ZUR BEKÄMPFUNG VON PHYTOPATHOGENEN PILZEN, DIE EINE AMINOSÄURESUBSTITUTION F129L IN DEM MITOCHONDRIALEN CYTOCHROM-B-PROTEIN ENTHALTEN, DAS RESISTENZ GEGENÜBER QO-INHIBITOREN VERLEIHT (I)
UTILISATION DE COMPOSÉS DE TYPE STROBILURINE POUR LUTTER CONTRE LES CHAMPIGNONS PHYTOPATHOGÈNES CONTENANT UNE SUBSTITUTION D'ACIDE AMINÉ F129L DANS LA PROTÉINE MITOCHONDRIALE DU CYTOCHROME B CONFÉRANT UNE RÉSISTANCE AUX INHIBITEURS DE QO (I)

(30) Priority: 28.04.2020 EP 20171942; 26.03.2021 EP 21165157
(43) Date of publication of application: 08.03.2023
(73) Proprietor: BASF SE, 67056 Ludwigshafen am Rhein (DE)
(72) Inventor: KOCH, Andreas, 67117 Limburgerhof (DE); FEHR, Marcus, 67117 Limburgerhof (DE); TEGGE, Vanessa, 67117 Limburgerhof (DE); DEY, Chandan, Mumbai 400705 (IN); POONOTH, Manojkumar, Mumbai 400705 (IN); KULKARNI, Sarang, Mumbai 400705 (IN); LE VEZOUET, Ronan, 67056 Ludwigshafen (DE); WINTER, Christian Harald, 67056 Ludwigshafen (DE); RUDOLF, Georg Christoph, 67056 Ludwigshafen (DE); RATH, Rakesh, Mumbai 400705 (IN); KHANNA, Smriti, Mumbai 400705 (IN); CRAIG, Ian Robert, 67056 Ludwigshafen (DE); GRAMMENOS, Wassilios, 67056 Ludwigshafen (DE); GROTE, Thomas, 67157 Wachenheim (DE); STAMMLER, Gerd, 67117 Limburgerhof (DE); MENTZEL, Tobias, 67117 Limburgerhof (DE); HADEN, Egon, 67346 Speyer (DE); RHEINHEIMER, Joachim, 67063 Ludwigshafen (DE)
(74) Representative: BASF IP Association
(86) International application number: PCT/EP2021/059727
(87) International publication number: WO 2021/219386

(56) References cited:
- WO-A1-2017/157923
- WO-A1-98/23156
- WO-A2-02/081742
- JP-A- H0 625 133
- J. H. LEIMINGER ET AL: "Occurrence of the F129L mutation in Alternaria solani populations in Germany in response to QoI application, and its effect on sensitivity", PLANT PATHOLOGY, vol. 63, no. 3, 1 June 2014 (2014-06-01), GB, pages 640 - 650, XP055720328, ISSN: 0032-0862, DOI: 10.1111/ppa.12120
- ANA C KLOSOWSKI ET AL: "Detection of the F129L mutation in the cytochrome b gene in Phakopsora pachyrhizi : F129L mutation in the CYTB gene in P. pachyrhizi", PEST MANAGEMENT SCIENCE, vol. 72, no. 6, 1 June 2016 (2016-06-01), pages 1211 - 1215, XP055720325, ISSN: 1526-498X, DOI: 10.1002/ps.4099

## Description

The present invention relates the non-therapeutic use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors (Qol), and to non-therapeutic methods for combating such fungi. The invention also relates to novel compounds, processes for preparing these compounds, to compositions comprising at least one such compound, to plant health applications, and to seeds coated with at least one such compound. The present invention also relates to a non-therapeutic method for controlling soybean rust fungi (*Phakopsora pachyrhizi*) with the amino acid substitution F129L in the mitochondrial cytochrome b protein.

"Qo inhibitor," as used herein, includes any substance that is capable of diminishing and/or inhibiting respiration by binding to a ubihydroquinone oxidation center of a cytochrome bc₁ complex in mitochondria. The oxidation center is typically located on the outer side of the inner mitochrondrial membrane. Many of these compounds are also known as strobilurin-type or strobilurin analogue compounds.

The mutation F129L in the mitochondrial cytochrome b (CYTB) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the CYTB (cytochrome b) gene resulting in a single amino acid substitution in the position 129 from F to L in the cytochrome b protein. Such F129L mutation is known to confer resistance to Qo inhibitors.

Qol fungicides, often referred to as strobilurin-type fungicides (Sauter 2007: Chapter 13.2. Strobilurins and other complex Ill inhibitors. In: Krämer, W.; Schirmer, U. (Ed.) - Modern Crop Protection Compounds. Volume 2. Wiley-VCH Verlag 457-495), are conventionally used to control a number of fungal pathogens in crops. Qo inhibitors typically work by inhibiting respiration by binding to a ubihydroquinone oxidation center of a cytochrome bc₁ complex (electron transport complex III) in mitochondria. Said oxidation center is located on the outer side of the inner mitochrondrial membrane. A prime example of the use of Qols includes the use of, for example, strobilurins on wheat for the control of *Septoria tritici* (also known as *Mycosphaerella graminicola*), which is the cause of wheat leaf blotch. Unfortunately, widespread use of such Qols has resulted in the selection of mutant pathogens which are resistant to such Qols (Gisi et al., Pest Manag Sci 56, 833-841, (2000)). Resistance to Qols has been detected in several phytopathogenic fungi such as *Blumeria graminis, Mycosphaerella fijiensis, Pseudoperonspora cubensis* or *Venturia inaequalis.* The major part of resistance to Qols in agricultural uses has been attributed to pathogens containing a single amino acid residue substitution G143A in the cytochrome b gene for their cytochrome bc₁ complex, the target protein of Qols which have been found to be controlled by specific Qols (WO 2013/092224). Despite several commercial Qol fungicides have also been widely used in soybean rust control, the single amino acid residue substitution G143A in the cytochrome b protein conferring resistance to Qol fungicides was not observed.

Instead soybean rust acquired a different genetic mutation in the cytochrome b gene causing a single amino acid substitution F129L which also confers resistance against Qol fungicides. The efficacy of Qol fungicides used against soybean rust conventionally, i.e. pyraclostrobin, azoxystrobin, picoxystrobin, orysastrobin, dimoxystrobin and metominostrobin, has decreased to a level with practical problems for agricultural practice (e.g. Klosowski et al (2016) Pest Manag Sci 72, 1211-1215).

Although it seems that trifloxystrobin was less affected by the F129L amino acid substitution to the same degree as other Qol fungicides such as azoxystrobin and pyraclostrobin, trifloxystrobin was never as efficacious on a fungal population bearing the F129L Qol resistance mutation as on a sensitive population (Crop Protection 27, (2008) 427-435).

WO 2017/157923 discloses the use of the tetrazole compound 1-[2-[[1-(4-chlorophenyl)-pyrazol-3-yl]oxymethyl]-3-methylphenyl]-4-methyltetrazol-5-one for combating phytopathogenic fungi containing said F129L amino acid substitution.

Thus, new methods are desirable for controlling pathogen induced diseases in crops comprising plants subjected to pathogens containing a F129L amino acid substitution in the mitochondrial cytochrome b protein conferring resistance to Qo inhibitors. Furthermore, in many cases, in particular at low application rates, the fungicidal activity of the known fungicidal strobilurin compounds is unsatisfactory, especially in case that a high proportion of the fungal pathogens contain a mutation in the mitochondrial cytochrome b gene conferring resistance to Qo inhibitors. Besides there is an ongoing need for new fungicidally active compounds which are more effective, less toxic and/or environmentally safer. Based on this, it was also an object of the present invention to provide compounds having improved activity and/or a broader activity spectrum against phytopathogenic fungi and/or even further reduced toxicity against non target organisms such as vertebrates and invertebrates.

The strobilurin-analogue compounds used to combat phytopathogenic fungi containing a F129L amino acid substitution in the mitochondrial cytochrome b protein conferring resistance to Qo inhibitors according to the present invention differ from trifloxystrobin inter alia by containing a specific group attached to the central phenyl ring in ortho position to the side chain defined herein as R³.

Any uses and methods according to the invention referred to in any part of the following text are to be understood as being of a non-therapeutic nature.

Accordingly, the present invention relates to the use of compounds of formula I 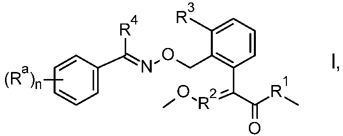 wherein
- R¹: is selected from O and NH;
- R²: is selected from CH and N;
- R³: is selected from halogen, C₁-C₄-alkyl, C₂-C₄-alkenyl, C₁-C₂-monohaloalkyl, C₁-C₂-dihaloalkyl, monohalo-ethenyl, dihalo-ethenyl, C₃-C₆-cycloalkyl and -O-C₁-C₄-alkyl;
- R⁴: is selected from C₁-C₄-alkyl, C₂-C₄-alkenyl, -C(=O)-C₁-C₂-alkyl, C₁-C₄-haloalkyl, C₂-C₄-haloalkenyl, -(C₁-C₂-alkyl)-O-(C₁-C₂-alkyl) and -CH₂-cyclopropyl ;
- R^{a}: is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via an oxygen atom or via a C₁-C₂-alkylene linker,
and wherein the aliphatic and cyclic moieties of R^{a} are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl and -O-C₁-C₄-haloalkyl;
   R⁵, R⁶ are independently of each other selected from the group consisting of H, C₁-C₆-alkyl, C₁-C₆-haloalkyl and C₂-C₄-alkynyl;
- n: is an integer selected from 0, 1, 2, 3, 4 and 5;
and in form or stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust (*Phakopsora pachyrhizi* and *Phakopsora meibromiae*) as well as fungi from the genera Alternaria, Pyrenophora and Rhizoctonia.

Preferred fungal species are *Alternaria solani, Phakopsora pachyrhizi, Phakopsora meibromiae, Pyrenophora teres, Pyrenophora tritici-repentis* and *Rhizoctonia solani;* in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain a such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

Although the present invention will be described with respect to particular embodiments, this description is not to be construed in a limiting sense. The present invention is limited by the claims.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds I.2" refers to compounds of formula I.2 or "compounds V" refers to compounds of formula V, etc..

The term "independently" when used in the context of selection of substituents for a variable, it means that where more than one substituent is selected from a number of possible substituents, those substituents may be the same or different.
The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₄-alkyl" refers to a straight-chained or branched saturated hydrocarbon group having 1 to 4 carbon atoms, for example, methyl (CH₃), ethyl (C₂H₅), propyl, 1-methylethyl (isopropyl), butyl, 1-methylpropyl, 2-methylpropyl, 1,1-dimethylethyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-alkynyl" refers to a straight-chain or branched unsaturated hydrocarbon radical having 2 to 4 carbon atoms and containing at least one triple bond such as ethynyl, prop-1-ynyl, prop-2-ynyl, but-1-ynyl, but-2-ynyl, but-3-ynyl, 1-methyl-prop-2-ynyl.

The term "C₁-C₄-haloalkyl" refers to a straight-chained or branched alkyl group having 1 to 4 carbon atoms wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "monohalo-ethenyl" refers to an ethenyl wherein one hydrogen atom is replaced by a halogen atom, e.g. 1-chloroethenyl, 1-bromoethenyl, 1-fluoroethenyl, 2-fluoroethenyl. Likewise, dihalo-ethenyl" refers to an ethenyl wherein two hydrogen atoms are replaced by halogen atoms.

The term "-O-C₁-C₄-alkyl" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl¬propoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl. The term "C₃-C₆-cycloalkenyl " refers to monocyclic saturated hydrocarbon radicals having 3 to 6 carbon ring members and one or more double bonds.

The term "3- to 6-membered heterocycloalkyl" refers to 3- to 6-membered monocyclic saturated ring system having besides carbon atoms one or more heteroatoms, such as O, N, S as ring members. The term "C₃-C₆-membered heterocycloalkenyl" refers to 3- to 6-membered monocyclic ring system having besides carbon atoms one or more heteroatoms, such as O, N and S as ring members, and one or more double bonds.

The term "-C₁-C₄-alkyl-C₃-C₆-cycloalkyl" refers to alkyl having 1 to 4 carbon atoms (as defined above), wherein one hydrogen atom of the alkyl radical is replaced by a cycloalkyl radical having 3 to 6 carbon atoms.

The term "phenyl" refers to C₆H₅.

The term "5- or 6-membered heteroaryl" which contains 1, 2, 3 or 4 heteroatoms from the group consisting of O, N and S, is to be understood as meaning aromatic heterocycles having 5 or 6 ring atoms. Examples include:
- 5-membered heteroaryl which in addition to carbon atoms, e.g. contain 1, 2 or 3 N atoms and/or one sulfur and/or one oxygen atom: for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which, in addition to carbon atoms, e.g. contain 1, 2, 3 or 4 N atoms as ring members, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The term "C₁-C₂-alkylene linker" means a divalent alkyl group such as -CH₂- or -CH₂-CH₂-that is bound at one end to the core structure of formula I and at the other end to the particular substituent.

As used herein, the "compounds", in particular "compounds I" include all the stereoisomeric and tautomeric forms and mixtures thereof in all ratios, prodrugs, isotopic forms, their agriculturally acceptable salts, N-oxides and S-oxides thereof.

The term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers). The term "tautomer" refers to the coexistence of two (or more) compounds that differ from each other only in the position of one (or more) mobile atoms and in electron distribution, for example, keto-enol tautomers. The term "agriculturally acceptable salts" as used herein, includes salts of the active compounds which are prepared with acids or bases, depending on the particular substituents found on the compounds described herein. "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

In respect of the variables, the embodiments of the intermediates correspond to the embodiments of the compounds I.

Preference is given to those compounds I and where applicable also to compounds of all sub-formulae provided herein, e. g. formulae I.1 and I.2, and to the intermediates such as compounds II, III, IV and V, wherein the substituents and variables (such as n, R¹, R², R³, R⁴, R⁵, R⁶, R^{a}, and R^{b}) have independently of each other or more preferably in combination (any possible combination of 2 or more substituents as defined herein) the following meanings:
Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:
One embodiment of the invention relates to the abovementioned use and or method of application (herein collectively referred to as "use") of compounds I, wherein R¹ is selected from O and NH; and R² is selected from CH and N, provided that R² is N in case R¹ is NH. More preferably R¹ is NH. In particular, R¹ is NH and R² is N. Another embodiment relates to the use of compounds I, wherein R¹ is O and R² is CH.

According to another embodiment, R³ is selected from halogen, C₁-C₄-alkyl, C₂-C₄-alkenyl, C₁-C₂-monohaloalkyl, C₁-C₂-dihaloalkyl, monohalo-ethenyl, dihalo-ethenyl, C₃-C₅-cycloalkyl and -O-C₁-C₄-alkyl; preferably from halogen, C₁-C₂-alkyl, C₁-C₂-monohaloalkyl, C₁-C₂-dihaloalkyl, C₃-C₄-cycloalkyl and -O-C₁-C₂-alkyl; more preferably from C₁-C₂-alkyl, C₁-C₂-monohaloalkyl, C₁-C₂-dihaloalkyl, C₃-C₄-cycloalkyl and -O-C₁-C₂-alkyl; even more preferably from halogen, C₁-C₂-alkyl, C₂-C₃-alkenyl, CHF₂, CFH₂, -O-C₁-C₂-alkyl and cyclopropyl; even more preferably from C₁-C₂-alkyl, ethenyl, CHF₂, CFH₂, OCH₃ and cyclopropyl; particularly preferred from methyl, ethenyl, CHF₂ and CFH₂; in particular methyl.

R⁴ is selected from C₁-C₄-alkyl, C₂-C₄-alkenyl, -C(=O)-C₁-C₂-alkyl, C₁-C₄-haloalkyl, C₂-C₄-haloalkenyl, -(C₁-C₂-alkyl)-O-(C₁-C₂-alkyl) and -CH₂-cyclopropyl; more preferably from C₁-C₄-alkyl and C₁-C₄-haloalkyl, particularly preferably from methyl and C₁-haloalkyl; in particular methyl.

According to a further embodiment, n is 1, 2, 3, 4 or 5; more preferably n is 1, 2 or 3, even more preferably n is 1 or 2; in particular n is 1.

According to a further embodiment, n is 0, 1, 2 or 3, more preferably 0, 1 or 2, in particular 0.

According to a further embodiment, n is 2 and the two substituents R^{a} are preferably in positions 2,3 (meaning one substituent in position 2, the other in position 3); 2,4; 2,5; 3,4 or 3,5; even more preferably in positions 2,3 or 2,4.

According to a further embodiment, n is 3 and the two substituents R^{a} are preferably in positions 2, 3 and 4.

According to a further embodiment, R^{a} is selected from CN, C₁-C₄-alkyl, C₂-C₄-alkenyl, C₂-C₄-alkynyl, -O-C₁-C₄-alkyl, -C(=O)-C₁-C₄-alkyl,-C(=N-O-C₁-C₄-alkyl)-C₁-C₄-alkyl, -O-CH₂-(=N-O-C₁-C₄-alkyl)-C₁-C₄-alkyl, -C(=N-O-C₁-C₄-alkyl)-C(=O-NH-C₁-C₄-alkyl), C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 5-membered heterocycloalkyl, 3- to 5-membered heterocycloalkenyl and 5- or 6-membered heteroaryl, wherein said heterocycloalkyl, hetercycloalkenyl and heteroaryl besides carbon atoms contain 1, 2 or 3 heteroatoms selected from N, O and S, wherein said phenyl, heterocycloalkyl, hetercycloalkenyl and heteroaryl are bound directly or via an oxygen atom or via a C₁-C₂-alkylene linker, and wherein the aliphatic and cyclic moieties of R^{a} are unsubstituted or carry 1, 2, or 3 of identical or different groups R^{b} which independently of one another are selected from halogen, CN, NH₂, NO₂, C₁-C₂-alkyl and C₁-C₂-haloalkyl.

More preferably, R^{a} is selected from C₁-C₄-alkyl, C₂-C₄-alkenyl, C₂-C₄-alkynyl, -O-C₁-C₄-alkyl, -C(=N-O-C₁-C₂-alkyl)-C₁-C₂-alkyl, -O-CH₂-C(=N-O-C₁-C₂-alkyl)-C₁-C₂-alkyl, C₃-C₄-cycloalkyl, -C₁-C₂-alkyl-C₃-C₄-cycloalkyl, phenyl, 3- to 5-membered heterocycloalkyl and 5- or 6-membered heteroaryl, wherein said heterocycloalkyl and heterocycloalkyl and heteroaryl besides carbon atoms contain 1 or 2 heteroatoms selected from N, O and S, wherein said phenyl, heterocycloalkyl and heteroaryl are bound directly or via an oxygen atom or via a methylene linker, and wherein the aliphatic or cyclic moieties of R^{a} are unsubstituted or carry 1, 2, or 3 of identical or different groups R^{b} which independently of one another are selected from halogen, CN, C₁-C₂-alkyl and C₁-C₂-haloalkyl.

Even more preferably R^{a} is selected from C₁-C₃-alkyl, C₂-C₃-alkenyl, C₂-C₃-alkynyl, -O-C₁-C₃-alkyl, -C(=N-O-C₁-C₂-alkyl)-C₁-C₂-alkyl, C₃-C₄-cycloalkyl, -C₁-C₂-alkyl-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, wherein said phenyl and heteroaryl are bound directly or via a methylene linker, and wherein the aliphatic and cyclic moieties of R^{a} are unsubstituted or carry 1, 2 or 3 of identical or different groups R^{b} which independently of one another are selected from halogen, CN, methyl and C₁-haloalkyl.

Particularly preferred R^{a} are selected from halogen, C₁-C₄-alkyl, C₂-C₃-alkenyl, C₂-C₃-alkynyl, -O-C₁-C₄-alkyl, -C(=N-O-C₁-C₂-alkyl)-C₁-C₂-alkyl and phenyl, wherein the aliphatic or cyclic moieties of R^{a} are unsubstituted or carry 1, 2 or 3 of identical or different groups R^{b} which independently of one another are selected from halogen, CN, methyl and C₁-haloalkyl.

According to a further embodiment, R⁵, R⁶ are independently of each other preferably selected from the group consisting of H, C₁-C₄-alkyl, C₁-C₄-haloalkyl and C₂-C₄-alkynyl, more preferably from H and C₁-C₄-alkyl.

According to a further preferred embodiment, the present invention relates to the use of compounds of formula I wherein:
- R¹: is selected from O and NH; and
- R²: is selected from CH and N, provided that R² is N in case R¹ is NH;
- R³: is selected from halogen, C₁-C₄-alkyl, C₂-C₄-alkenyl, C₁-C₂-monohaloalkyl, C₁-C₂-dihaloalkyl, C₃-C₄-cycloalkyl and -O-C₁-C₄-alkyl;
- R⁴: is selected from C₁-C₄-alkyl, C₁-C₄-haloalkyl, -C(=O)-C₁-C₄-alkyl, -(C₁-C₂-alkyl)-O-(C₁-C₂-alkyl) and -CH₂-cyclopropyl;
- R^{a}: is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via an oxygen atom or via a C₁-C₂-alkylene linker,
and wherein the aliphatic and cyclic moieties of R^{a} are unsubstituted or carry 1, 2, 3, 4 or up to the maximum number of identical or different groups R^{b}:
   R^{b} is selected from halogen, CN, NH₂, NO₂, C₁-C₄-alkyl, C₁-C₄-haloalkyl, -O-C₁-C₄-alkyl and -O-C₁-C₄-haloalkyl;
   R⁵, R⁶ are independently of each other selected from the group consisting of H, C₁-C₆-alkyl and C₂-C₄-alkynyl;
- n: is an integer selected from 0, 1, 2 and 3;
and in form or stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

Certain strobilurin type compounds of formula I have been described in EP 370629 and WO 98/23156. However, it is not mentioned that these compounds inhibit fungal pathogens containing a F129L substitution in the mitochondrial cytochrome b protein conferring resistance to Qo inhibitors.

The compounds according to the present invention differ from those described in the abovementioned publications that R³ is an aliphatic or cyclic substituent and R^{a} is a non-halogenated substituent.

Therefore, according to a second aspect, the invention provides novel compounds of formula I which are represented by formula I 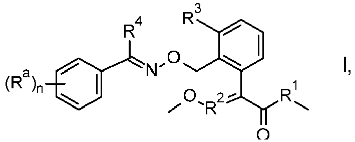 wherein
- R¹: is selected from O and NH;
- R²: is selected from CH and N;
- R³: is selected from C₁-C₄-alkyl, C₂-C₄-alkenyl, C₁-C₂-monohaloalkyl, C₁-C₂-dihaloalkyl, monohalo-ethenyl, dihalo-ethenyl, C₃-C₆-cycloalkyl and -O-C₁-C₄-alkyl;
- R⁴: is selected from C₁-C₄-alkyl, C₂-C₄-alkenyl, C₁-C₄-haloalkyl, C₂-C₄-haloalkenyl, -(C₁-C₂-alkyl)-O-(C₁-C₂-alkyl) and -(C₁-C₂-alkyl)-O-(C₁-C₂-haloalkyl);
- R^{a}: is selected from CN, NH-C₁-C₄-alkyl, N(C₁-C₄-alkyl)₂, C₁-C₄-alkyl, C₂-C₄-alkenyl, C₂-C₄-alkynyl, -O-C₁-C₄-alkyl, -C(=O)-C₁-C₄-alkyl,-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via an oxygen atom or via a C₁-C₂-alkylene linker,
and wherein the aliphatic and cyclic moieties of R^{a} are unsubstituted or carry 1, 2, 3, 4 or up to the maximum number of identical or different groups R^{b}:
   R^{b} is selected from CN, NH₂, NO₂, C₁-C₄-alkyl and -O-C₄-C₄-alkyl;
- n: is an integer selected from 0, 1, 2, 3, 4 and 5;
and in form or stereoisomers and tautomers thereof, and the N-oxides and the agriculturally acceptable salts thereof.

One embodiment of the invention relates to preferred compounds I, wherein R¹ is selected from O and NH; and R² is selected from CH and N, provided that R² is N in case R¹ is NH. More preferably R¹ is NH. In particular, R¹ is NH and R² is N. Another embodiment relates to compounds I, wherein R¹ is O and R² is CH.

According to another embodiment, R³ is selected from C₁-C₄-alkyl, C₂-C₃-alkenyl, C₁-C₂-monohaloalkyl, C₁-C₂-dihaloalkyl, monohalo-ethenyl, dihalo-ethenyl, C₃-C₆-cycloalkyl and -O-C₁-C₄-alkyl; preferably from C₁-C₂-alkyl, C₁-C₂-monohaloalkyl, C₁-C₂-dihaloalkyl, C₃-C₄-cycloalkyl and -O-C₁-C₂-alkyl; preferably selected from C₁-C₄-alkyl, C₂-C₃-alkenyl, monohalomethyl, dihalo-methyl, C₃-C₄-cycloalkyl and -O-C₁-C₄-alkyl; further more preferably selected from C₁-C₂-alkyl, CHF₂, CFH₂, cyclopropyl and OCH₃; particularly preferred from methyl, CHF₂ and CFH₂; in particular R³ is methyl.

According to a further embodiment, R⁴ is selected from C₁-C₄-alkyl, C₂-C₄-alkenyl, C₁-C₄-haloalkyl, C₂-C₄-haloalkenyl and -(C₁-C₂-alkyl)-O-(C₁-C₂-alkyl); more preferably from C₁-C₄-alkyl, and C₁-C₄-haloalkyl, even more preferably from methyl and C₁-haloalkyl; in particular methyl.

According to a further embodiment, n is 1, 2, 3, 4 or 5; more preferably n is 1, 2 or 3, even more preferably n is 1 or 2; in particular n is 1.

According to a further embodiment, n is 0, 1, 2 or 3, more preferably 0, 1 or 2, in particular 0.

According to a further embodiment, n is 2 and the two substituents R^{a} are preferably in positions 2,3 (meaning one substituent in position 2, the other in position 3); 2,4; 2,5; 3,4 or 3,5; even more preferably in positions 2,3 or 2,4.

According to a further embodiment, n is 3 and the two substituents R^{a} are preferably in positions 2, 3 and 4.

According to a further embodiment, R^{a} is selected from CN, NH-C₁-C₄-alkyl, N(C₁-C₄-alkyl)₂, C₁-C₄-alkyl, C₂-C₄-alkenyl, C₂-C₄-alkynyl, -O-C₁-C₄-alkyl, -C(=O)-C₁-C₄-alkyl, -C=(N-O-C₁-C₂-alkyl)-C₁-C₂-alkyl, C₃-C₄-cycloalkyl, -O-C₃-C₄-cycloalkyl, phenyl, 3- to 5-membered heterocycloalkyl, 3- to 5-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S, wherein said phenyl, heterocycloalkyl, heterocycloalkenyl and heteroaryl are bound directly or via an oxygen atom or via a C₁-C₂-alkylene linker.

Preferably, R^{a} is selected from CN, NH-C₁-C₂-alkyl, N(C₁-C₂-alkyl)₂, C₁-C₄-alkyl, C₂-C₄-alkenyl, C₂-C₄-alkynyl, -O-C₁-C₄-alkyl, -C(=O)-C₁-C₂-alkyl, -C=(N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, wherein said phenyl, heterocycloalkyl and heteroaryl are bound directly or via an oxygen atom or via a methylene linker.

More preferably, R^{a} is selected from CN, C₁-C₃-alkyl, -O-C₁-C₃-alkyl, -C=(N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, wherein said phenyl, heterocycloalkyl and heteroaryl are bound directly or via an oxygen atom or via a methylene linker.

In particular, R^{a} is selected from CN, C₁-C₂-alkyl, ethenyl, ethynyl, -O-C₁-C₂-alkyl and -C=(N-O-CH₃)-CH₃.

According to the abovementioned embodiments for R^{a}, the abovementioned heterocycloalkyl is more preferably a 4-membered heterocycloalkyl, wherein said heterocycloalkyl besides carbon atoms contains 1 heteroatom selected from N, O and S, preferably N.

According to the abovementioned embodiments for R^{a}, the abovementioned heteroaryl is more preferably a 5-membered heteroaryl, wherein said heteroaryl besides carbon atoms contains 1 or 2 heteroatoms selected from N, O and S, preferably from N and O.

According to the abovementioned embodiments for R^{a}, the aliphatic and cyclic moieties of R^{a} are unsubstituted or carry 1, 2, 3, 4 or up to the maximum number of identical or different groups R^{b} selected from CN, NH₂, NO₂, C₁-C₄-alkyl and -O-C₁-C₄-alkyl; more preferably only the cyclic moieties of R^{a} are unsubstituted or carry 1, 2, 3, 4 or up to the maximum number of identical or different groups R^{b} selected from CN, NH₂, NO₂, C₁-C₄-alkyl and -O-C₁-C₄-alkyl; even more preferably only the phenyl moiety of R^{a} is unsubstituted or carries 1, 2, 3, 4 or 5 identical or different groups R^{b} selected from CN, NH₂, NO₂, C₁-C₄-alkyl and -O-C₁-C₄-alkyl; in particular said phenyl is unsubstituted or carries 1, 2 or 3 identical or different groups R^{b} selected from CN, NH₂, NO₂, C₁-C₄-alkyl and -O-C₁-C₄-alkyl.

According to a further preferred embodiment, the present invention relates to compounds of formula I wherein:
- R¹: is selected from O and NH; and
- R²: is selected from CH and N, provided that R² is N in case R¹ is NH;
- R³: is selected from C₁-C₄-alkyl, C₂-C₄-alkenyl, C₁-C₂-monohaloalkyl, C₁-C₂-dihaloalkyl, monohalo-ethenyl, dihalo-ethenyl, C₃-C₄-cycloalkyl and -O-C₁-C₄-alkyl;
- R⁴: is selected from C₁-C₄-alkyl, C₁-C₄-haloalkyl, and -(C₁-C₂-alkyl)-O-(C₁-C₂-alkyl);
- R^{a}: is selected from C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -O-CH₂-C(=N-O-C₁-C₄-alkyl)-C₁-C₄-alkyl, C₃-C₆-cycloalkyl, -C₁-C₂-alkyl-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via a C₁-C₂-alkylene linker,
and wherein said phenyl is unsubstituted or carries 1,2 or 3 identical or different groups R^{b}:
   R^{b} is selected from CN, NH₂, NO₂, C₁-C₄-alkyl, and -O-C₄-C₄-alkyl;
- n: is an integer selected from 0, 1, 2 and 3;
and in form or stereoisomers and tautomers thereof, and the N-oxides and the agriculturally acceptable salts thereof.

According to a further embodiment, R¹ is O and R² is N, which compounds are of formula I.1:

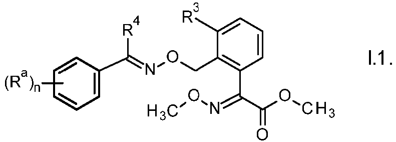

According to a further embodiment, R¹ is O and R² is CH, which compounds are of formula I.2:

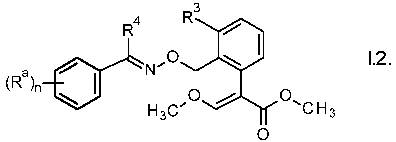

According to a further embodiment, R¹ is NH and R² is N, which compounds are of formula I.3:

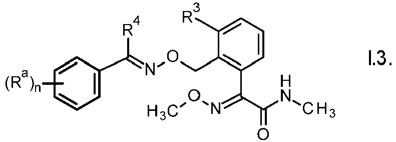

Preferably, R³ of compounds I is one of the following radicals 3-1 to 3-5:

| **No.** | **R³** |
|---|---|
| 3-1 | CH₃ |
| 3-2 | OCH₃ |

| **No.** | **R³** |
|---|---|
| 3-3 | CHF₂ |
| 3-4 | C₃H₅ |

| **No.** | **R³** |
|---|---|
| 3-5 | CH=CH₂ |

Even more preferably R³ is CH₃, OCH₃, CHF₂ or C₃H₅, in particular CH₃.

Particularly preferred embodiments of the invention relate to compounds I, wherein the R⁴ is one of the following radicals 4-1 to 4-5:

| **No.** | **R⁴** |
|---|---|
| 4-1 | CH₃ |
| 4-2 | C₂H₅ |

| **No.** | **R⁴** |
|---|---|
| 4-3 | CH₂OCH₃ |
| 4-4 | CH₂CF₃ |

| **No.** | **R⁴** |
|---|---|
| 4-5 | CHF₂ |

Particularly preferred embodiments of the invention relate to compounds I, wherein the R^{a} is selected of one of the following radicals a-1 to a-10:

| **No.** | **R^{a}** |
|---|---|
| a-1 | CH₃ |
| a-2 | OCH₃ |
| a-3 | C₂H₅ |
| a-4 | CH=CH₂ |

| **No.** | **R^{a}** |
|---|---|
| a-5 | C₆H₅ |
| a-6 | C≡CH |
| a-7 | C≡CCH₃ |
| a-8 | C₃H₅ |

| **No.** | **R^{a}** |
|---|---|
| a-9 | C(=NOCH₃)CH₃ |
| a-10 | CN |

According to a further embodiment, n is 1. More preferably, R^{a} is in ortho-position (2-R^{a}), which compounds are of formula I.A: 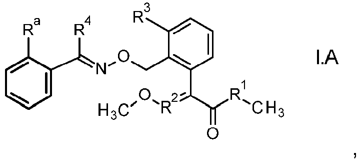 wherein even more preferably R¹ is O and R² is N. According to a further embodiment, R^{a} is in meta-position (3-R^{a}), which compounds are of formula I.B: 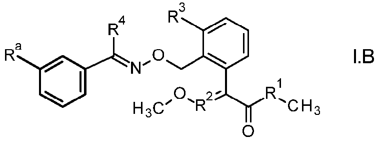 wherein even more preferably R¹ is O and R² is N.

According to a further embodiment, n is 2. More preferably, n is 2 and the two R^{a} substituents are both in meta -position (3,5-R^{a}), which compounds are of formula I.C: 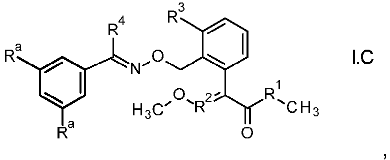 wherein even more preferably R² is N. According to a further embodiment, n is 2 and the two R^{a} substituents are both in ortho-position (2,6-R^{a}), which compounds are of formula I.D: 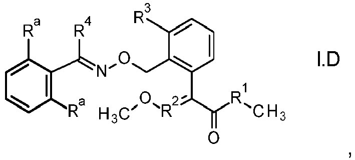 wherein even more preferably R² is N. According to a further embodiment, n is 2 and the two R^{a} substituents are in ortho- and meta-position, which compounds are of formula I.E: 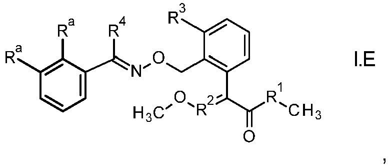 wherein even more preferably R² is N. According to a further embodiment, n is 2 and the two R^{a} substituents are in ortho- and para-position, which compounds are of formula I.F: 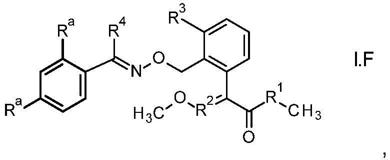 wherein even more preferably R² is N.

In an embodiment, compounds I are of formula I.3 and n, R^{a}, R³ and R⁴ are as per any row of per Table A below, which compounds are named I.3-A-1 to I.3-A-217.
In another embodiment, compounds I are of formula I.2 and n, R^{a}, R³ and R⁴ are as per any row of Table A below, which compounds are named I.2-A-1 to I.2-A-217.
In an embodiment, compounds I are of formula I.1 and n, R^{a}, R³ and R⁴ are as per any row of Table A below, which compounds are named I.1-A-1 to I.1-A-217.

**Table A:**

| **No.** | **n** | **R^{a}** | **R³** | **R⁴** |
|---|---|---|---|---|
| A-1 | 0 | - | CH₃ | CH₃ |
| A-2 | 1 | 2-CH₃ | CH₃ | CH₃ |
| A-3 | 1 | 2-OCH₃ | CH₃ | CH₃ |
| A-4 | 1 | 2-C₂H₅ | CH₃ | CH₃ |
| A-5 | 1 | 2-CH=CH₂ | CH₃ | CH₃ |
| A-6 | 1 | 2-C₆H₅ | CH₃ | CH₃ |
| A-7 | 1 | 2-C≡CH | CH₃ | CH₃ |
| A-8 | 1 | 2-C≡CCH₃ | CH₃ | CH₃ |
| A-9 | 1 | 2-C₃H₅ | CH₃ | CH₃ |
| A-10 | 1 | 2-C(=NOCH₃)CH₃ | CH₃ | CH₃ |
| A-11 | 1 | 2-CN | CH₃ | CH₃ |
| A-12 | 1 | 3-CH₃ | CH₃ | CH₃ |
| A-13 | 1 | 3-OCH₃ | CH₃ | CH₃ |
| A-14 | 1 | 3-C₂H₅ | CH₃ | CH₃ |
| A-15 | 1 | 3-CH=CH₂ | CH₃ | CH₃ |
| A-16 | 1 | 3-C₆H₅ | CH₃ | CH₃ |
| A-17 | 1 | 3-C≡CH | CH₃ | CH₃ |
| A-18 | 1 | 3-C≡CCH₃ | CH₃ | CH₃ |
| A-19 | 1 | 3-C₃H₅ | CH₃ | CH₃ |
| A-20 | 1 | 3-C(=NOCH₃)CH₃ | CH₃ | CH₃ |
| A-21 | 1 | 3-CN | CH₃ | CH₃ |
| A-22 | 1 | 4-CH₃ | CH₃ | CH₃ |
| A-23 | 1 | 4-OCH₃ | CH₃ | CH₃ |
| A-24 | 1 | 4-C₂H₅ | CH₃ | CH₃ |
| A-25 | 1 | 4-CH=CH₂ | CH₃ | CH₃ |
| A-26 | 1 | 4-C₆H₅ | CH₃ | CH₃ |
| A-27 | 1 | 4-C≡CH | CH₃ | CH₃ |
| A-28 | 1 | 4-C≡CCH₃ | CH₃ | CH₃ |
| A-29 | 1 | 4-C₃H₅ | CH₃ | CH₃ |
| A-30 | 1 | 4-C(=NOCH₃)CH₃ | CH₃ | CH₃ |
| A-31 | 1 | 4-CN | CH₃ | CH₃ |
| A-32 | 0 | - | CH₃ | C₂H₅ |
| A-33 | 1 | 2-CH₃ | CH₃ | C₂H₅ |
| A-34 | 1 | 2-OCH₃ | CH₃ | C₂H₅ |
| A-35 | 1 | 2-C₂H₅ | CH₃ | C₂H₅ |
| A-36 | 1 | 2-CH=CH₂ | CH₃ | C₂H₅ |
| A-37 | 1 | 2-C₆H₅ | CH₃ | C₂H₅ |
| A-38 | 1 | 2-C≡CH | CH₃ | C₂H₅ |
| A-39 | 1 | 2-C≡CCH₃ | CH₃ | C₂H₅ |
| A-40 | 1 | 2-C₃H₅ | CH₃ | C₂H₅ |
| A-41 | 1 | 2-C(=NOCH₃)CH₃ | CH₃ | C₂H₅ |
| A-42 | 1 | 2-CN | CH₃ | C₂H₅ |
| A-43 | 1 | 3-CH₃ | CH₃ | C₂H₅ |
| A-44 | 1 | 3-OCH₃ | CH₃ | C₂H₅ |
| A-45 | 1 | 3-C₂H₅ | CH₃ | C₂H₅ |
| A-46 | 1 | 3-CH=CH₂ | CH₃ | C₂H₅ |
| A-47 | 1 | 3-C₆H₅ | CH₃ | C₂H₅ |
| A-48 | 1 | 3-C≡CH | CH₃ | C₂H₅ |
| A-49 | 1 | 3-C≡CCH₃ | CH₃ | C₂H₅ |
| A-50 | 1 | 3-C₃H₅ | CH₃ | C₂H₅ |
| A-51 | 1 | 3-C(=NOCH₃)CH₃ | CH₃ | C₂H₅ |
| A-52 | 1 | 3-CN | CH₃ | C₂H₅ |
| A-53 | 1 | 4-CH₃ | CH₃ | C₂H₅ |
| A-54 | 1 | 4-OCH₃ | CH₃ | C₂H₅ |
| A-55 | 1 | 4-C₂H₅ | CH₃ | C₂H₅ |
| A-56 | 1 | 4-CH=CH₂ | CH₃ | C₂H₅ |
| A-57 | 1 | 4-C₆H₅ | CH₃ | C₂H₅ |
| A-58 | 1 | 4-C≡CH | CH₃ | C₂H₅ |
| A-59 | 1 | 4-C≡CCH₃ | CH₃ | C₂H₅ |
| A-60 | 1 | 4-C₃H₅ | CH₃ | C₂H₅ |
| A-61 | 1 | 4-C(=NOCH₃)CH₃ | CH₃ | C₂H₅ |
| A-62 | 1 | 4-CN | CH₃ | C₂H₅ |
| A-63 | 0 | - | CH₃ | C₂H₅ |
| A-64 | 1 | 2-CH₃ | CH₃ | C₂H₅ |
| A-65 | 1 | 2-OCH₃ | CH₃ | C₂H₅ |
| A-66 | 1 | 2-C₂H₅ | CH₃ | C₂H₅ |
| A-67 | 1 | 2-CH=CH₂ | CH₃ | C₂H₅ |
| A-68 | 1 | 2-C₆H₅ | CH₃ | C₂H₅ |
| A-69 | 1 | 2-C≡CH | CH₃ | C₂H₅ |
| A-70 | 1 | 2-C≡CCH₃ | CH₃ | C₂H₅ |
| A-71 | 1 | 2-C₃H₅ | CH₃ | C₂H₅ |
| A-72 | 1 | 2-C(=NOCH₃)CH₃ | CH₃ | C₂H₅ |
| A-73 | 1 | 2-CN | CH₃ | C₂H₅ |
| A-74 | 1 | 3-CH₃ | CH₃ | C₂H₅ |
| A-75 | 1 | 3-OCH₃ | CH₃ | C₂H₅ |
| A-76 | 1 | 3-C₂H₅ | CH₃ | C₂H₅ |
| A-77 | 1 | 3-CH=CH₂ | CH₃ | C₂H₅ |
| A-78 | 1 | 3-C₆H₅ | CH₃ | C₂H₅ |
| A-79 | 1 | 3-C≡CH | CH₃ | C₂H₅ |
| A-80 | 1 | 3-C≡CCH₃ | CH₃ | C₂H₅ |
| A-81 | 1 | 3-C₃H₅ | CH₃ | C₂H₅ |
| A-82 | 1 | 3-C(=NOCH₃)CH₃ | CH₃ | C₂H₅ |
| A-83 | 1 | 3-CN | CH₃ | C₂H₅ |
| A-84 | 1 | 4-CH₃ | CH₃ | C₂H₅ |
| A-85 | 1 | 4-OCH₃ | CH₃ | C₂H₅ |
| A-86 | 1 | 4-C₂H₅ | CH₃ | C₂H₅ |
| A-87 | 1 | 4-CH=CH₂ | CH₃ | C₂H₅ |
| A-88 | 1 | 4-C₆H₅ | CH₃ | C₂H₅ |
| A-89 | 1 | 4-C≡CH | CH₃ | C₂H₅ |
| A-90 | 1 | 4-C≡CCH₃ | CH₃ | C₂H₅ |
| A-91 | 1 | 4-C₃H₅ | CH₃ | C₂H₅ |
| A-92 | 1 | 4-C(=NOCH₃)CH₃ | CH₃ | C₂H₅ |
| A-93 | 1 | 4-CN | CH₃ | C₂H₅ |
| A-94 | 0 | - | CH₃ | CH₂CF₃ |
| A-95 | 1 | 2-CH₃ | CH₃ | CH₂CF₃ |
| A-96 | 1 | 2-OCH₃ | CH₃ | CH₂CF₃ |
| A-97 | 1 | 2-C₂H₅ | CH₃ | CH₂CF₃ |
| A-98 | 1 | 2-CH=CH₂ | CH₃ | CH₂CF₃ |
| A-99 | 1 | 2-C₆H₅ | CH₃ | CH₂CF₃ |
| A-100 | 1 | 2-C≡CH | CH₃ | CH₂CF₃ |
| A-101 | 1 | 2-C≡CCH₃ | CH₃ | CH₂CF₃ |
| A-102 | 1 | 2-C₃H₅ | CH₃ | CH₂CF₃ |
| A-103 | 1 | 2-C(=NOCH₃)CH₃ | CH₃ | CH₂CF₃ |
| A-104 | 1 | 2-CN | CH₃ | CH₂CF₃ |
| A-105 | 1 | 3-CH₃ | CH₃ | CH₂CF₃ |
| A-106 | 1 | 3-OCH₃ | CH₃ | CH₂CF₃ |
| A-107 | 1 | 3-C₂H₅ | CH₃ | CH₂CF₃ |
| A-108 | 1 | 3-CH=CH₂ | CH₃ | CH₂CF₃ |
| A-109 | 1 | 3-C₆H₅ | CH₃ | CH₂CF₃ |
| A-110 | 1 | 3-C≡CH | CH₃ | CH₂CF₃ |
| A-111 | 1 | 3-C≡CCH₃ | CH₃ | CH₂CF₃ |
| A-112 | 1 | 3-C₃H₅ | CH₃ | CH₂CF₃ |
| A-113 | 1 | 3-C(=NOCH₃)CH₃ | CH₃ | CH₂CF₃ |
| A-114 | 1 | 3-CN | CH₃ | CH₂CF₃ |
| A-115 | 1 | 4-CH₃ | CH₃ | CH₂CF₃ |
| A-116 | 1 | 4-OCH₃ | CH₃ | CH₂CF₃ |
| A-117 | 1 | 4-C₂H₅ | CH₃ | CH₂CF₃ |
| A-118 | 1 | 4-CH=CH₂ | CH₃ | CH₂CF₃ |
| A-119 | 1 | 4-C₆H₅ | CH₃ | CH₂CF₃ |
| A-120 | 1 | 4-C≡CH | CH₃ | CH₂CF₃ |
| A-121 | 1 | 4-C≡CCH₃ | CH₃ | CH₂CF₃ |
| A-122 | 1 | 4-C₃H₅ | CH₃ | CH₂CF₃ |
| A-123 | 1 | 4-C(=NOCH₃)CH₃ | CH₃ | CH₂CF₃ |
| A-124 | 1 | 4-CN | CH₃ | CH₂CF₃ |
| A-125 | 0 | - | CH₃ | CH₂OCH₃ |
| A-126 | 1 | 2-CH₃ | CH₃ | CH₂OCH₃ |
| A-127 | 1 | 2-OCH₃ | CH₃ | CH₂OCH₃ |
| A-128 | 1 | 2-C₂H₅ | CH₃ | CH₂OCH₃ |
| A-129 | 1 | 2-CH=CH₂ | CH₃ | CH₂OCH₃ |
| A-130 | 1 | 2-C₆H₅ | CH₃ | CH₂OCH₃ |
| A-131 | 1 | 2-C≡CH | CH₃ | CH₂OCH₃ |
| A-132 | 1 | 2-C≡CCH₃ | CH₃ | CH₂OCH₃ |
| A-133 | 1 | 2-C₃H₅ | CH₃ | CH₂OCH₃ |
| A-134 | 1 | 2-C(=NOCH₃)CH₃ | CH₃ | CH₂OCH₃ |
| A-135 | 1 | 2-CN | CH₃ | CH₂OCH₃ |
| A-136 | 1 | 3-CH₃ | CH₃ | CH₂OCH₃ |
| A-137 | 1 | 3-OCH₃ | CH₃ | CH₂OCH₃ |
| A-138 | 1 | 3-C₂H₅ | CH₃ | CH₂OCH₃ |
| A-139 | 1 | 3-CH=CH₂ | CH₃ | CH₂OCH₃ |
| A-140 | 1 | 3-C₆H₅ | CH₃ | CH₂OCH₃ |
| A-141 | 1 | 3-C≡CH | CH₃ | CH₂OCH₃ |
| A-142 | 1 | 3-C≡CCH₃ | CH₃ | CH₂OCH₃ |
| A-143 | 1 | 3-C₃H₅ | CH₃ | CH₂OCH₃ |
| A-144 | 1 | 3-C(=NOCH₃)CH₃ | CH₃ | CH₂OCH₃ |
| A-145 | 1 | 3-CN | CH₃ | CH₂OCH₃ |
| A-146 | 1 | 4-CH₃ | CH₃ | CH₂OCH₃ |
| A-147 | 1 | 4-OCH₃ | CH₃ | CH₂OCH₃ |
| A-148 | 1 | 4-C₂H₅ | CH₃ | CH₂OCH₃ |
| A-149 | 1 | 4-CH=CH₂ | CH₃ | CH₂OCH₃ |
| A-150 | 1 | 4-C₆H₅ | CH₃ | CH₂OCH₃ |
| A-151 | 1 | 4-C≡CH | CH₃ | CH₂OCH₃ |
| A-152 | 1 | 4-C≡CCH₃ | CH₃ | CH₂OCH₃ |
| A-153 | 1 | 4-C₃H₅ | CH₃ | CH₂OCH₃ |
| A-154 | 1 | 4-C(=NOCH₃)CH₃ | CH₃ | CH₂OCH₃ |
| A-155 | 1 | 4-CN | CH₃ | CH₂OCH₃ |
| A-156 | 0 | - | CH₃ | CHF₂ |
| A-157 | 1 | 2-CH₃ | CH₃ | CHF₂ |
| A-158 | 1 | 2-OCH₃ | CH₃ | CHF₂ |
| A-159 | 1 | 2-C₂H₅ | CH₃ | CHF₂ |
| A-160 | 1 | 2-CH=CH₂ | CH₃ | CHF₂ |
| A-161 | 1 | 2-C₆H₅ | CH₃ | CHF₂ |
| A-162 | 1 | 2-C≡CH | CH₃ | CHF₂ |
| A-163 | 1 | 2-C≡CCH₃ | CH₃ | CHF₂ |
| A-164 | 1 | 2-C₃H₅ | CH₃ | CHF₂ |
| A-165 | 1 | 2-C(=NOCH₃)CH₃ | CH₃ | CHF₂ |
| A-166 | 1 | 2-CN | CH₃ | CHF₂ |
| A-167 | 1 | 3-CH₃ | CH₃ | CHF₂ |
| A-168 | 1 | 3-OCH₃ | CH₃ | CHF₂ |
| A-169 | 1 | 3-C₂H₅ | CH₃ | CHF₂ |
| A-170 | 1 | 3-CH=CH₂ | CH₃ | CHF₂ |
| A-171 | 1 | 3-C₆H₅ | CH₃ | CHF₂ |
| A-172 | 1 | 3-C≡CH | CH₃ | CHF₂ |
| A-173 | 1 | 3-C≡CCH₃ | CH₃ | CHF₂ |
| A-174 | 1 | 3-C₃H₅ | CH₃ | CHF₂ |
| A-175 | 1 | 3-C(=NOCH₃)CH₃ | CH₃ | CHF₂ |
| A-176 | 1 | 3-CN | CH₃ | CHF₂ |
| A-177 | 1 | 4-CH₃ | CH₃ | CHF₂ |
| A-178 | 1 | 4-OCH₃ | CH₃ | CHF₂ |
| A-179 | 1 | 4-C₂H₅ | CH₃ | CHF₂ |
| A-180 | 1 | 4-CH=CH₂ | CH₃ | CHF₂ |
| A-181 | 1 | 4-C₆H₅ | CH₃ | CHF₂ |
| A-182 | 1 | 4-C≡CH | CH₃ | CHF₂ |
| A-183 | 1 | 4-C≡CCH₃ | CH₃ | CHF₂ |
| A-184 | 1 | 4-C₃H₅ | CH₃ | CHF₂ |
| A-185 | 1 | 4-C(=NOCH₃)CH₃ | CH₃ | CHF₂ |
| A-186 | 1 | 4-CN | CH₃ | CHF₂ |
| A-187 | 0 | - | CH₃ | CH₂C₃H₅ |
| A-188 | 1 | 2-CH₃ | CH₃ | CH₂C₃H₅ |
| A-189 | 1 | 2-OCH₃ | CH₃ | CH₂C₃H₅ |
| A-190 | 1 | 2-C₂H₅ | CH₃ | CH₂C₃H₅ |
| A-191 | 1 | 2-CH=CH₂ | CH₃ | CH₂C₃H₅ |
| A-192 | 1 | 2-C₆H₅ | CH₃ | CH₂C₃H₅ |
| A-193 | 1 | 2-C≡CH | CH₃ | CH₂C₃H₅ |
| A-194 | 1 | 2-C≡CCH₃ | CH₃ | CH₂C₃H₅ |
| A-195 | 1 | 2-C₃H₅ | CH₃ | CH₂C₃H₅ |
| A-196 | 1 | 2-C(=NOCH₃)CH₃ | CH₃ | CH₂C₃H₅ |
| A-197 | 1 | 2-CN | CH₃ | CH₂C₃H₅ |
| A-198 | 1 | 3-CH₃ | CH₃ | CH₂C₃H₅ |
| A-199 | 1 | 3-OCH₃ | CH₃ | CH₂C₃H₅ |
| A-200 | 1 | 3-C₂H₅ | CH₃ | CH₂C₃H₅ |
| A-201 | 1 | 3-CH=CH₂ | CH₃ | CH₂C₃H₅ |
| A-202 | 1 | 3-C₆H₅ | CH₃ | CH₂C₃H₅ |
| A-203 | 1 | 3-C≡CH | CH₃ | CH₂C₃H₅ |
| A-204 | 1 | 3-C≡CCH₃ | CH₃ | CH₂C₃H₅ |
| A-205 | 1 | 3-C₃H₅ | CH₃ | CH₂C₃H₅ |
| A-206 | 1 | 3-C(=NOCH₃)CH₃ | CH₃ | CH₂C₃H₅ |
| A-207 | 1 | 3-CN | CH₃ | CH₂C₃H₅ |
| A-208 | 1 | 4-CH₃ | CH₃ | CH₂C₃H₅ |
| A-209 | 1 | 4-OCH₃ | CH₃ | CH₂C₃H₅ |
| A-210 | 1 | 4-C₂H₅ | CH₃ | CH₂C₃H₅ |
| A-211 | 1 | 4-CH=CH₂ | CH₃ | CH₂C₃H₅ |
| A-212 | 1 | 4-C₆H₅ | CH₃ | CH₂C₃H₅ |
| A-213 | 1 | 4-C≡CH | CH₃ | CH₂C₃H₅ |
| A-214 | 1 | 4-C≡CCH₃ | CH₃ | CH₂C₃H₅ |
| A-215 | 1 | 4-C₃H₅ | CH₃ | CH₂C₃H₅ |
| A-216 | 1 | 4-C(=NOCH₃)CH₃ | CH₃ | CH₂C₃H₅ |
| A-217 | 1 | 4-CN | CH₃ | CH₂C₃H₅ |

### Synthesis

The compounds can be obtained by various routes in analogy to prior art processes known (e.g EP 463488) and, advantageously, by the synthesis shown in the following schemes 1 to 4 and in the experimental part of this application.

A suitable method to prepare compounds I is illustrated in Scheme 1. 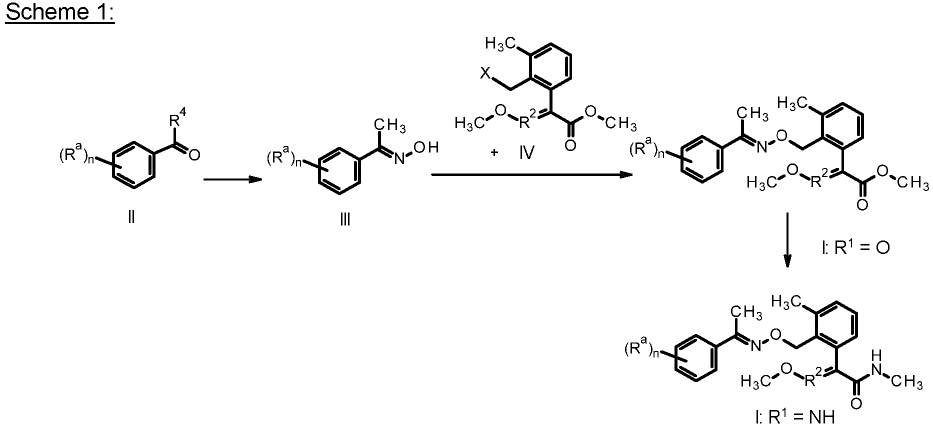 It starts with the conversion of a ketone to the corresponding oxime using hydxroxylamine hydrochloride and a base such as pyridine, sodium hydroxide or sodium acetate in polar solvents such as methanol, methanol-water mixture, or ethanol at reaction temperatures of 60 to 100 °C, preferably at about 65 °C. In cases where a *E*/*Z* mixture was obtained, the isomers could be separated by purifycation techniques known in art (e.g. column chromatography, crystallization, distillation etc.). Then, coupling with the intermediate IV, wherein X is a leaving group such as halogen, toluene- and methanesulfonates, preferably X is Cl or Br, is carried out under basic conditions using e.g. sodium hydride, cesium carbonate or potassium carbonate as a base and using an organic solvent such as dimethyl formamide (DMF) or acetonitrile, preferably cesium carbonate as base and acetonitrile as solvent at room temperature (RT) of about 24 °C . The ester compound I wherein R¹ is O can be converted to the amide of formula I wherein R¹ is NH by reaction with methyl amine (preferably 40% aq. solution) using tetrahydrofuran (THF) as solvent at RT.

Another general method to prepare the compounds I is depicted in Scheme 2. 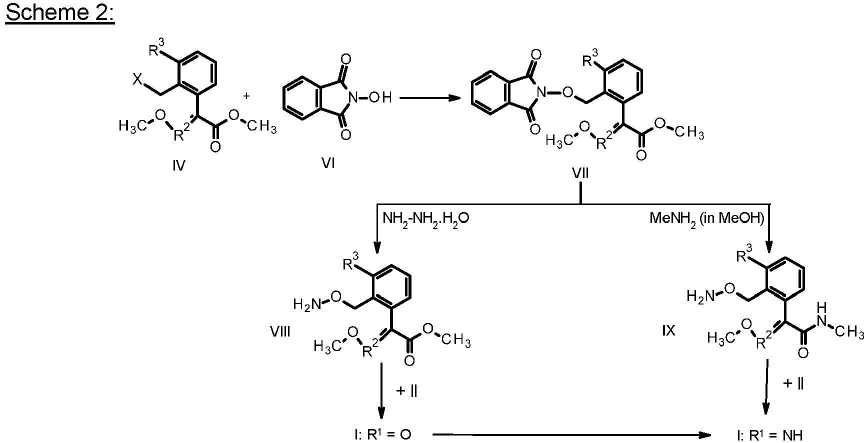 Intermediate IV is reacted with N-hydroxysuccimide VI, using a base such as triethylamine in DMF. The reaction temperature is usually 50 to 70 °C preferably about 70 °C. Conversion to the correspondding O-benzylhydroxyl amine, intermediate VIII, was achieved through removal of the phthalimide group, preferably using hydrazine hydrate in methanol as solvent at 25 °C. Alternatively, removal of the phthalimide group using methyl amine in methanol as solvent at 25 °C can provide intermediate IX. Intermediate VIII and intermediate IX, respectively can be condensed with ketones using acetic acid or pyridine in methanol as solvent at temperature of 50 to 65 °C. Alternatively, the condensation could also carried out with titanium (IV) ethoxide (Ti(OEt)₄) using THF as solvent at about 70 °C. The desired product is usually accompanied by an undesired isomer, which can be removed e.g by column chromatography, crystallization.

A general method for preparation of intermediate IV is shown in Scheme 3. 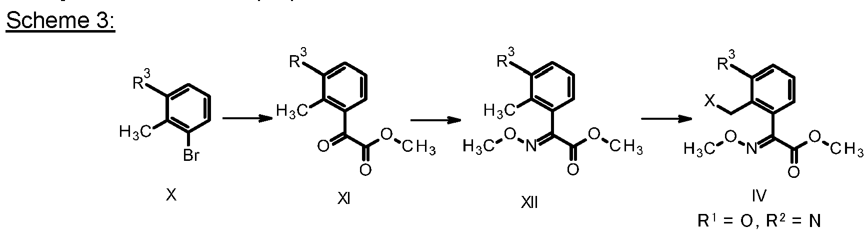 Compound XI could be obtained from X by lithium-halogen exchange or by generating Grignard reagent and further reaction with dimethyl oxalate or chloromethyl oxalate in presence of a solvent. The preferred solvent is THF, 2-methyl-THF and the temperature can be between -70 to - 78 °C. Conversion of intermediate XI to intermediate XII can be achieved using N-methylhydroxylamine hydrochloride and a base such as pyridine or sodium acetate in polar solvents such as methanol. The reaction temperature is preferably about 65 °C. An *E*/*Z* mixture is usually obtained, the isomers can be separated by purification techniques known in art (e.g. column chromatography, crystallization). Bromination of intermediate XII provides the desired intermediate compounds IV, wherein R¹ is O and R² = N. This reaction of intermediate XII with N-bromosuccinimide in solvents such as carbon tetrachloride, chlorobenzene, acetonitrile, using radical initiators such as 1,1'-azobis (cyclohexanecarbonitrile) or azobisisobutyronitrile and is carried out at temperatures of 70 to 100 °C. The preferred radical initiator is 1,1'-azobis (cyclohexanecarbonitrile), preferred solvent chlorobenzene and preferred temperature 80 °C.

The synthesis of compounds containing different substituents R³ follows similar sequence as in Scheme 3, wherein R³ is bromo. Coupling of intermediate III with intermediate IV, wherein R³ is bromo, provides compounds I as described above. Using standard chemical reactions, such as Suzuki or Stille reaction, the bromo group can be converted e.g. to other R³ substituents such as cycloalkyl, alkoxy and alkenyl. Additional transformations e.g. of ethenyl provide compounds I with other R³ substituents such as ethyl, CN and haloalkyl.

Most of the ketones of general formula II were commercially available, however for the ones which were not commercially available, preparation of these was carried out in house using methods known in prior art. Scheme 4 depicts various methods known in literature for the synthesis of these ketones. 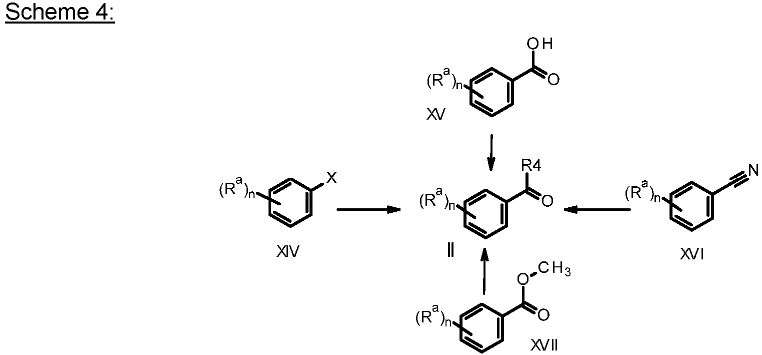 The ketone II can be obtained from the corresponding halogen bearing precursors XIV, wherein X is preferably bromine or iodine. Lithium-halogen exchange (J Org Chem, 1998, 63 (21), 7399-7407) in compound XIII using n-butyllithium or synthesis of the corresponding Grignard reagent (Nature Comm, 2017, 8(1), 1-7) using THF as solvent, and subsequent reaction with N-methoxy-N-methylacetamide at about -70 to -78 °C can provide the ketone II. Alternatively, the coupling reaction of compound XIV and tributyl(1-ethoxyvinyl)stannane in presence of a transition metal catalyst, preferably palladium, with suitable ligands in a solvent such as dioxane and at a reaction temperature of about 100 °C, followed by treatment with 1N HCl can provide ketone II (Org Lett, 2016, 18(7), 1630-1633, WO 2018/115380). Reaction of XIV with 1,4-butanediol vinyl ether in the presence of transition metal catalyst, preferably palladium with suitable ligands and solvent such as 1,2-propane diol and base such as sodium carbonate and reaction temperature of about 120 °C followed by treatment with 1N HCl can provide ketone II (Chem A Eur J, 2008, 14(18), 5555-5566). Another method uses acid compounds XV, which can be converted to the corresponding Weinreb amide or carboxylic ester XVII and subsequent reaction with methylmagnesium bromide (MeMgBr) in solvent such as THF and temperatures of -78 to 0 °C, preferably 0 °C, to provide ketone II. Another method uses the reaction of nitrile XVI with MeMgBr which is carried out in solvent such as THF or toluene, preferably THF, and reaction temperature is 25 to 60 °C, preferably 60 °C, followed by treatment with 1N HCl (Eur J Med Chem, 2015, 102, 582-593).

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e. g. wheat, rye, barley, triticale, oats, or rice; beet, fruits, leguminous plants such as soybean, oil plants, cucurbits, fiber plants, citrus fruits, vegetables, lauraceous plants, energy and raw material plants, corn; tobacco; nuts; coffee; tea; bananas; vines (table grapes and grape juice grape vines); natural rubber plants; or ornamental and forestry plants; on the plant propagation material, such as seeds; and on the crop material of these plants.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc.

Corn is also known as Indian corn or maize (*Zea mays*) which comprises all kinds of corn such as field corn and sweet corn. According to the invention all soybean cultivars or varieties are comprised, in particular indeterminate and determinate cultivars or varieties.

The term "cultivated plants" is to be understood as including plants which have been modified by mutagenesis or genetic engineering to provide a new trait to a plant or to modify an already present trait.

The compounds I and compositions thereof, respectively, are particularly suitable for controlling the following causal agents of plant diseases: rusts on soybean and cereals (e.g. *Phakopsora pachyrhizi* and *P. meibomiae* on soybean; *Puccinia tritici* and *P. striiformis* on wheat); molds on specialty crops, soybean, oil seed rape and sunflowers (e.g. *Botrytis cinerea* on strawberries and vines, *Sclerotinia sclerotiorum, S. minor* and *S. rolfsii* on oil seed rape, sunflowers and soybean); Fusarium diseases on cereals (e.g. *Fusarium culmorum* and *F. graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Septoria tritici* and *S. nodorum* on cereals, *S. glycines* on soybean, *Cercospora* spp. on corn and soybean).

The compounds I and compositions thereof, respectively, are also suitable for controlling harmful microorganisms in the protection of stored products or harvest, and in the protection of materials.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

An agrochemical composition comprises a fungicidally effective amount of a compound I. The term "fungicidally effective amount" denotes an amount of the composition or of the compounds I, which is sufficient for controlling harmful fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

Plant propagation materials may be treated with compounds I as such or a composition comprising at least one compound I prophylactically either at or before planting or transplanting.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types (see "Catalogue of pesticide formulation types and international coding system", Technical Monograph No. 2, 6^{th} Ed. May 2008, CropLife International) are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005. The invention also relates to agrochemical compositions comprising an auxiliary and at least one compound I. Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

The agrochemical compositions generally comprise between 0.01 and 95 %, preferably between 0.1 and 90%, more preferably between 1 and 70 %, and in particular between 10 and 60 %, by weight of active substance (e.g. at least one compound I). Further, the agrochemical compositions generally comprise between 5 and 99.9 %, preferably between 10 and 99.9 %, more preferably between 30 and 99 %, and in particular between 40 and 90 %, by weight of at least one auxiliary.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials, such as seeds, e. g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

Various types of oils, wetters, adjuvants, fertilizers, or micronutrients, and further pesticides (e. g. fungicides, growth regulators, herbicides, insecticides, safeners) may be added to the compounds I or the compositions thereof as premix, or, not until immediately prior to use (tank mix). These agents can be admixed with the compositions according to the invention in a weight ratio of 1:100 to 100:1, preferably 1:10 to 10:1.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-di-oxo-1,5-dioxonan-7-yl] 2-methylpropanoate (A.2.3), fenpicoxamid (A.2.4), florylpicoxamid (A.2.5), metarylpicoxamid (A.2.6);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-N-(1,1,3-trimethyl-indan-4-yl)-pyridine-3-carboxamide (A.3.32), 2-(difluoromethyl)-*N*-[(3R)-1,1,3-trimethylindan-4-yl]-pyridine-3-carboxamide (A.3.33), 2-(difluoromethyl)-*N*-(3-ethyl-1,1-dimethyl-indan-4-yl)-pyridine-3-carboxamide (A.3.34), 2-(difluoromethyl)-*N*-[(3R)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3R)-3-isobutyl-1,1-dimethyl-indan-4-yl]pyridine-3-carboxamide (A.3.39) cyclobutrifluram (A.3.24);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(tri-fluoromethoxy)phenyl]-2-pyridyl]propan-2-ol (B.1.32), fluooxytioconazole (B.1.33), ipfentri-fluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methyl-sulfanyl-acetamide (D.1.9), 2-[(3-ethynyl-8-methyl-6-quinolyl)oxy]-N-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flumetylsulforim (K.1.60), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), seboctylamine (K.1.61), tebufloquin (K.1.24), tecloftalam (K.1.25), triazoxide (K.1.26), *N'*-(4-(4-chloro-3-trifluoromethyl-phenoxy)-2,5-dimethyl-phenyl)-*N*-ethyl-N-methyl formamidine (K.1.27), *N'*-(4-(4-fluoro-3-trifluoromethyl-phenoxy)-2,5-dimethyl-phenyl)-*N*-ethyl-*N*-methyl formamidine (K.1.28), *N'*-[4-[[3-[(4-chlorophenyl)methyl]-1,2,4-thiadiazol-5-yl]oxy]-2,5-dimethyl-phenyl]-*N*-ethyl-*N*-methyl-formamidine (K.1.29), *N'*-(5-bromo-6-indan-2-yloxy-2-methyl-3-pyridyl)-*N*-ethyl-*N*-methyl-formamidine (K.1.30), *N'*-[5-bromo-6-[1-(3,5-difluorophenyl)-ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), N'-[5-bromo-2-methyl-6-(1-phenylethoxy)-3-pyridyl]-*N*-ethyl-*N*-methyl-formamidine (K.1.33), *N'*-(2-methyl-5-trifluoromethyl-4-(3-trimethylsilanyl-propoxy)-phenyl)-*N*-ethyl-*N*-methyl formamidine (K.1.34), *N'*-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (Z)-3-amino-2-cyano-3-phenyl-prop-2-enoate (K.1.40), picarbutrazox (K.1.41), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benzothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), N'-(2,5-dimethyl-4-phenoxy-phenyl)-*N*-ethyl-*N*-methyl-formamidine (K.1.53), aminopyrifen (K.1.54), fluopimomide (K.1.55), *N'*-[5-bromo-2-methyl-6-(1-methyl-2-propoxy-ethoxy)-3-pyridyl]-*N*-ethyl-*N*-methyl-formamidine (K.1.56), *N'*-[4-(4,5-dichlorothiazol-2-yl)oxy-2,5-dimethyl-phenyl]-*N*-ethyl-*N*-methyl-formamidine (K.1.57), flufenoxadiazam (K.1.58), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (WO2018/177894, WO 2020/212513);

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1:1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.6); particularly selected from (A.2.3), (A.2.4) and (A.2.6).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.33), (B.1.34), (B.1.37), (B.1.38), (B.1.43), (B.1.46), (B.1.53), (B.1.54) and (B.1.55); particularly selected from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (I.2.2) and (I.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The compositions comprising mixtures of active ingredients can be prepared by usual means, e. g. by the means given for the compositions of compounds I.

### Examples:

### Synthetic process

### Example 1: Methyl (2E)-2-[2-[[(E)-3-(2-fluorophenyl)ethylideneamino]oxymethyl]-3-methylphenyl]-2-methoxyimino-acetate

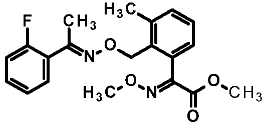

### Step 1: 1-(2-Fluorophenyl)ethanone oxime

1-(2-fluorophenyl)ethenone (10 g, 1.0 eq) was taken in methanol (300 ml) and hydroxyl amine hydrochloride (7.54 g, 1.8 eq) was added. Pyridine (33.45 g, 2 eq) was added drop wise at 25 °C. Reaction mixture was stirred at 50 °C for 2 hr. Reaction was monitored using LCMS & TLC. Methanol was evaporated under vacuum. Crude mass was diluted with water (200 ml) and it was extracted with ethyl acetate (3 x 100 ml). Combined organic layer was again washed with water and brine. Organic layer was dried over sodium sulphate and concentrated under vacuum. Crude compound was purified by flash column chromatography. Pure compound was eluted with 0% to 20% ethyl acetate (EtOAc) in heptane. Evaporation of solvent afforded 8 g title compound as white solid (Yield 72%). ¹H NMR 300 MHz, DMSO-d6: δ 11.4 (s ,1 H), 7.46-7.41 (m, 2 H), 7.27-7.23 (m, 2H), 2.14 (s, 3H).

### Step 2: Ethyl (2E)-2-[2-[[(E)-1-(2-fluorophenyl)ethylideneamino]oxymethyl]-3-methyl-phenyl]-2-methoxyimino-acetate (Ex. 2)

1-(2-fluorophenyl)ethanone oxime (0.3 g, 3 eq) was taken in dimethyl formamide (DMF, 5 ml) and Cs₂CO₃ (3.27 g, 2.0 eq) was added. The reaction mixture was stirred for 30 minutes at room temperature (RT; at about 25 °C) and then added methyl (2*E*)-2-[2-(bromomethyl)-3-methyl-phenyl]-2-methoxyimino-acetate (0.6 g, 3.02 eq). The reaction mixture was stirred at RT for 32 hr and monitored by TLC and LCMS. Reaction was quenched with water (45 ml) and the product was extracted in ethyl acetate (3 x 35 ml). The combined organic layer was washed with brine (50 ml), dried over sodium sulphate and concentrated under vacuum. Crude material was purified by flash chromatography. Pure compound was eluted by using 35-20% EtOAc in heptane. Evaporation of solvent afforded an off-white solid title compound (0.328 g, 45% yield). ¹H NMR (300 MHz, DMSO-d6): δ 7.56 - 7.36 (m, 2H), 7.33 - 7.32 (m, 4H), 7.03 (dd, J = 6.2, 2.8 Hz, 3H), 5.00 (s, 2H), 3.93 (s, 3H), 3.64 (s, 3H), 2.42 (s, 3H), 2.08 (d, J = 2.5 Hz, 3H).

### Example 2: (2E)-2-[2-[[(E)-1-(2-fluorophenyl)ethylideneamino]oxymethyl]-3-methyl-phenyl]-2-methoxyimino-N-methyl-acetamide

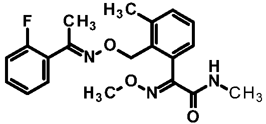

Methyl (2*E*)-2-[2-[[(*E*)-1-(2-fluorophenyl)ethylideneamino]oxymethyl]-3-methyl-phenyl]-2-methoxyimino-acetate (ex. 1; 8 g,1 eq) was taken in THF (80 ml) and methylamine (40% aqueous) solution (16 ml, 2 vol) was added. The reaction mixture was stirred at 25 °C for 5 hr and monitored by TLC and LCMS. Reaction was quenched with water (200 ml) and the product was extracted in ethyl acetate (3 x 150 ml). The combined organic layer was washed with brine (150 ml), dried over sodium sulphate and concentrated under vacuum. Crude material was purified by flash chromatography. Pure compound was eluted by using 30-40% EtOAc in heptane. Evaporation of solvent afforded white solid title compound (7 g, 87.7% yield). ¹H NMR (500 MHz, DMSO-d6): δ 8.20 (q, J = 4.7 Hz, 1H), 7.44 (ddt, J = 7.8, 5.6, 2.0 Hz, 2H), 7.37 - 7.14 (m, 4H), 6.95 (dd, J = 7.1, 2.0 Hz, 1H), 5.01 (s, 2H), 3.86 (s, 3H), 2.65 (d, J = 4.8 Hz, 3H), 2.42 (s, 3H), 2.09 (d, J = 2.6 Hz, 3H).

### Example 3: Methyl (2E)-2-[2-[[(E)-1-(3,5-dichlorophenyl)ethylideneamino]oxymethyl]-3-methylphenyl]-2-methoxyimino-acetate

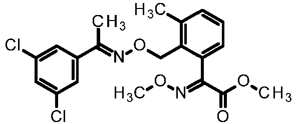

### Step 1: 1-(3,5-dichlorophenyl)ethanone oxime

3-(3,5-Dichlorophenyl)ethanone (3.0 g, 3eq) was taken in methanol (30 ml) and NH₂OH (0.735 g, 2 eq) followed by pyridine (3.04 g, 2.5 eq) were added. Reaction mixture was heated to 70 °C and stirred for 3 hr. Reaction was monitored using LCMS & TLC. Solvent was evaporated and the residue was diluted with water (50 ml). The product was extracted in with ethyl acetate (3 x 30 ml). The combined organic layer was washed with brine (50 ml), dried over sodium sulphate and concentrated under vacuum. Crude material was purified by flash chromatography. Pure compound was eluted by using 15-20% EtOAc in heptane. Evaporation of solvent afforded white solid compound 1-(3,5-dichlorophenyl)ethanone oxime (1 g, 92.6% yield).

### Step 2: Methyl (2E)-2-[2-[[(E)-1-(3,5-dichlorophenyl)ethylideneamino]oxymethyl]-3-methylphenyl]-2-methoxyimino-acetate

3-(3,5-Dichlorophenyl)ethanone oxime (0.4 g, 1 eq) was taken in acetonitrile (10 ml) and Cs₂CO₃ (1.8 g, 2.5 eq) was added. The reaction mixture was stirred for 30 min at RT and then added methyl (2*E*)-2-[2-(bromomethyl)-3-methyl-phenyl]-2-methoxyimino-acetate (0.65 g, 1.05 eq). The reaction mixture was stirred at RT for 3 hr and monitored by TLC and LCMS. Reaction was quenched with water (50 ml) and the product was extracted in ethyl acetate (3 x 30 ml). The combined organic layer was washed with brine (50 ml), dried over sodium sulphate and concentrated under vacuum. Crude material was purified by flash chromatography. Pure compound was eluted by using 20-25% EtOAc in heptane. Evaporation of solvent afforded an off-white solid title compound (0.6 g, 68% yield). ¹H NMR (500 MHz, DMSO-d6): δ 7.66 (t, J = 1.9 Hz, 1H), 7.61 (d, J = 1.9 Hz, 2H), 7.36 - 7.23 (m, 2H), 7.05 - 6.98 (m, 1H), 5.04 (s, 2H), 3.91 (s, 3H), 3.70 (s, 3H), 2.43 (s, 3H), 2.30 (s, 3H).

### Example 4: (2E)-2-[2-[[(E)-1-(3,5-dichlorophenyl)ethylideneamino]oxymethyl]-3-methyl-phenyl]-2-methoxyimino-N-methyl-acetamide

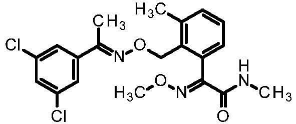

Methyl (2*E*)-2-[2-[[(*E*)-3-(3,5-dichlorophenyl)ethylideneamino]oxymethyl]-3-methyl-phenyl]-2-methoxyimino-acetate (ex. 3; 0.6 g, 1 eq) was taken in THF (6 ml) and methyl amine (40% aq.) solution (1.2 ml, 2v) was added. The reaction mixture was stirred at RT for 3 hr and monitored by TLC and LCMS. Reaction was quenched with water (25 ml) and the product was extracted in ethyl acetate (3 x 20 ml). The combined organic layer was washed with brine (25 ml), dried over sodium sulphate and concentrated under vacuum. Crude material was purified by flash chromatography. Pure compound was eluted by using 40-45% EtOAc in heptane. Evaporation of solvent afforded white solid title compound (example 2, 0.53 g, 85% yield). ¹H NMR (500 MHz, DMSO-d6): δ 8.24 (d, J = 4.8 Hz, 1H), 7.69 - 7.58 (m, 3H), 7.37 - 7.15 (m, 2H), 6.95 (dd, J = 7.1, 1.9 Hz, 1H), 5.05 (s, 2H), 3.86 (s, 3H), 2.68 (d, J = 4.7 Hz, 3H), 2.42 (s, 3H), 2.11 (s, 3H).

### Example 5: Methyl (2E)-2-methoxyimino-2-[3-methyl-2-[[(E)-1-(p-tolyl)ethylideneamino] oxymethyl]phenyl]acetate

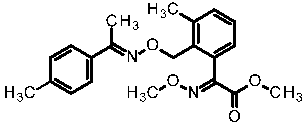

### Step 1: 1-(p-tolyl)ethanone oxime

To a solution of 1-(p-tolyl)ethanone (1.0 g, 4.45 mmol, 3 eq.) in methanol (10 mL) was added hydroxylamine hydrochloride (0.77 g, 11.17 mmol, 1.5 eq) followed by addition of sodium acetate (1.82 g, 15 mmol, 2 eq.) at RT under nitrogen atmosphere. Reaction mixture was refluxed for 2 hrs. Reaction was monitored by TLC. Reaction mixture was concentrated on rotavapor. To this crude residue was added water (20 mL) and stirred for 0.5 hr. Solid material filtered and dried to obtain pure title compound (1.1 g, yield 98 %) as white solid. MS: [M + H]⁺ 150.

### Step 2: Methyl (2E)-2-methoxyimino-2-[3-methyl-1-[[(E)-3-(p-tolyl)ethylideneamino] oxymethyl]phenyl]acetate

To a stirred solution of 1-(p-tolyl)ethanone oxime (0.15g, 1.0 mmol, 1 eq) in acetonitrile (2mL) was added Cs₂CO₃ (0.66 g, 2.0 mmol, 2 eq). The reaction mixture was stirred at 25 °C for 30 min. Then, methyl (2*E*)-2-[2-(bromomethyl)-3-methyl-phenyl]-2-methoxyimino-acetate (0.33 g, 1.1 mmol, 1.1 eq) was added. The mixture was stirred at 25 °C for 6 h. Reaction was monitored by TLC and LCMS. To this reaction mixture was added water (30 mL) and extracted with EtOAc (3 x 30 mL). Combined organic layer was washed with H₂O (2 x 25 mL), followed by brine wash (2 x 20 mL). Organic layer was dried over Na₂SO₄ and Concentrated to afford crude compound which was further purified by flash column chromatography using 0-20% EtOAc in heptane as the eluent to obtain pure title compound as white solid (0.37 g, Yield 96%). ¹H NMR (500 MHz, chloroform-d): δ 7.42 (d, *J =* 8.2 Hz, 2H), 7.26 - 7.19 (m, 3H), 7.07 (d, *J* = 8.0 Hz, 2H), 6.94 (dd, *J* = 7.2, 1.8 Hz, 2H), 5.03 (s, 2H), 3.94 (s, 3H), 3.70 (s, 3H), 2.41 (s, 3H), 2.27 (s, 3H), 2.06 (s, 3H). MS: [M + H] ⁺ 369.

### Example 6: (2E)-2-Methoxyimino-N-methyl-2-[3-methyl-2-[[(E)-1-(p-tolyl)ethylidene-amino]oxymethyl]phenyl]acetamide

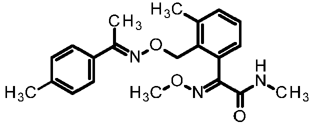

To a stirred solution of methyl (2*E*)-2-methoxyimino-2-[3-methyl-1-[[(*E*)-3-(p-tolyl)-ethylideneamino]oxymethyl]phenyl]acetate in THF (5 mL), methyl amine solution in water (5.0 mL, 40 %) was added at RT. Reaction was continued for 1 hr. Reaction was monitored by TLC. Reaction mixture was evaporated on rotavapor, residue was diluted with EtOAc (20 mL) and washed with 1N HCl (3 x 20 mL), followed by brine wash (2 x 20 mL). Organic layer was dried over Na₂SO₄ and Concentrated to afford crude compound which was further purified by flash column chromatography using 0-50% EtOAc in heptane as the eluent to afford pure title compound as white solid (0.200 g, Yield 88%). ¹H NMR (500 MHz, DMSO-*d*₆): δ 8.20 (d, *J =* 5.0 Hz, 1H), 7.54 - 7.48 (m, 2H), 7.31 - 7.22 (m, 2H), 7.19 (d, *J =* 8.0 Hz, 2H), 6.95 (dd, *J =* 6.9, 2.1 Hz, 1H), 4.99 (s, 2H), 3.86 (s, 3H), 2.69 (d, *J* = 4.7 Hz, 3H), 2.43 (s, 3H), 2.31 (s, 3H), 2.08 (s, 3H). MS: [M + H] ⁺ 368.

### Example 7: (2E)-2-methoxyimino-N-methyl-2-[3-methyl-2-[[(E)-[3,3,3-trifluoro-1-[3-(trifluoromethyl)phenyl]propylidene]amino]oxymethyl]phenyl]acetamide

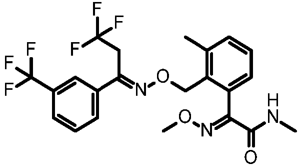

3,3,3-Trifluoro-1-[3-(trifluoromethyl)phenyl]propan-1-one (0.5 g, 1 eq), prepared in analogy to prior art process (Chem Commun, 2016, 52, 13668-13670), was taken in THF (10 ml) and (2E)-2-[2-(aminooxymethyl)-3-methyl-phenyl]-2-methoxyimino-N-methyl-acetamide (0.98 g, 2 eq) followed by Ti(OEt)₄ (1.33g, 3 eq) were added. The mixture was heated to 70°C and stirred for 12 hr. The reaction was monitored by TLC and LCMS. The reaction was quenched with water (25 ml) followed by EtOAc (25 ml). The emulsion formed was filtered through celite and washed with EtOAc (50 ml). The layers were separated and the aequous layer was extracted in EtOAc (2 x 25ml). The combined organic layer was washed with brine (25 ml), dried over sodium sulphate and concentrated under vacuum. Crude material was purified by flash chromatography. Pure compound was eluted by using 40-45% EtOAc in heptane. Evaporation of solvent followed by crystallization in heptane afforded an off-white solid (0.34g, 35% yield). ¹H NMR (500 MHz, DMSO-d6): δ 8.27 (q, J = 4.7 Hz, 1H), 8.07 - 8.00 (m, 2H), 7.85 - 7.79 (m, 1H), 7.68 (t, J = 7.8 Hz, 1H), 7.35 - 7.24 (m, 2H), 6.97 (dd, J = 7.3, 1.7 Hz, 1H), 5.12 (s, 2H), 4.03-3.96 (q, J = 10 Hz, 2H), 3.86 (s, 3H), 2.67 (d, J = 4.7 Hz, 3H), 2.43 (s, 3H).

The following examples in Table S were synthesized as per general Scheme 1 described above (except Ex. 7 and 212 which were synthesized as per scheme 2) and characterized by LCMS as described in Table L.

**Table L: LCMS Methods**

| **LCMS Method A** | |
|---|---|
| **Method details** | **Device details** |
| Column: Agilent Eclipse Plus C18 (50 mm × 4.6 mm × 3 µm particles) | LCMS2020 (Shimadzu) |
| | Ionization source: ESI |
| Mobile Phase: | Mass range: 100 - 800 amu |
| A: 10 mM Ammonium formate in water. | Polarity: Dual (positive and negative simultaneous scan) |
| B: 0.1 % Formic acid in acetonitrile | |
| Gradient: 10 % B to 100 % B in 1.5 min. | Mode: Scan |
| Hold 1 min 100 % B. 1 min 10 % B. Run time: 3.50 or 3.75 min. | LC System: Nexera High pressure gradient system, Binary pump |
| Flow: 1.2 ml/min; | Detector: PDA |
| Column oven: 30°C/40°C | Scanning wavelength: 220 nm / max plot |

| **LCMS Method B** | |
|---|---|
| **Method details** | **Device details** |
| Column: Luna-C18 (30 mm × 2.0 mm × 3 µm particles) | LCMS DELIVER-220 (Shimadzu) |
| | Ionization source: ESI |
| Mobile Phase: | Mass range: 100 - 1000 amu |
| A: 0.037% Trifluoroacetic acid in water. | Polarity: Positive |
| B: 0.018% Trifluoroacetic acid in HPLC grade acetonitrile | Mode: Scan |
| | LC System: Nexera High pressure gradient system, Binary pump |
| Gradient: 5-95% B in 3.00 min .5% B in 0.01 min, 5-95% B (0.01-1.60 min), 95-100% B (1.60 - 2.50 min), 100 -5% (2.50 - 2.52 min) with a hold at 5% B for 0.48 min. | |
| | Detector: DAD |
| | Scanning wavelength: 220 nm / max plot |
| Flow: 0.8 mL/min; | |
| Column oven: 40°C | |

| **LCMS Method C** | |
|---|---|
| **Method details** | **Device details** |
| Column: Xbridge Shield RP18 (50 mm x 2.1 mm, 5 µm particles) | Agilent |
| | Ionization source: ESI |
| Mobile Phase: | Mass range: 100 - 1000 amu |
| A: H₂O+10 mM NH₄HCO₃ | Polarity: Positive |
| B: Acetonitrile | Mode: Scan |
| Gradient: 5% B in 0.40 min and 5-95% B at 0.40-3.40 min, hold on 95% B for 0.45 min, and then 95-5%B in 0.01 min. | LC System: Nexera High pressure gradient system, Binary pump |
| | Detector: DAD |
| Flow: 0.8 ml/min; | Scanning wavelength: 220 nm / max plot |
| Column oven: 40°C | |

| **LCMS Method D** | |
|---|---|
| **Method details** | **Device details** |
| Column: Agilent Eclipse Plus C18 (50 mm × 4.6 mm × 3 µm particles) | LCMS 2020 (Shimadzu) |
| | Ionization source: ESI |
| Mobile Phase: | Mass range: 100 - 800 amu |
| A: 10 mM NH₄(HCOO) in water | Polarity: Dual (positive and negative simultaneous scan) |
| B: Acetonitrile | |
| Gradient: 10 % B to 100 % B in 5 min, hold on 100 % B for 3 min, 2 min 10 % B. | Mode: Scan |
| | LC System: Nexera High pressure gradient system, Binary pump |
| Run time: 10 min. | |
| Flow: 1.2 ml/min; | Detector: PDA |
| Column oven: 40°C | Scanning wavelength: 220 nm / max plot |

Used LCMS Method in Table S to be found in Column LCMS.

**Table S:**

| **No.** | **Structure** | **Rₜ [min]** | **Mass** | **LCMS** |
|---|---|---|---|---|
| 1 | 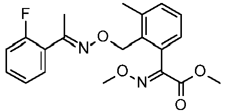 | 2.08 | 373.7 | A |
| 2 | 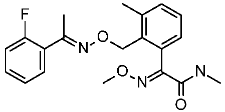 | 1.941 | 372 | A |
| 3 | 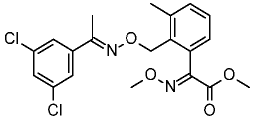 | 2.252 | 422.9 | A |
| 4 | 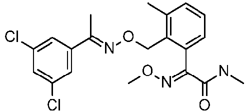 | 2.15 | 421.9 | A |
| 5 | 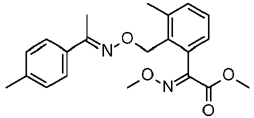 | 2.144 | 369 | A |
| 6 | 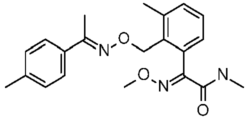 | 2.027 | 368 | A |
| 7 | 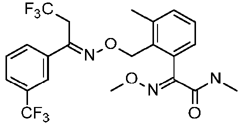 | 2.123 | 490 | A |
| 8 | 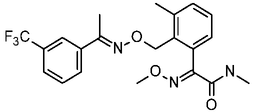 | 2.15 | 422.5 | A |
| 9 | 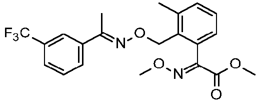 | 2.19 | 423.5 | A |
| 10 | 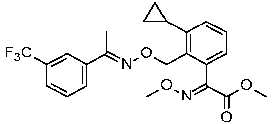 | 2.22 | 449.2 3 | A |
| 11 | 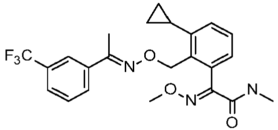 | 2.13 | 448.4 | A |
| 12 | 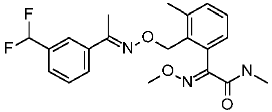 | 1.95 | 404 | A |
| 13 | 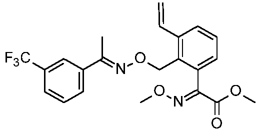 | 2.18 | 435.3 | A |
| 14 | 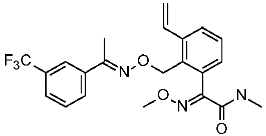 | 2.11 | 434.4 | A |
| 15 | 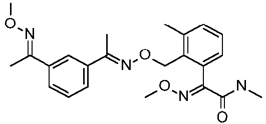 | 2.05 | 425.2 | A |
| 16 | 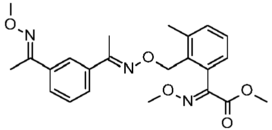 | 2.17 | 426.2 | A |
| 17 | 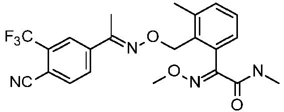 | 1.99 | 447.1 | A |
| 18 | 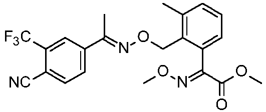 | 2.09 | 448.2 | A |
| 19 | 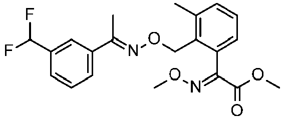 | 2.06 | 404 | A |
| 20 | 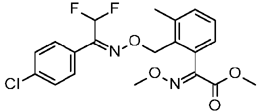 | 2.155 | 425 | A |
| 21 | 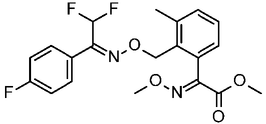 | 2.06 | 408.5 | A |
| 22 | 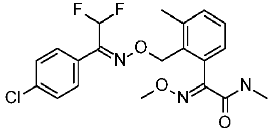 | 2.08 | 424 | A |
| 23 | 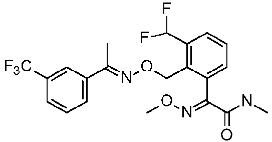 | 2.04 | 458.3 | A |
| 24 | 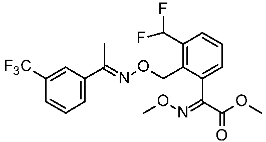 | 2.07 | 458.9 | A |
| 25 | 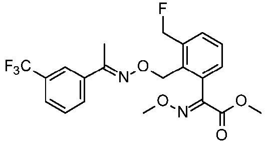 | 2.07 | 441.0 5 | A |
| 26 | 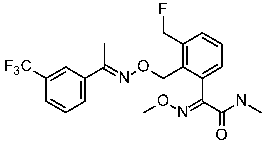 | 1.984 | 440 | A |
| 27 | 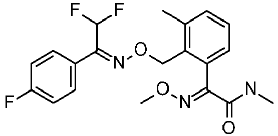 | 1.97 | 408 | A |
| 28 | 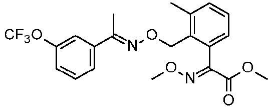 | 2.17 | 439 | A |
| 29 | 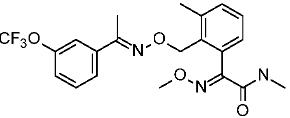 | 2.09 | 438 | A |
| 30 | 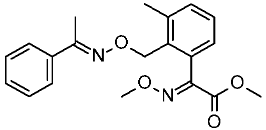 | 2.058 | 355 | A |
| 31 | 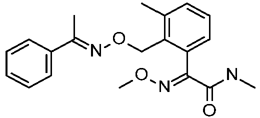 | 1.963 | 354 | A |
| 32 | 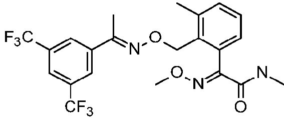 | 2.17 | 490 | A |
| 33 | 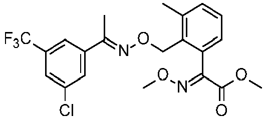 | 2.25 | 456.9 | A |
| 34 | 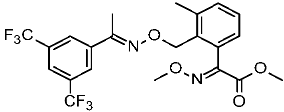 | 2.25 | 491 | A |
| 35 | 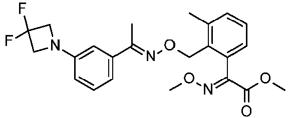 | 2.1 | 446.8 | A |
| 36 | 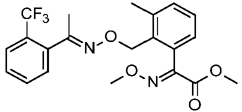 | 2.101 | 423 | A |
| 37 | 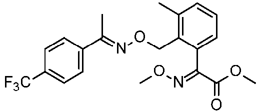 | 2.155 | 422.9 | A |
| 38 | 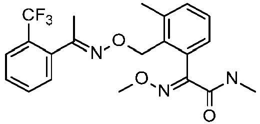 | 1.999 | 422 | A |
| 39 | 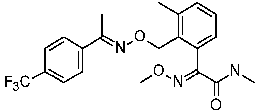 | 2.059 | 422 | A |
| 40 | 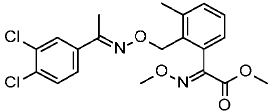 | 2.271 | 423.7 | A |
| 41 | 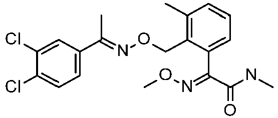 | 2.15 | 422 | A |
| 42 | 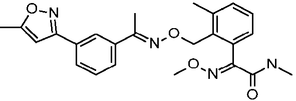 | 1.94 | 435.9 | A |
| 43 | 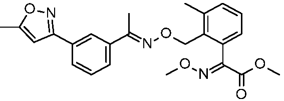 | 2.09 | 436 | A |
| 44 | 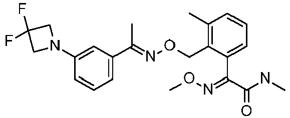 | 1.99 | 445.9 | A |
| 45 | 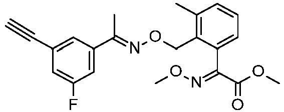 | 2.13 | 397 | A |
| 46 | 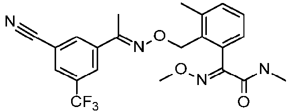 | 2.01 | 447 | A |
| 47 | 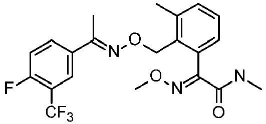 | 2.08 | 440 | A |
| 48 | 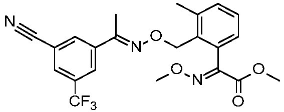 | 2.11 | 448 | A |
| 49 | 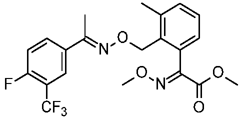 | 2.18 | 441 | A |
| 50 | 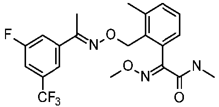 | 2.11 | 440.8 | A |
| 51 | 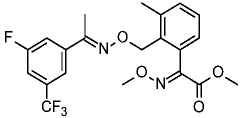 | 2.2 | 441 | A |
| 52 | 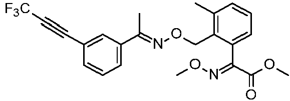 | 2.274 | 447.8 | A |
| 53 | 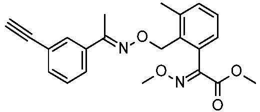 | 2.094 | 379.8 | A |
| 54 | 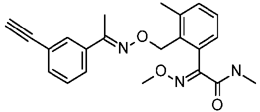 | 1.984 | 378 | A |
| 55 | 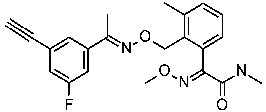 | 2.02 | 396 | A |
| 56 | 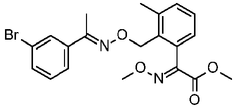 | 2.197 | 435.6 | A |
| 57 | 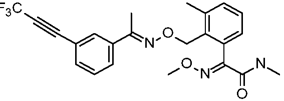 | 2.208 | 446.1 | A |
| 58 | 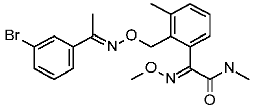 | 2.091 | 432.8 | A |
| 59 | 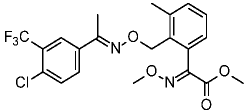 | 2.26 | 457 | A |
| 60 | 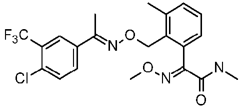 | 2.15 | 456 | A |
| 61 | 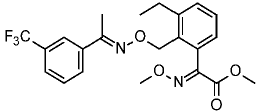 | 2.22 | 437 | A |
| 62 | 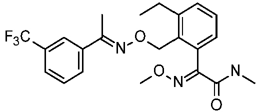 | 2.146 | 436 | A |
| 63 | 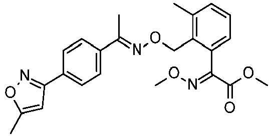 | 2.099 | 436 | A |
| 64 | 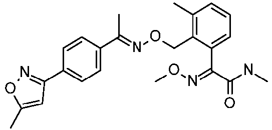 | 1.97 | 435 | A |
| 65 | 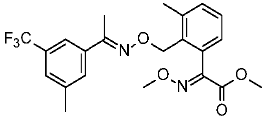 | 2.24 | 437 | A |
| 66 | 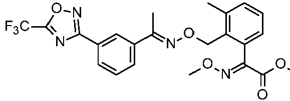 | 2.24 | 491 | A |
| 67 | 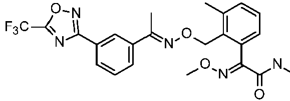 | 2.15 | 490 | A |
| 68 | 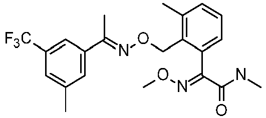 | 2.14 | 436 | A |
| 69 | 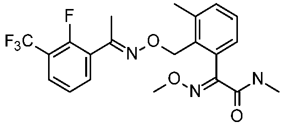 | 2.059 | 440 | A |
| 70 | 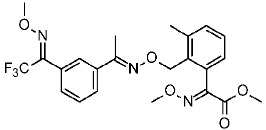 | 2.197 | 480 | A |
| 71 | 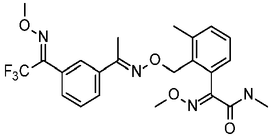 | 2.091 | 479 | A |
| 72 | 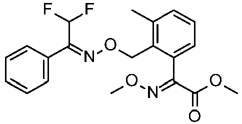 | 1.337 | 391 | A |
| 73 | 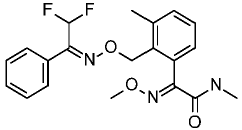 | 1.256 | 390 | A |
| 74 | 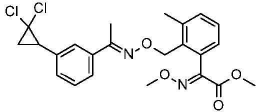 | 2.208 | 463 | A |
| 75 | 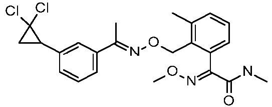 | 2.101 | 462 | A |
| 76 | 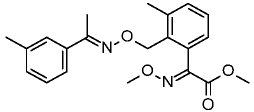 | 2.22 | 369 | A |
| 77 | 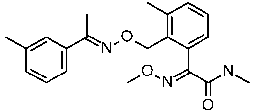 | 2.1 | 368 | A |
| 78 | 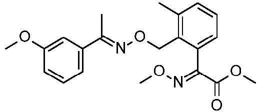 | 2.133 | 385 | A |
| 79 | 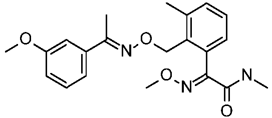 | 2.005 | 384 | A |
| 80 | 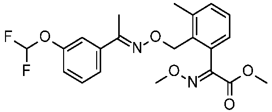 | 2.13 | 421 | A |
| 81 | 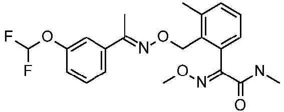 | 2.037 | 420 | A |
| 82 | 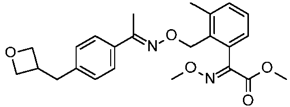 | 2.08 | 425 | A |
| 83 | 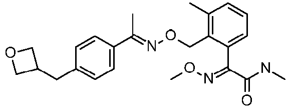 | 1.92 | 424 | A |
| 84 | 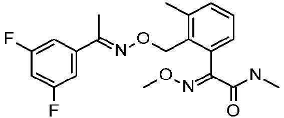 | 2.08 | 390 | A |
| 85 | 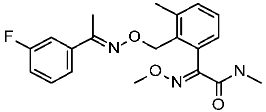 | 2.03 | 372 | A |
| 86 | 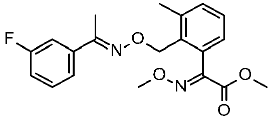 | 2.17 | 373 | A |
| 87 | 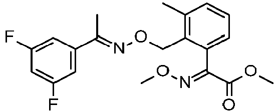 | 2.08 | 391 | A |
| 88 | 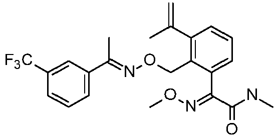 | 2.24 | 448 | A |
| 89 | 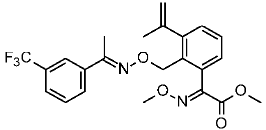 | 2.15 | 449 | A |
| 90 | 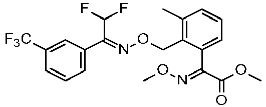 | 2.261 | 459 | A |
| 91 | 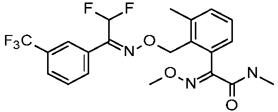 | 2.155 | 458 | A |
| 92 | 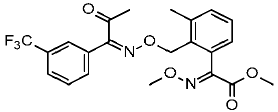 | 2.21 | 451 | A |
| 93 | 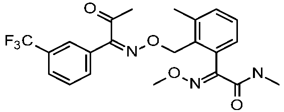 | 2.11 | 450 | A |
| 94 | 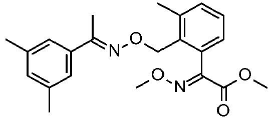 | 2.187 | 383 | A |
| 95 | 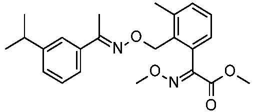 | 2.22 | 397 | A |
| 96 | 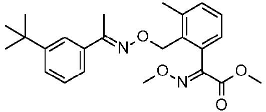 | 2.283 | 411 | A |
| 97 | 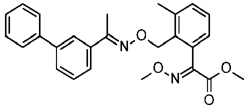 | 2.208 | 431 | A |
| 98 | 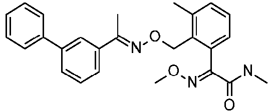 | 5.01 | 430 | D |
| 99 | 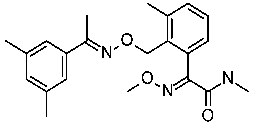 | 2.08 | 382 | A |
| 100 | 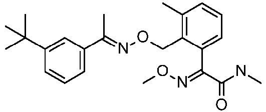 | 2.187 | 410 | A |
| 101 | 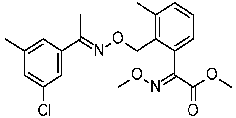 | 2.22 | 403 | A |
| 102 | 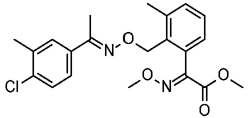 | 2.21 | 403 | A |
| 103 | 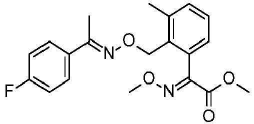 | 2.08 | 373 | A |
| 104 | 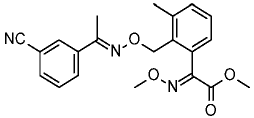 | 1.995 | 380 | A |
| 105 | 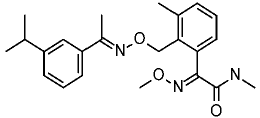 | 2.144 | 396 | A |
| 106 | 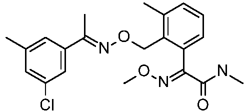 | 2.112 | 402 | A |
| 107 | 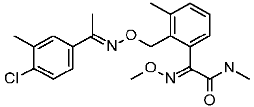 | 2.123 | 402 | A |
| 108 | 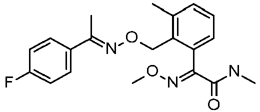 | 1.952 | 372 | A |
| 109 | 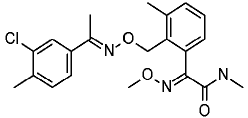 | 2.123 | 402 | A |
| 110 | 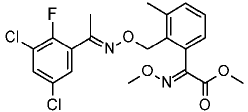 | 2.25 | 441 | A |
| 111 | 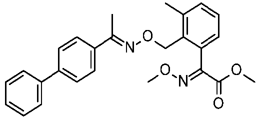 | 2.2 | 431 | A |
| 112 | 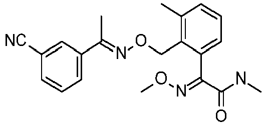 | 1.87 | 379 | A |
| 113 | 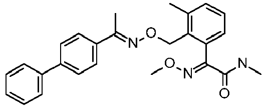 | 2.11 | 430 | A |
| 114 | 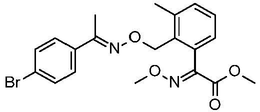 | 2.17 | 435 | A |
| 115 | 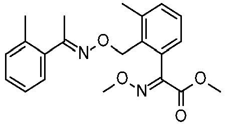 | 2.113 | 369 | A |
| 116 | 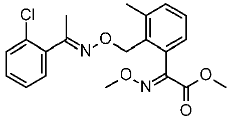 | 2.101 | 389 | A |
| 117 | 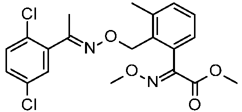 | 2.197 | 423 | A |
| 118 | 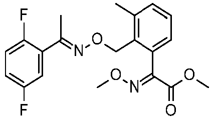 | 2.091 | 391 | A |
| 119 | 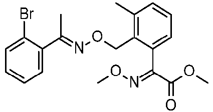 | 2.12 | 434 | A |
| 120 | 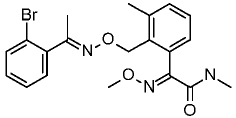 | 2.005 | 433 | A |
| 121 | 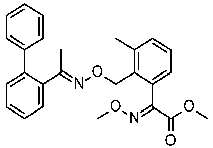 | 2.2 | 431 | A |
| 122 | 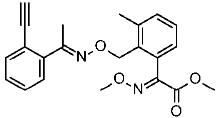 | 2.05 | 379 | A |
| 123 | 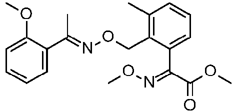 | 2.04 | 385 | A |
| 124 | 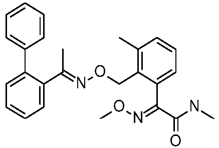 | 2.11 | 430 | A |
| 125 | 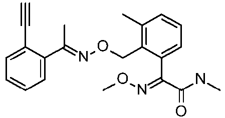 | 1.93 | 378 | A |
| 126 | 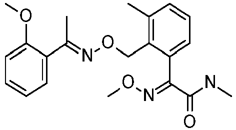 | 1.931 | 384 | A |
| 127 | 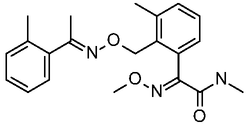 | 1.984 | 368 | A |
| 128 | 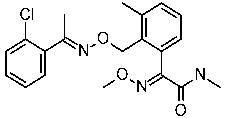 | 1.984 | 388 | A |
| 129 | 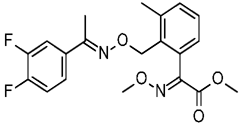 | 2.112 | 391 | A |
| 130 | 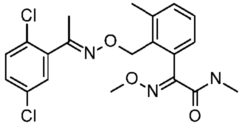 | 2.08 | 422 | A |
| 131 | 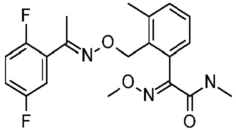 | 1.984 | 390 | A |
| 132 | 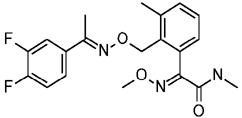 | 1.984 | 390 | A |
| 133 | 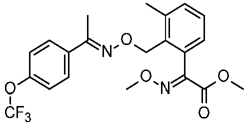 | 2.187 | 439 | A |
| 134 | 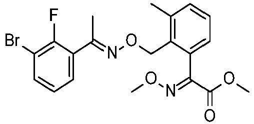 | 2.155 | 453 | A |
| 135 | 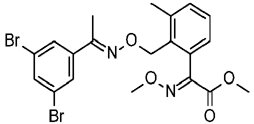 | 2.29 | 513 | A |
| 136 | 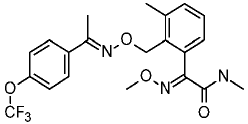 | 2.08 | 438 | A |
| 137 | 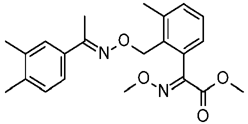 | 2.18 | 383 | A |
| 138 | 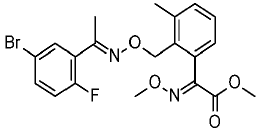 | 2.261 | 453 | A |
| 139 | 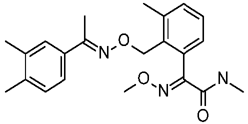 | 2.155 | 382 | A |
| 140 | 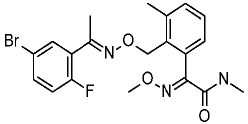 | 2.144 | 450 | A |
| 141 | 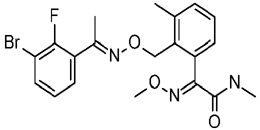 | 2.069 | 452 | A |
| 142 | 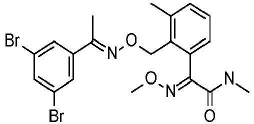 | 2.208 | 512 | A |
| 143 | 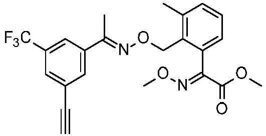 | 2.197 | 447 | A |
| 144 | 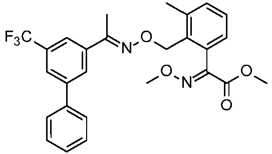 | 2.304 | 499 | A |
| 145 | 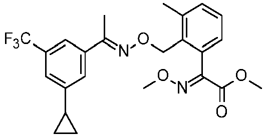 | 2.261 | 463 | A |
| 146 | 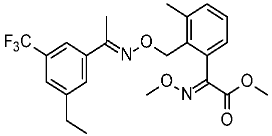 | 2.261 | 451 | A |
| 147 | 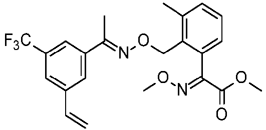 | 2.24 | 449 | A |
| 148 | 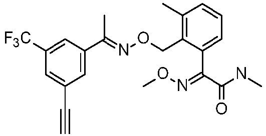 | 2.187 | 446 | A |
| 149 | 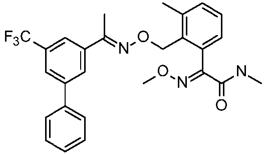 | 2.347 | 498 | A |
| 150 | 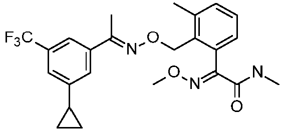 | 2.272 | 462 | A |
| 151 | 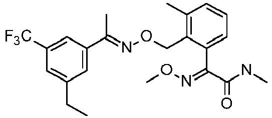 | 2.261 | 450 | A |
| 152 | 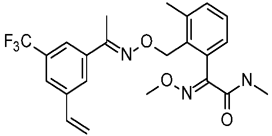 | 2.229 | 448 | A |
| 153 | 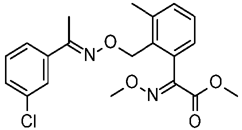 | 2.155 | 389 | A |
| 154 | 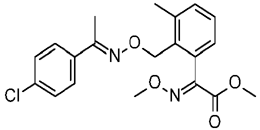 | 2.144 | 389 | A |
| 155 | 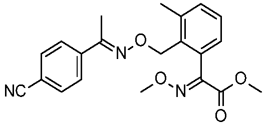 | 1.995 | 380 | A |
| 156 | 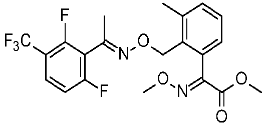 | 2.133 | 459 | A |
| 157 | 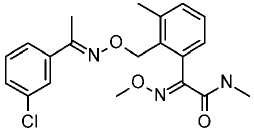 | 2.132 | 388 | A |
| 158 | 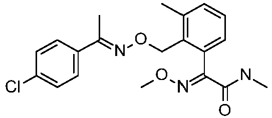 | 2.133 | 388 | A |
| 159 | 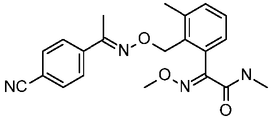 | 1.941 | 379 | A |
| 160 | 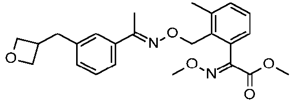 | 2.08 | 425 | A |
| 161 | 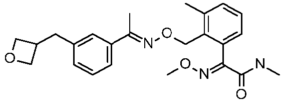 | | | |
| 162 | 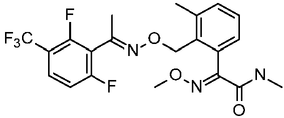 | 2.091 | 458 | A |
| 163 | 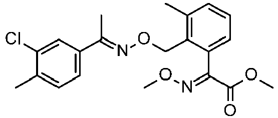 | 2.229 | 403 | A |
| 164 | 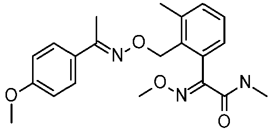 | 1.995 | 384 | A |
| 165 | 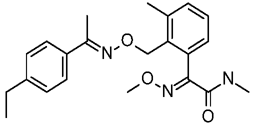 | 2.187 | 382 | A |
| 166 | 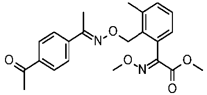 | 2.048 | 397 | A |
| 167 | 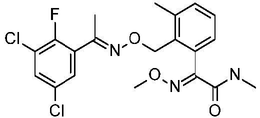 | 2.219 | 440 | A |
| 168 | 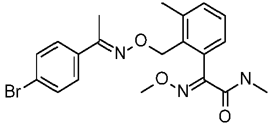 | 2.133 | 434 | A |
| 169 | 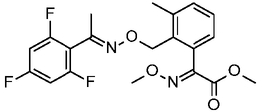 | 2.112 | 409 | A |
| 170 | 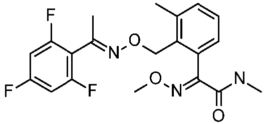 | 1.984 | 408 | A |
| 171 | 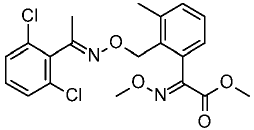 | 2.29 | 423 | A |
| 172 | 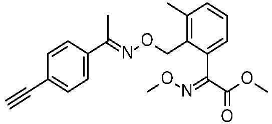 | 2.165 | 379 | A |
| 173 | 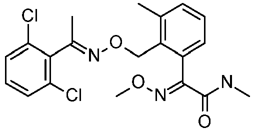 | 2.069 | 422 | A |
| 174 | 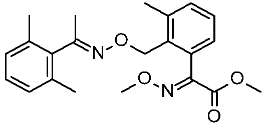 | 2.24 | 383 | A |
| 175 | 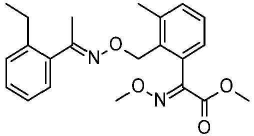 | 2.261 | 383 | A |
| 176 | 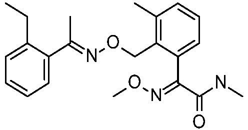 | 2.145 | 382 | A |
| 177 | 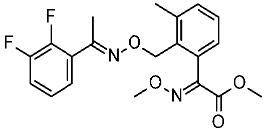 | 2.165 | 391 | A |
| 178 | 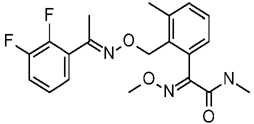 | 2.037 | 390 | A |
| 179 | 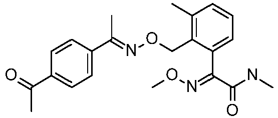 | 1.888 | 396 | A |
| 180 | 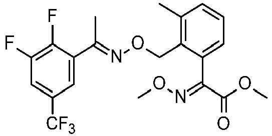 | 2.273 | 459 | A |
| 181 | 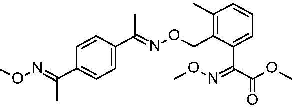 | 2.261 | 426 | A |
| 182 | 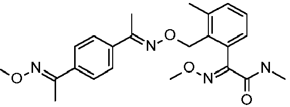 | 2.144 | 425 | A |
| 183 | 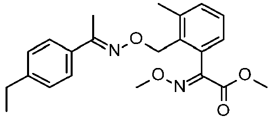 | 2.251 | 383 | A |
| 184 | 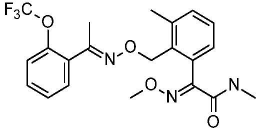 | 2.123 | 438 | A |
| 185 | 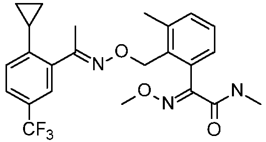 | 2.23 | 462 | A |
| 186 | 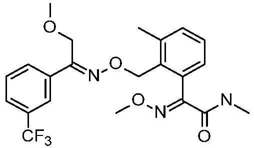 | 2.112 | 452 | A |
| 187 | 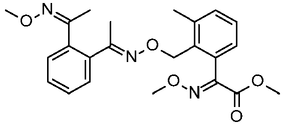 | 2.027 | 426 | A |
| 188 | 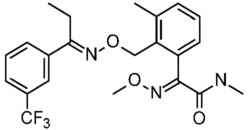 | 2.24 | 437 | A |
| 189 | 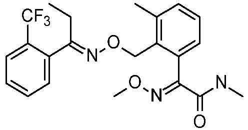 | 2.144 | 436 | A |
| 190 | 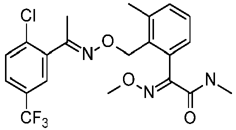 | 2.187 | 456 | A |
| 191 | 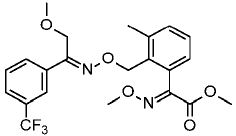 | 2.229 | 453 | A |
| 192 | 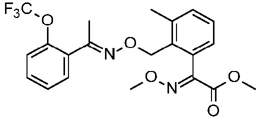 | 2.24 | 439 | A |
| 193 | 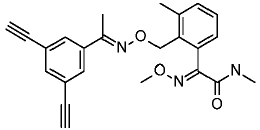 | 2.101 | 402 | A |
| 194 | 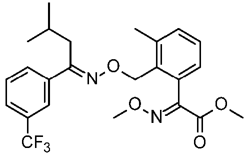 | 2.421 | 465 | A |
| 195 | 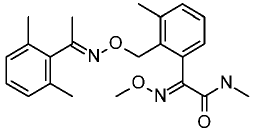 | 2.144 | 382 | A |
| 196 | 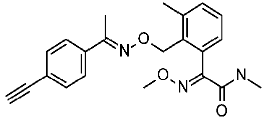 | 1.931 | 378 | A |
| 197 | 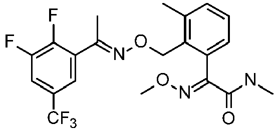 | 2.176 | 458 | A |
| 198 | 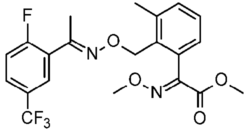 | 2.204 | 441 | A |
| 199 | 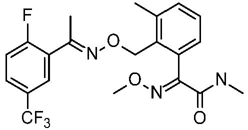 | 2.144 | 440 | A |
| 200 | 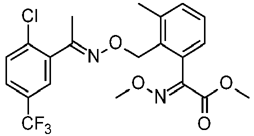 | 2.315 | 457 | A |
| 201 | 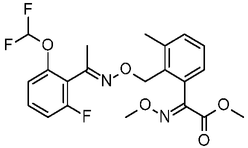 | 2.133 | 439 | A |
| 202 | 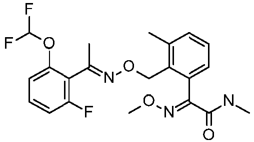 | 2.016 | 438 | A |
| 203 | 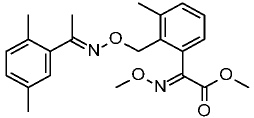 | 2.283 | 383 | A |
| 204 | 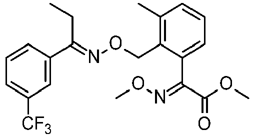 | 2.315 | 437 | A |
| 205 | 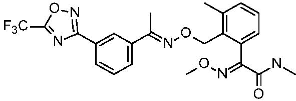 | 2.15 | 490 | A |
| 206 | 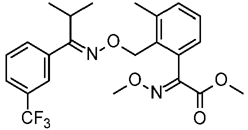 | 2.336 | 451 | A |
| 207 | 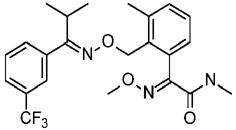 | 2.229 | 450 | A |
| 208 | 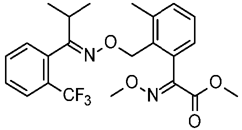 | 2.219 | 452 | A |
| 209 | 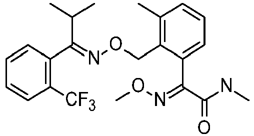 | 2.187 | 450 | A |
| 210 | 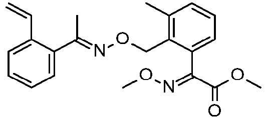 | 2.219 | 381 | A |
| 211 | 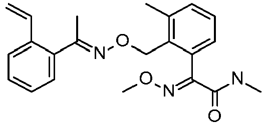 | 2.091 | 380 | A |
| 212 | 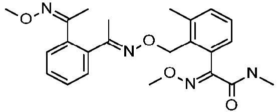 | 1.952 | 425 | A |
| 213 | 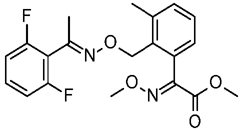 | 2.123 | 391 | A |
| 214 | 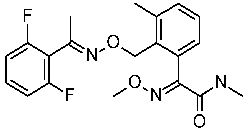 | 1.947 | 391 | A |
| 215 | 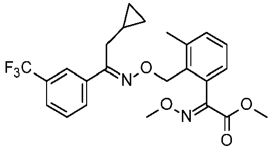 | 2.357 | 463 | A |
| 216 | 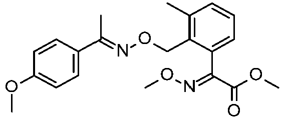 | 2.048 | 385 | A |
| 217 | 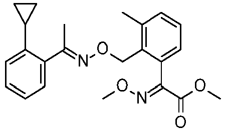 | 2.208 | 395 | A |
| 218 | 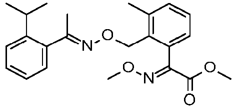 | 2.261 | 397 | A |
| 219 | 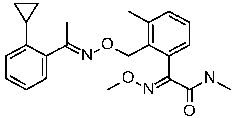 | 2.101 | 394 | A |
| 220 | 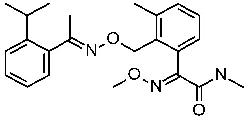 | 2.155 | 396 | A |
| 221 | 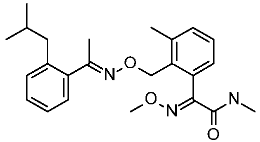 | 2.251 | 410 | A |
| 222 | 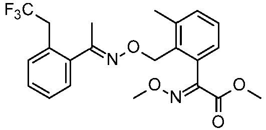 | 2.165 | 437 | A |
| 223 | 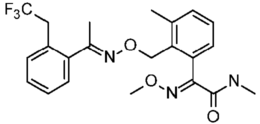 | 2.048 | 436 | A |
| 224 | 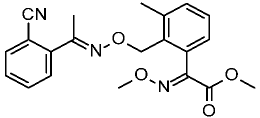 | 1.963 | 380 | A |
| 225 | 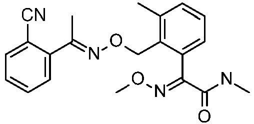 | 1.853 | 379 | A |
| 226 | 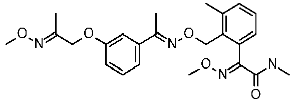 | 2.069 | 455 | A |
| 227 | 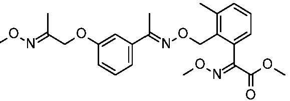 | 2.187 | 456 | A |
| 228 | 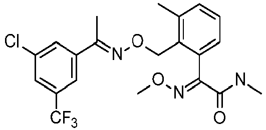 | 2.25 | 456 | A |
| 229 | 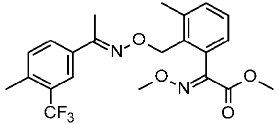 | 2.24 | 437 | A |
| 230 | 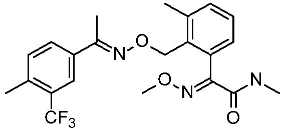 | 2.155 | 436.3 | A |
| 231 | 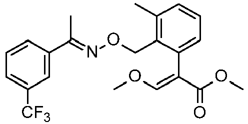 | 2.16 | 422 | A |
| 232 | 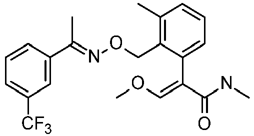 | 2.165 | 421 | A |
| 233 | 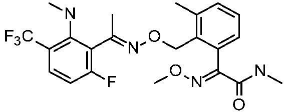 | 2.21 | 469 | A |
| 234 | 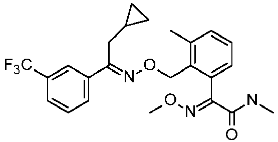 | 2.251 | 462 | A |
| 235 | 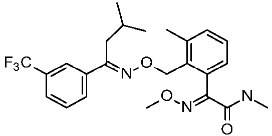 | 2.251 | 465 | A |
| 236 | 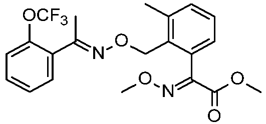 | 2.24 | 439 | A |
| 237 | 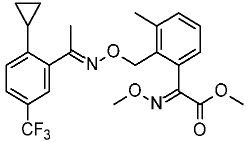 | 2.325 | 463 | A |
| 238 | 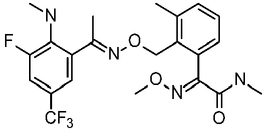 | 2.165 | 469 | A |
| 239 | 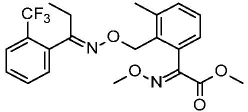 | 2.315 | 437 | A |
| 240 | 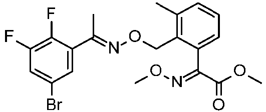 | 2.315 | 469 | A |
| 241 | 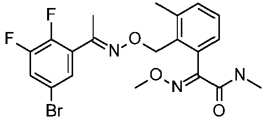 | 2.208 | 468 | A |
| 242 | 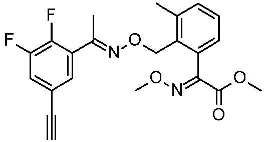 | 2.219 | 415 | A |
| 243 | 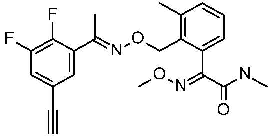 | 2.112 | 414 | A |
| 244 | 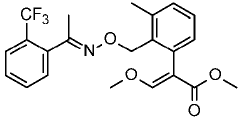 | 2.18 | 422 | A |
| 245 | 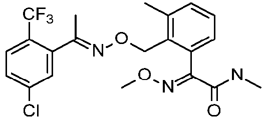 | 2.176 | 456 | A |
| 246 | 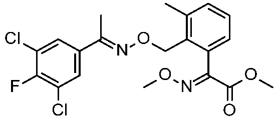 | 2.4 | 441 | A |
| 247 | 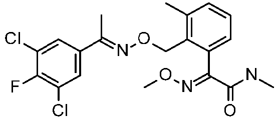 | 2.283 | 440 | A |
| 248 | 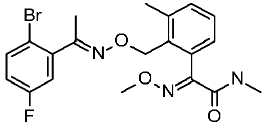 | 2.048 | 452 | A |
| 249 | 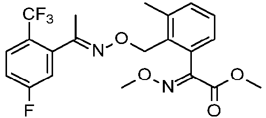 | 2.133 | 441 | A |
| 250 | 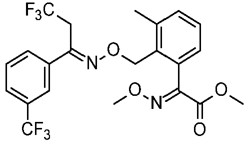 | 2.251 | 491 | A |
| 251 | 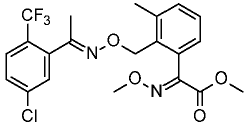 | 2.197 | 457 | A |
| 252 | 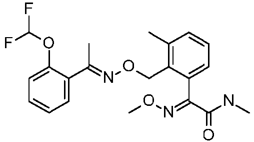 | 1.963 | 420 | A |
| 253 | 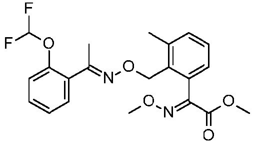 | 208 | 421 | A |
| 254 | 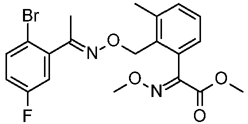 | 2.176 | 453 | A |
| 255 | 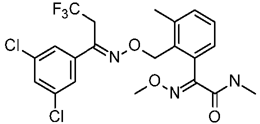 | 2.229 | 490 | A |
| 256 | 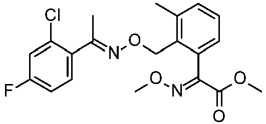 | 2.155 | 407 | A |
| 257 | 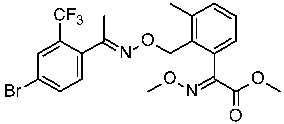 | 2.251 | 503 | A |
| 258 | 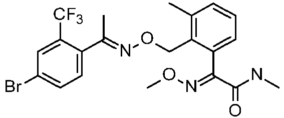 | 2.155 | 502 | A |
| 259 | 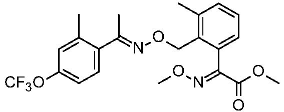 | 2.251 | 453 | A |
| 260 | 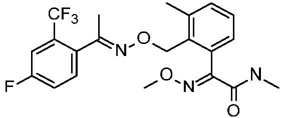 | 2.059 | 440 | A |
| 261 | 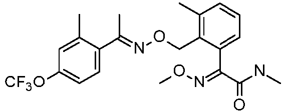 | 2.165 | 452 | A |
| 262 | 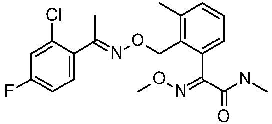 | 2.034 | 406 | A |
| 263 | 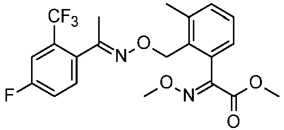 | 2.144 | 441 | A |
| 264 | 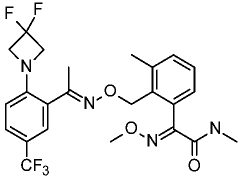 | 2.144 | 513 | A |
| 265 | 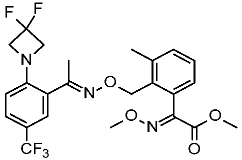 | 2.229 | 514 | A |
| 266 | 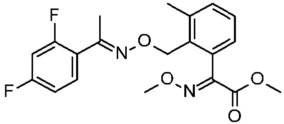 | 2.069 | 391 | A |
| 267 | 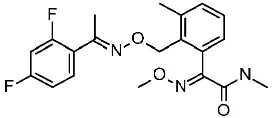 | 390 | 2.005 | A |
| 268 | 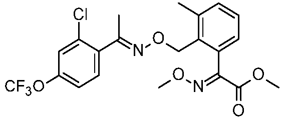 | 2.283 | 473 | A |
| 269 | 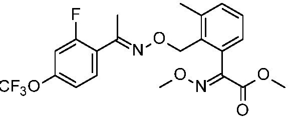 | 2.229 | 457 | A |
| 270 | 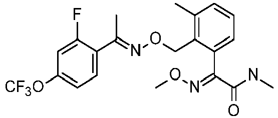 | 2.144 | 456 | A |
| 271 | 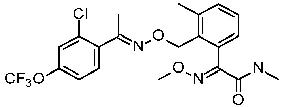 | 2.176 | 472 | A |
| 272 | 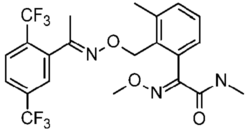 | 2.123 | 490 | A |
| 273 | 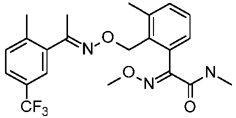 | 2.123 | 436 | A |
| 274 | 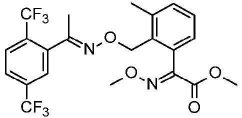 | 2.219 | 491 | A |
| 275 | 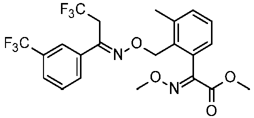 | 2.165 | 491 | A |
| 276 | 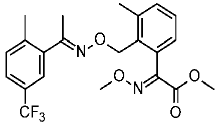 | 2.219 | 437 | A |
| 277 | 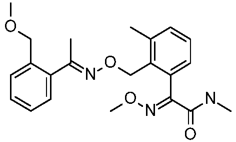 | 1.952 | 398 | A |
| 278 | 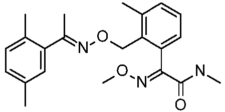 | 2.155 | 382 | A |
| 279 | 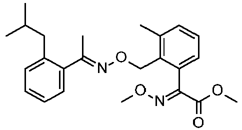 | 2.347 | 411 | A |
| 280 | 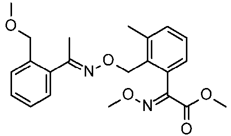 | 2.06 | 399 | A |
| 281 | 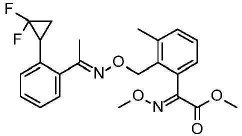 | 2.176 | 431 | A |
| 282 | 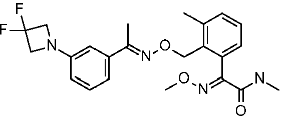 | 1.99 | 445.9 | A |
| 283 | 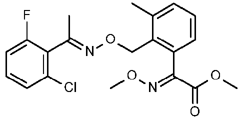 | 2.12 | 407 | A |
| 284 | 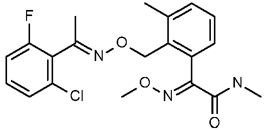 | 2.0 | 406 | A |
| 285 | 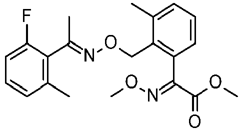 | 2.16 | 387 | A |
| 286 | 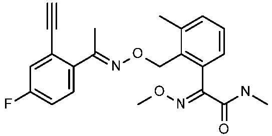 | 2.02 | 396 | A |
| 287 | 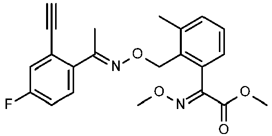 | 2.14 | 397 | A |
| 288 | 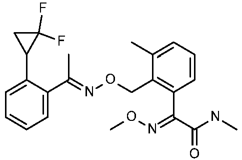 | 2.02 | 430 | A |
| 289 | 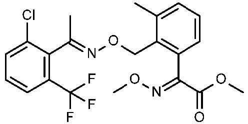 | 2.20 | 457 | A |
| 290 | 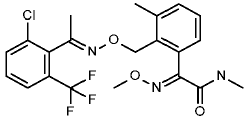 | 2.1 | 456 | A |
| 291 | 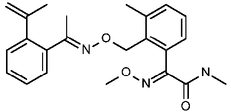 | 1.95 | 394 | A |
| 292 | 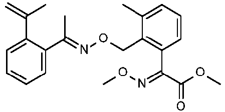 | 2.25 | 395 | A |
| 293 | 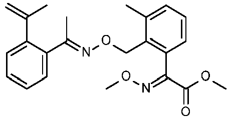 | 2.02 | 386 | A |
| 294 | 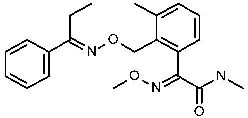 | 2.05 | 369 | A |
| 295 | 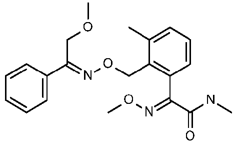 | 1.94 | 384 | A |
| 296 | 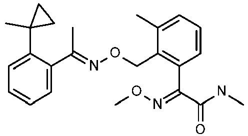 | 2.18 | 408 | A |
| 297 | 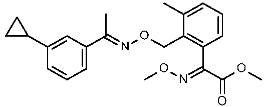 | 2.20 | 395 | A |
| 298 | 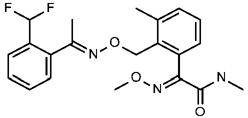 | 1.98 | 404 | A |
| 299 | 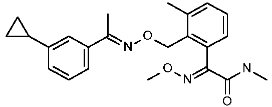 | 2.14 | 394 | A |
| 300 | 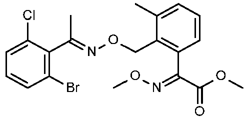 | 2.22 | 469 | A |
| 301 | 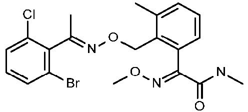 | 2.1 | 468 | A |
| 302 | 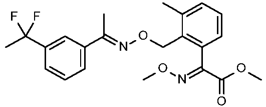 | 2.16 | 419 | A |
| 303 | 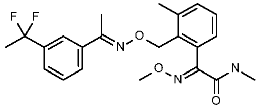 | 2.04 | 418 | A |
| 304 | 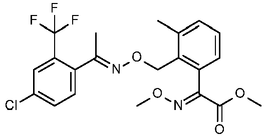 | 1.416 | 456.8 | A |
| 305 | 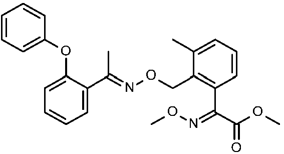 | 1.95 | 447 | B |
| 306 | 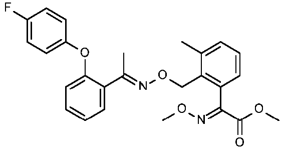 | 1.96 | 465 | B |
| 307 | 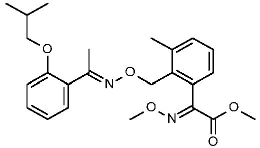 | 1.99 | 427 | B |
| 308 | 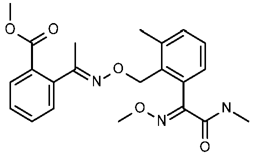 | 1.64 | 412 | B |
| 309 | 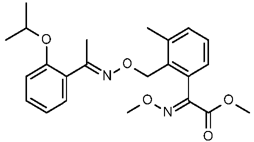 | 1.9 | 413 | B |
| 310 | 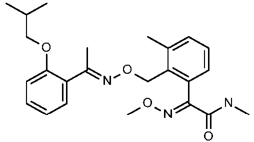 | 1.9 | 426 | B |
| 311 | 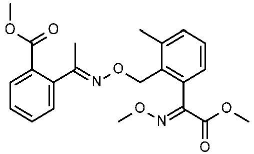 | 1.74 | 413 | B |
| 312 | 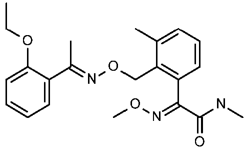 | 1.76 | 398 | B |
| 313 | 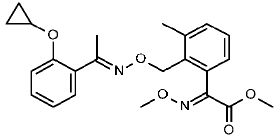 | 1.88 | 411 | B |
| 314 | 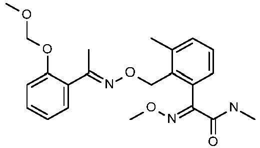 | 1.69 | 414 | B |
| 315 | 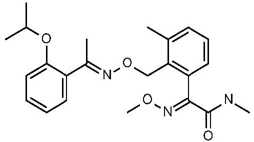 | 1.82 | 412 | B |
| 316 | 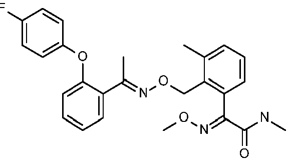 | 464 | 1.86 | B |
| 317 | 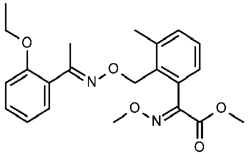 | 1.86 | 399 | B |
| 318 | 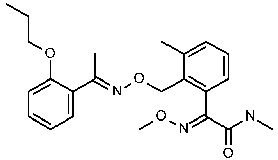 | 1.83 | 412 | B |
| 319 | 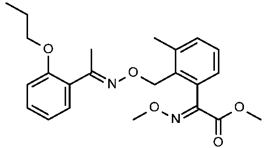 | 1.93 | 413 | B |
| 320 | 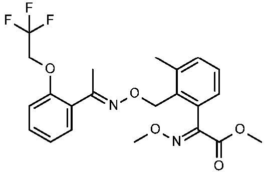 | 1.86 | 453 | B |
| 321 | 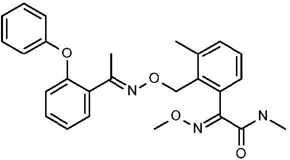 | 1.87 | 446 | B |
| 322 | 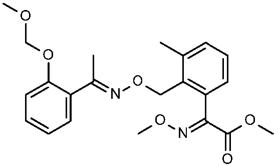 | 1.8 | 415 | B |
| 323 | 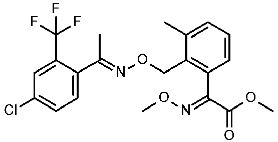 | 1.386 | 456.7 | A |
| 324 | 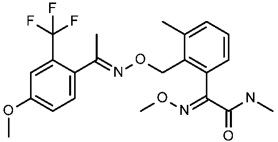 | 1.79 | 452 | B |
| 325 | 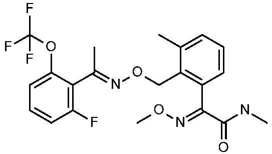 | 1.64 | 456 | B |
| 326 | 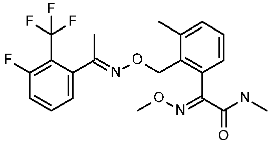 | 1.77 | 440 | B |
| 327 | 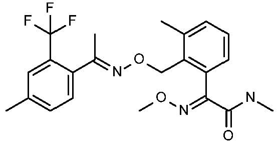 | 1.83 | 436 | B |
| 328 | 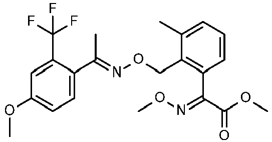 | 1.88 | 453 | B |
| 329 | 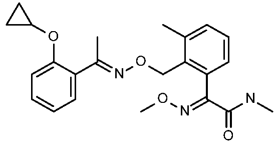 | 1.78 | 410 | B |
| 330 | 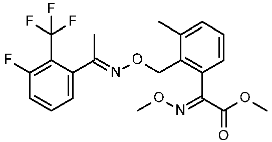 | 1.86 | 441 | B |
| 331 | 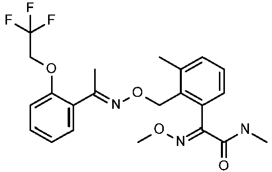 | 1.77 | 452 | B |
| 332 | 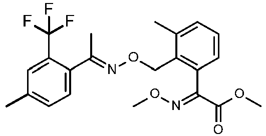 | 1.93 | 437 | B |
| 333 | 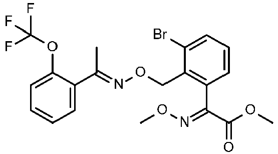 | 2.25 | 503 | A |
| 334 | 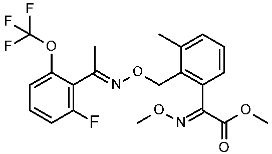 | 1.9 | 457 | B |
| 335 | 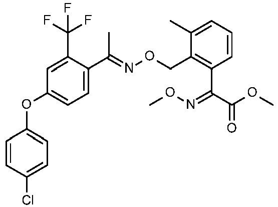 | 1.53 | 549 | A |
| 336 | 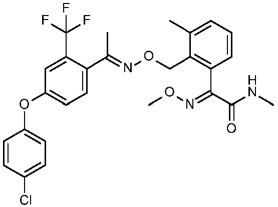 | 1.458 | 548.1 | A |
| 337 | 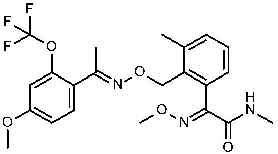 | 1.67 | 468 | B |
| 338 | 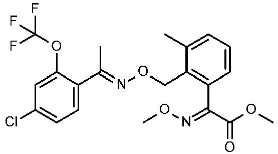 | 2 | 473 | B |
| 339 | 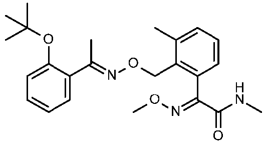 | 1.85 | 426 | B |
| 340 | 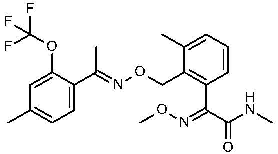 | 1.7 | 452 | B |
| 341 | 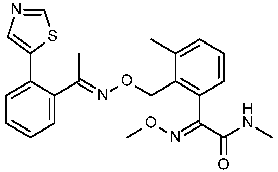 | 1.65 | 437 | B |
| 342 | 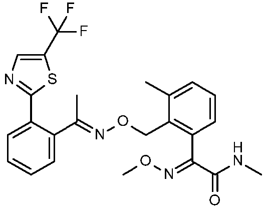 | 1.88 | 505 | B |
| 343 | 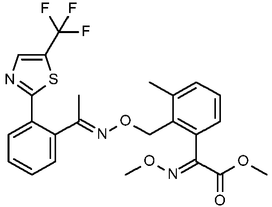 | 1.95 | 506 | B |
| 344 | 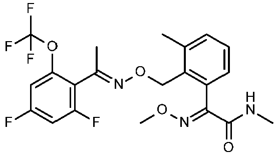 | 1.68 | 474 | B |
| 345 | 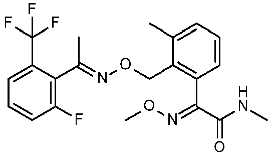 | 1.6 | 440 | B |
| 346 | 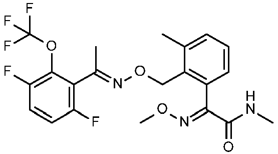 | 1.82 | 474 | B |
| 347 | 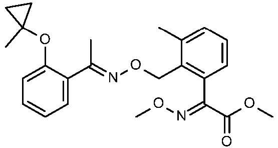 | 1.92 | 355 | B |
| 348 | 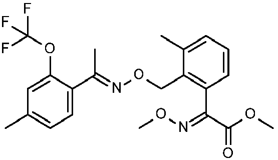 | 1.97 | 453 | B |
| 349 | 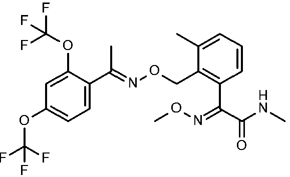 | 3.04 | 522 | C |
| 350 | 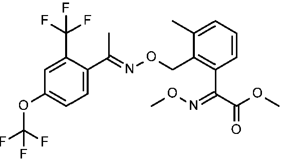 | 1.99 | 507 | B |
| 351 | 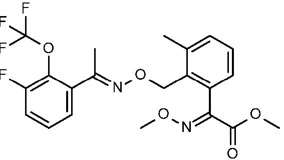 | 1.92 | 457 | B |
| 352 | 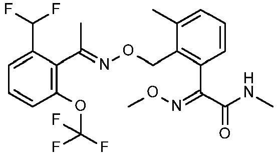 | 1.84 | 488 | B |
| 353 | 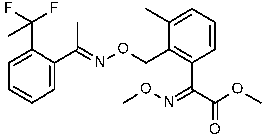 | 1.86 | 419 | B |
| 354 | 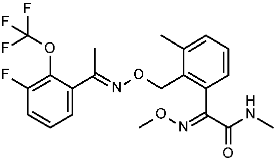 | 1.82 | 456 | B |
| 355 | 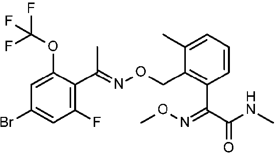 | 2.97 | 535 | C |
| 356 | 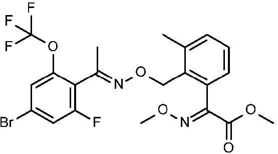 | 3.13 | 536 | C |
| 357 | 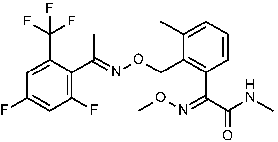 | 1.62 | 458 | B |
| 358 | 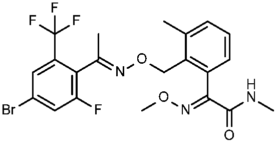 | 2.93 | 519 | C |
| 359 | 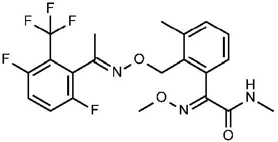 | 1.95 | 459 | B |
| 360 | 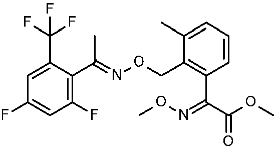 | 1.73 | 459 | B |
| 361 | 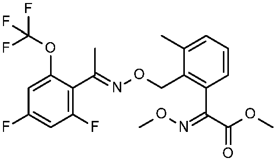 | 1.76 | 475 | B |
| 362 | 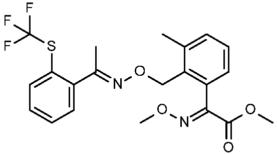 | 1.93 | 455 | B |
| 363 | 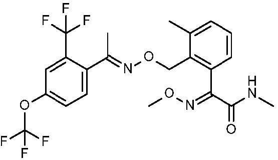 | 1.89 | 506 | B |
| 364 | 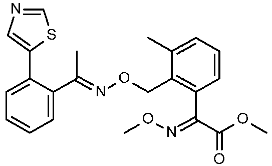 | 1.74 | 438 | A |
| 365 | 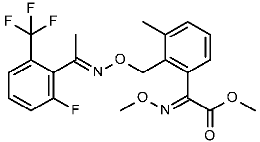 | 1.7 | 441 | B |
| 366 | 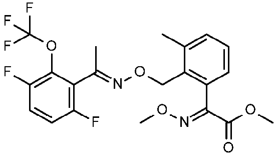 | 1.9 | 475 | B |
| 367 | 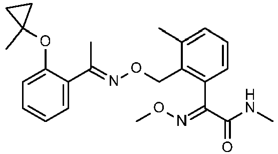 | 1.84 | 354 | B |
| 368 | 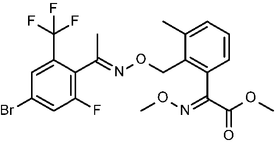 | 3.09 | 520 | C |
| 369 | 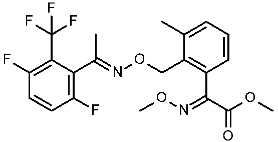 | 1.87 | 458 | B |
| 370 | 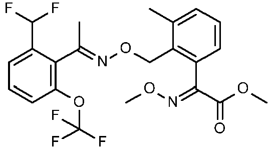 | 1.94 | 489 | B |
| 371 | 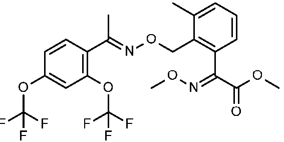 | 3.79 | 523 | C |
| 372 | 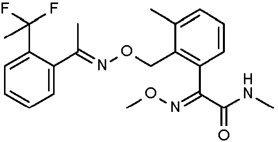 | 1.76 | 418 | B |
| 373 | 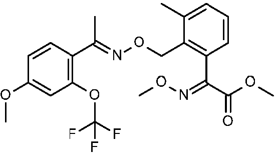 | | | |
| 374 | 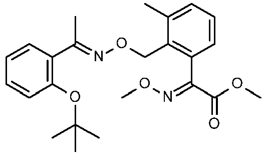 | 1.94 | 427 | B |
| 375 | 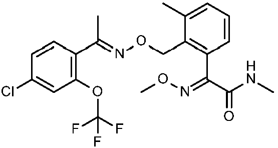 | 1.91 | 472 | B |
| 376 | 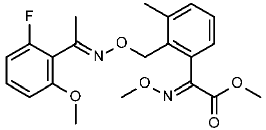 | 2.07 | 403 | A |
| 377 | 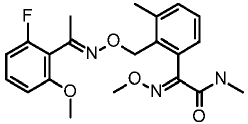 | 1.95 | 402 | A |
| 378 | 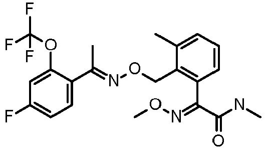 | 1.67 | 456 | B |
| 379 | 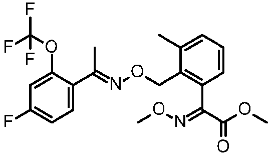 | 2.2 | 457 | A |
| 380 | 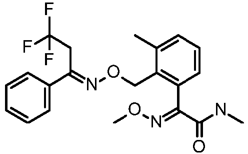 | 2.04 | 422 | A |
| 381 | 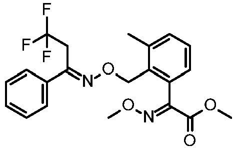 | 2.13 | 423 | A |
| 382 | 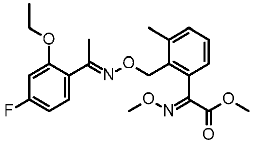 | 2.2 | 417 | A |
| 383 | 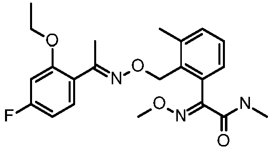 | 2.07 | 416 | A |
| 384 | 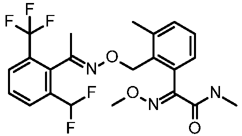 | 1.67 | 472 | B |
| 385 | 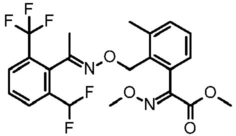 | 1.78 | 473 | B |
| 386 | 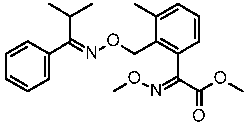 | 2.24 | 383 | A |
| 387 | 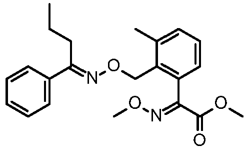 | 2.25 | 383 | A |
| 388 | 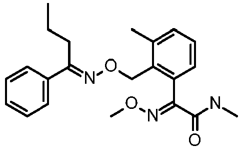 | 2.14 | 382 | A |
| 389 | 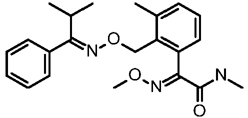 | 2.11 | 382 | A |
| 390 | 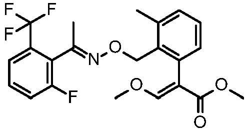 | 2.18 | 440 | A |
| 391 | 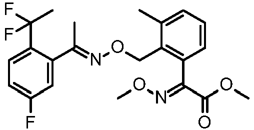 | 2.15 | 437 | A |
| 392 | 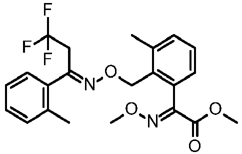 | 2.16 | 437 | A |
| 393 | 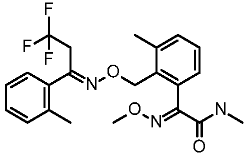 | 2.03 | 436 | A |
| 394 | 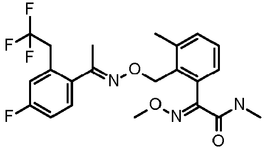 | 2.08 | 454 | A |
| 395 | 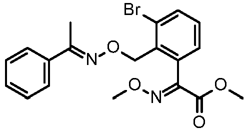 | 2.19 | 421 | A |
| 396 | 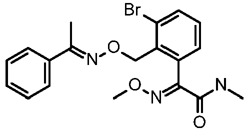 | 2.05 | 420 | A |
| 397 | 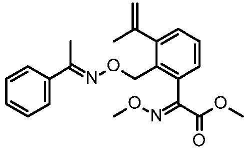 | 2.23 | 381 | A |
| 398 | 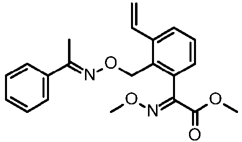 | 2.18 | 367 | A |
| 399 | 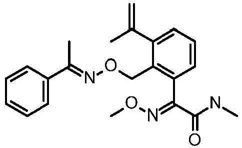 | 2.03 | 380 | A |
| 400 | 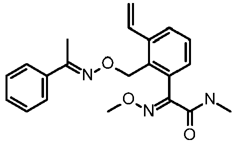 | 1.99 | 366 | A |
| 401 | 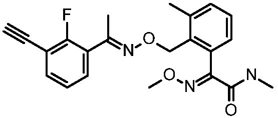 | 2.03 | 396 | A |
| 402 | 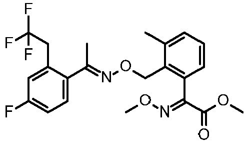 | 2.197 | 455 | A |
| 403 | 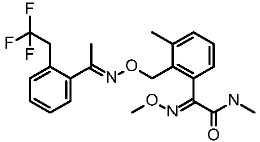 | 1.25 | 436 | A |
| 404 | 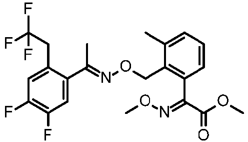 | 2.167 | 473 | A |
| 405 | 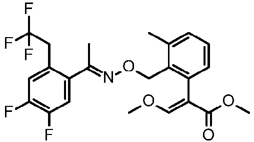 | 2.22 | 472 | A |
| 406 | 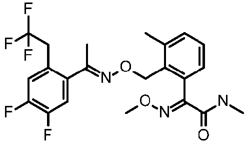 | 2.12 | 472 | A |
| 407 | 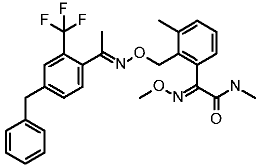 | 2.26 | 512 | A |
| 408 | 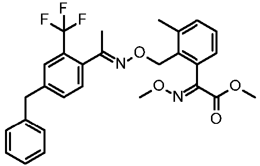 | 2.29 | 513 | A |
| 409 | 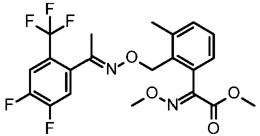 | 2.21 | 459 | A |
| 410 | 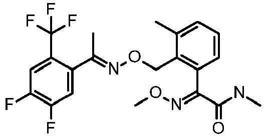 | 2.04 | 458 | A |
| 411 | 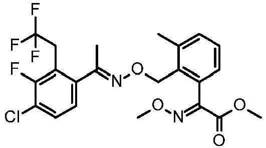 | 2.24 | 489 | A |
| 412 | 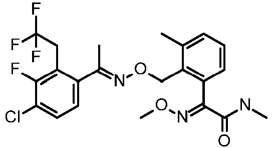 | 2.13 | 488 | A |
| 413 | 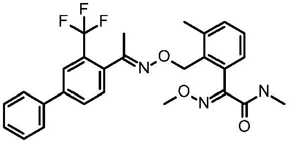 | 2.25 | 498 | A |
| 414 | 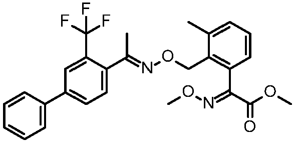 | 2.34 | 499 | A |
| 415 | 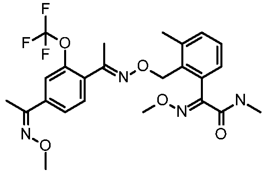 | 2.18 | 509 | A |
| 416 | 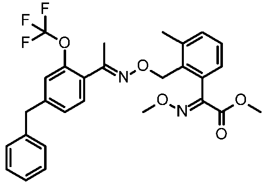 | 2.27 | 529 | A |
| 417 | 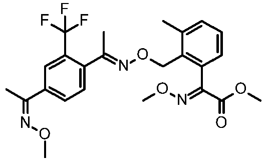 | 2.24 | 494 | A |
| 418 | 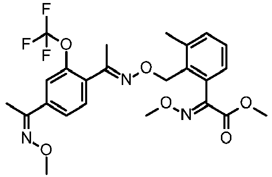 | 2.28 | 510 | A |
| 419 | 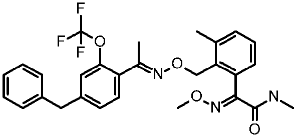 | 2.106 | 528 | A |
| 420 | 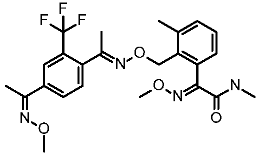 | 2.02 | 493 | A |
| 421 | 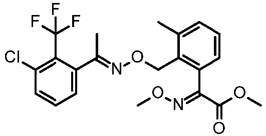 | 2.18 | 457 | A |
| 422 | 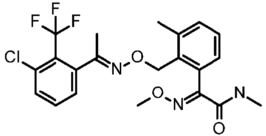 | 2.12 | 456 | A |
| 423 | 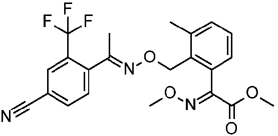 | 2.03 | 448 | A |
| 424 | 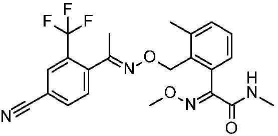 | 1.898 | 447 | A |
| 425 | 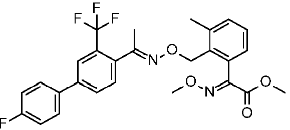 | 2.26 | 517 | A |
| 426 | 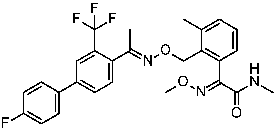 | 2.15 | 516 | A |
| 427 | 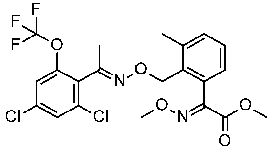 | 1.86 | 507 | B |
| 428 | 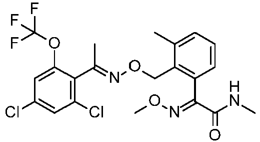 | 1.75 | 506 | B |
| 429 | 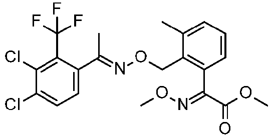 | 2.27 | 491 | A |
| 430 | 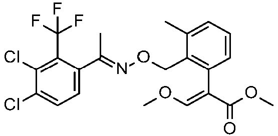 | 2.3 | 490 | A |
| 431 | 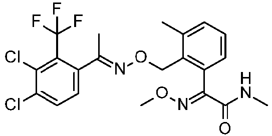 | 2.17 | 490 | A |
| 432 | 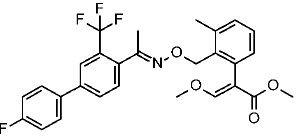 | 2.33 | 516 | A |
| 433 | 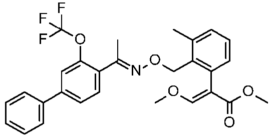 | 2.4 | 514 | A |
| 434 | 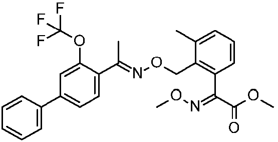 | 2.33 | 515 | A |
| 435 | 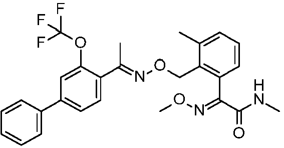 | 2.15 | 514 | A |
| 436 | 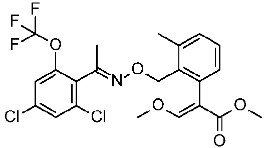 | 1.86 | 506 | B |
| 437 | 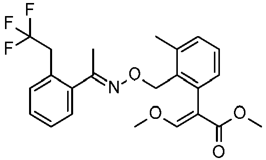 | 1.71 | 436 | B |
| 438 | 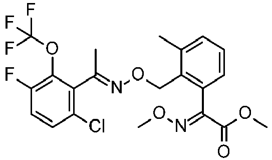 | 1.77 | 491 | B |
| 439 | 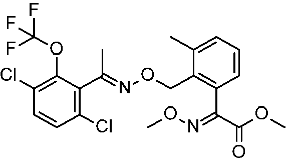 | 1.82 | 507 | B |
| 440 | 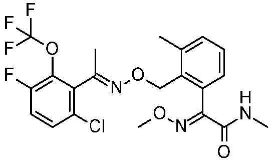 | 1.66 | 490 | B |
| 441 | 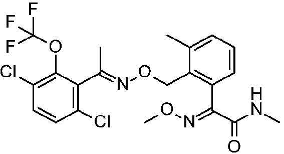 | 1.71 | 506 | B |
| 442 | 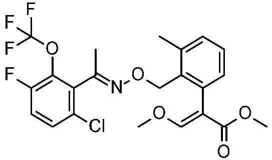 | 1.77 | 490 | B |
| 443 | 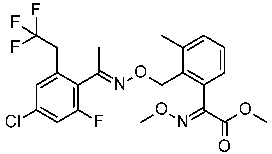 | 1.72 | 489 | B |
| 444 | 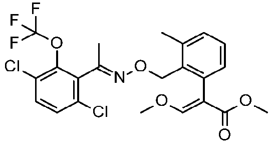 | 1.83 | 506 | B |
| 445 | 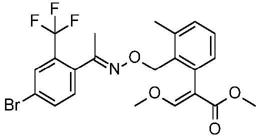 | 2.23 | 500 | A |
| 446 | 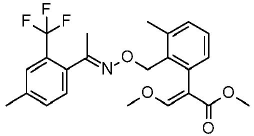 | 2.12 | 436 | A |
| 447 | 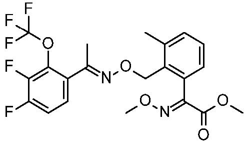 | 1.87 | 475 | B |
| 448 | 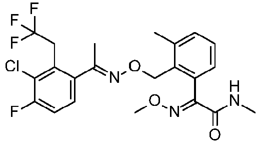 | 1.75 | 488 | B |
| 449 | 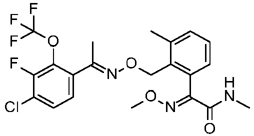 | 1.8 | 490 | B |
| 450 | 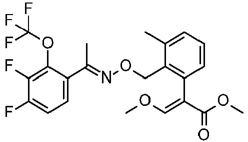 | 1.89 | 474 | B |
| 451 | 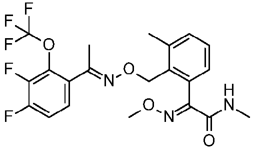 | 1.78 | 474 | B |
| 452 | 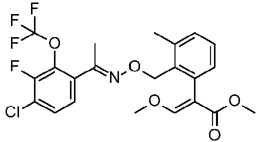 | 1.91 | 490 | B |
| 453 | 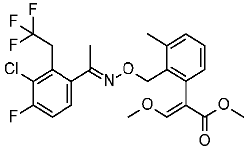 | 1.85 | 488 | B |
| 454 | 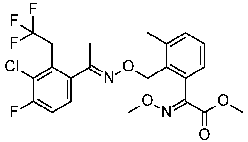 | 1.83 | 489 | B |
| 455 | 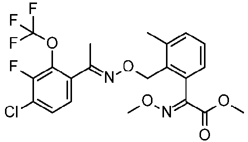 | 1.9 | 491 | B |
| 456 | 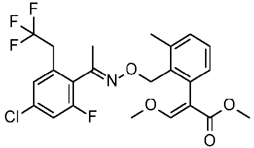 | 1.81 | 488 | B |
| 457 | 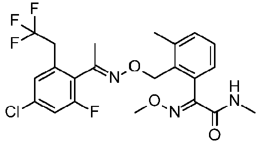 | 1.72 | 488 | B |
| 458 | 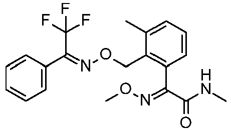 | 2.04 | 408 | A |
| 459 | 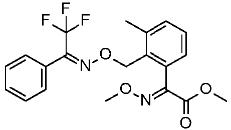 | 2.16 | 409 | A |
| 460 | 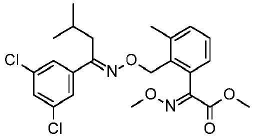 | 2.64 | 465 | A |
| 461 | 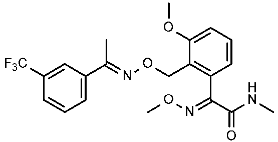 | 2.00 | 438 | A |
| 462 | 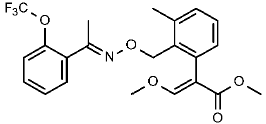 | 2.21 | 438 | A |
| 463 | 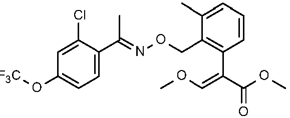 | 2.31 | 472 | A |
| 464 | 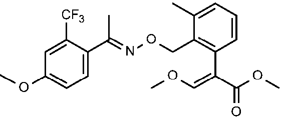 | 2.10 | 452 | A |

### Biological studies

### Green House and detached leaf tests

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml. This stock solution was then diluted with the described solvent-emulsifier-water mixture to the final concentration given in the table below.

### Use example 1. Curative control of soybean rust on soybeans caused by Phakopsora pachyrhizi (PHAKPA K4)

Leaves of potted soybean seedlings were inoculated with spores of *Phakopsora pachyrhizi.* The strain used contains the amino acid substitution F129L in the mitochondrial cytochrome b protein conferring resistance to Qo inhibitors. To ensure the success of the artificial inoculation, the plants were transferred to a humid chamber with a relative humidity of about 95% and 20 to 24 °C for 24 hr. The next day the plants were cultivated for 3 days in a greenhouse chamber at 23 to 27 °C and a relative humidity between 60 and 80 %. Then the plants were sprayed to run-off with the previously described spray solution, containing the concentration of active ingredient or their mixture as described below. The plants were allowed to air-dry. Then the trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 2. Protective control of soybean rust on soybeans caused by Phakopsora pachyrhizi (PHAKPA P2)

Leaves of potted soybean seedlings were sprayed to run-off with the previously described spray solution, containing the concentration of active ingredient or their mixture as described below. The plants were allowed to air-dry. The trial plants were cultivated for 2 days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 20 to 24 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 3. Protective control of soybean rust on soybeans caused by Phakopsora pachyrhizi (PHAKPA P6)

Leaves of potted soybean seedlings were sprayed to run-off with the previously described spray solution, containing the concentration of active ingredient as described below. The plants were allowed to air-dry. The trial plants were cultivated for six days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 23 to 27 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 4. Protective control of soybean rust on detached soybean leaves caused by Phakopsora pachyrhizi (PHAKPA P1 DL)

Leaves of potted soybean seedlings were sprayed to run-off with the previously described spray solution, containing the concentration of active ingredient as described below. The plants were left for drying in a green house chamber at 20 °C and 14 hours lightning over night. The next day, leaves were harvested and placed on water agar plates. Subsequently, the leaves were inoculated with spores of *Phakopsora pachyrhizi.* Two different isolates were used: one being sensitive to Qo inhibitors (wt); and one which contains the amino acid substitution F129L in the mitochondrial cytochrome b protein conferring resistance to Qo inhibitors (F129L). Inoculated leaves were incubated for 16 to 24 h at room temperature in a dark dust chamber, followed by incubation for 2 to 3 weeks in an incubator at 20 °C and 12 hours light/day. The extent of fungal attack on the leaves was visually assessed as % diseased leaf area.

### Micro titer plate tests

The active compounds were formulated separately as a stock solution having a concentration of 10,000 ppm in dimethyl sulfoxide. The stock solutions were mixed according to the ratio, pipetted onto a micro titer plate (MTP) and diluted with water to the stated concentrations.

After addition of the respective spore suspension as indicated in the different use examples below, plates were placed in a water vapor-saturated chamber at a temperature of 18°C. Using an absorption photometer, the MTPs were measured at 405 nm 7 days after the inoculation. The measured parameters were compared to the growth of the active compound-free control variant (100%) and the fungus-free blank value to determine the relative growth in % of the pathogens in the respective active compounds.

### Use example 5. Activity against Pyricularia oryzae causing rice blast (PYRIOR)

A spore suspension of *Pyricularia oryzae* in an aqueous biomalt or yeast-bactopeptone-glycerine or DOB solution was used.

### Use example 6. Activity against Septoria tritici causing leaf blotch on wheat (SEPTTR)

A spore suspension of *Septoria tritici* in an aqueous biomalt or yeast-bactopeptone-glycerine or DOB solution was used.

### Use example 7. Activity against Colletotrichum orbiculare causing anthracnose (COLLLA)

A spore suspension of *Colletotrichum orbiculare* in an aqueous 2% malt solution was used.

### Use example 8. Activity against Leptosphaeria nodorum causing wheat leaf spots (LEPTNO)

A spore suspension of *Leptosphaeria nodorum* in an aqueous biomalt or yeast-bactopeptone-glycerine or DOB solution was used.

### Use example 9. Activity against Alternaria solani causing early blight (ALTESO, wt and F129L)

Two different spore suspensions of *Alternaria solani* in an aqueous biomalt or yeast-bactopeptone-glycerine or DOB solution were used: a sensitive wild-type isolate (wt) and a Qo inhibitor-resistant isolate containing the amino acid substitution F129L in the mitochondrial cytochrome b protein conferring resistance to Qo inhibitors (F129L).

### Use example 10. Activity against Pyrenophora teres causing net blotch on barley (PYRNTE, wt and F129L)

Two different spore suspensions of *Pyrenophora teres* in an aqueous biomalt or yeast-bactopeptone-glycerine or DOB solution were used: a sensitive wild-type isolate (wt) and a Qo inhibitor-resistant isolate containing the amino acid substitution F129L in the mitochondrial cytochrome b protein conferring resistance to Qo inhibitors (F129L).

### Use example 11. Activity against Cercospora sojina causing frogeye leaf spot of soybeans (CERCSO)

A spore suspension of *Cercospora sojina* in an aqueous biomalt or yeast-bactopeptone-glycerine or DOB solution was then added.

### Use example 12. Activity against Microdochium nivale causing snow mould (MONGNI)

A spore suspension of *Microdochium nivale* in an aqueous biomalt or yeast-bactopeptone-glycerine or DOB solution was used.

The results of the abovementioned use examples are given in the following Tables.

The test results in Tables 1 and C1 to C4 below are given for the control of phytopathogenic fungi containing the amino acid substitution F129L in the mitochondrial cytochrome b protein conferring resistance to Qo inhibitors.

**Table 1:**

| **Treatment with compound** | | **% PHAKPA (F129L) Disease level** | | | |
|---|---|---|---|---|---|
| **No.** | **Structure** | **P2 at 4 ppm** | **P2 at 16 ppm** | **P6 at 4 ppm** | **P6 at 16 ppm** |
| 1 | 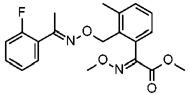 | 80 | 27 | 90 | 56 |
| 2 | 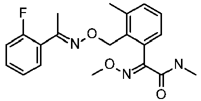 | 5 | 0 | 26 | 1 |
| 3 | 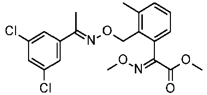 | 30 | 3 | 40 | 6 |
| 4 | 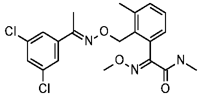 | 2 | 0 | 13 | 1 |
| 5 | 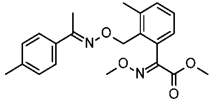 | 28 | 1 | 50 | 4 |
| 6 | 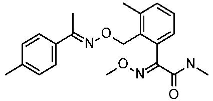 | 1 | 0 | 19 | 1 |
| 8 | 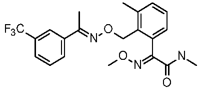 | 4 | 0 | 5 | 0 |
| 9 | 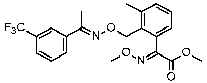 | 35 | 23 | 41 | 4 |
| 10 | 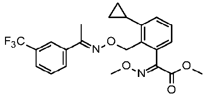 | 22 | 2 | 45 | 3 |
| 11 | 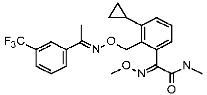 | 23 | 1 | 20 | 0 |
| 12 | 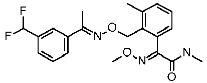 | 25 | 1 | 37 | 9 |
| 13 | 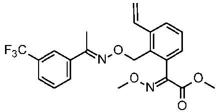 | 28 | 0 | 25 | 0 |
| 14 | 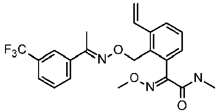 | 6 | 0 | 11 | 1 |
| 15 | 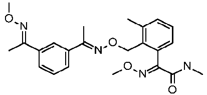 | 4 | 0 | 24 | 1 |
| 17 | 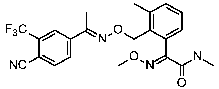 | 70 | 50 | 63 | 57 |
| 19 | 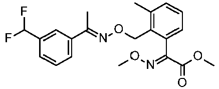 | 87 | 0 | 87 | 1 |
| 20 | 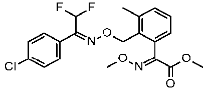 | 73 | 29 | 97 | 18 |
| 21 | 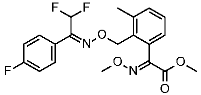 | 100 | 77 | 97 | 88 |
| 22 | 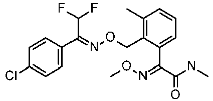 | 100 | 90 | 100 | 93 |
| 23 | 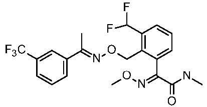 | 12 | 1 | 20 | 1 |
| 24 | 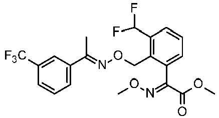 | 36 | 60 | 4 | 28 |
| 25 | 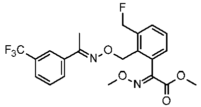 | 16 | 3 | 50 | 2 |
| 26 | 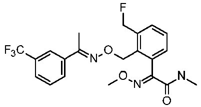 | 12 | 0 | 19 | 1 |
| 27 | 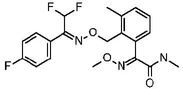 | 5 | 0 | 32 | 0 |
| 28 | 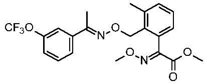 | | 0 | | 0 |
| 29 | 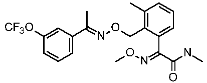 | | 0 | | 0 |
| 30 | 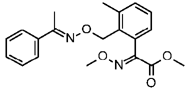 | 53 | 1 | 77 | 2 |
| 31 | 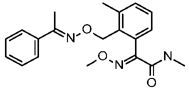 | 0 | 0 | 4 | 0 |
| 32 | 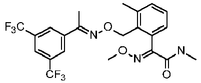 | 80 | 50 | 73 | 43 |
| 33 | 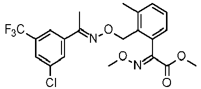 | 50 | 33 | 60 | 75 |
| 34 | 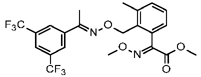 | 100 | 90 | 90 | 83 |
| 36 | 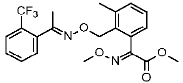 | 100 | 42 | 100 | 43 |
| 37 | 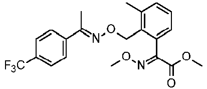 | 40 | 3 | 40 | 2 |
| 38 | 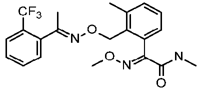 | 37 | 4 | 47 | 1 |
| 39 | 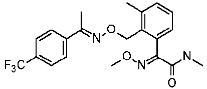 | 30 | 4 | 15 | 2 |
| 40 | 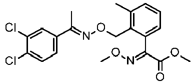 | 40 | 18 | 63 | 22 |
| 41 | 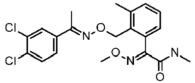 | 15 | 9 | 36 | 14 |
| 42 | 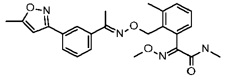 | 60 | 23 | 60 | 20 |
| 43 | 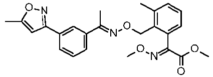 | 100 | 100 | 97 | 87 |
| 44 | 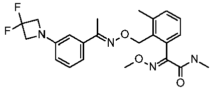 | 77 | 17 | 77 | 21 |
| 46 | 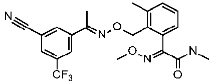 | 63 | 50 | 90 | 57 |
| 47 | 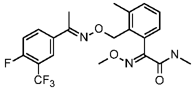 | 13 | 0 | 12 | 0 |
| 48 | 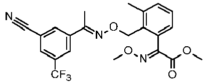 | 100 | 80 | 100 | 67 |
| 49 | 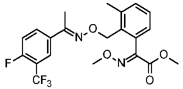 | 60 | 18 | 60 | 7 |
| 50 | 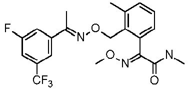 | 2 | 1 | 6 | 0 |
| 51 | 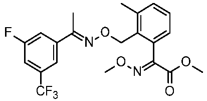 | 28 | 2 | 15 | 0 |
| 52 | 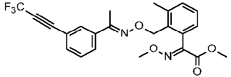 | 63 | 28 | 90 | 43 |
| 53 | 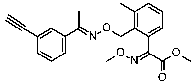 | 63 | 17 | 100 | 47 |
| 54 | 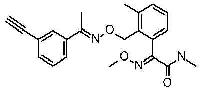 | 0 | 1 | 20 | 0 |
| 55 | 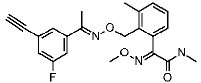 | 3 | 1 | 13 | 5 |
| 56 | 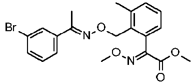 | 43 | 24 | 67 | 19 |
| 57 | 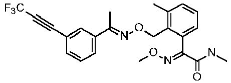 | 25 | 8 | 43 | 15 |
| 58 | 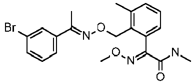 | 2 | 0 | 4 | 0 |
| 59 | 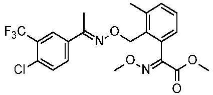 | 67 | 20 | 63 | 11 |
| 60 | 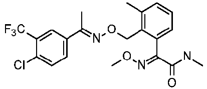 | 23 | 1 | 53 | 2 |
| 61 | 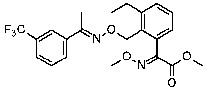 | 70 | 15 | 90 | 18 |
| 62 | 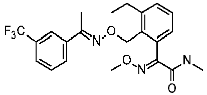 | 43 | 0 | 60 | 3 |
| 63 | 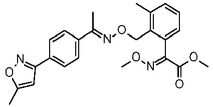 | 90 | 77 | 93 | 77 |
| 64 | 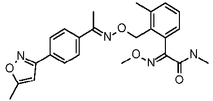 | 87 | 73 | 80 | 43 |
| 65 | 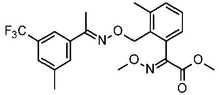 | 93 | 77 | 100 | 87 |
| 66 | 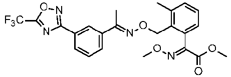 | 100 | 70 | 93 | 87 |
| 67 | 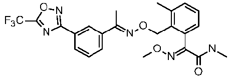 | 67 | 2 | 80 | 30 |
| 69 | 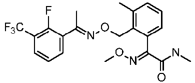 | 2 | 0 | 13 | 0 |
| 70 | 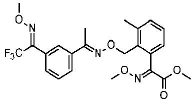 | 30 | 0 | 10 | 1 |
| 71 | 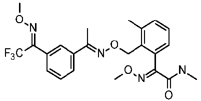 | 2 | 0 | 6 | 0 |
| 72 | 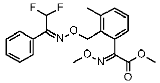 | 100 | 87 | 93 | 80 |
| 73 | 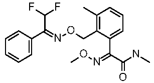 | 0 | | 19 | |
| 74 | 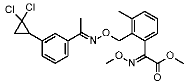 | 27 | 6 | 16 | 3 |
| 75 | 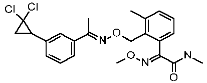 | 8 | 0 | 13 | 1 |
| 76 | 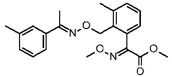 | 30 | 3 | 9 | 0 |
| 77 | 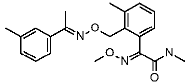 | 67 | 4 | 40 | 3 |
| 78 | 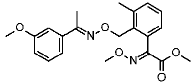 | 40 | 1 | 30 | 1 |
| 79 | 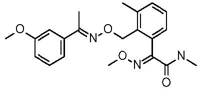 | 21 | 2 | 44 | 4 |
| 80 | 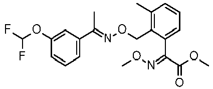 | 50 | 10 | 30 | 3 |
| 81 | 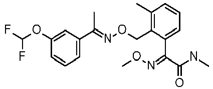 | 6 | 0 | 2 | 0 |
| 82 | 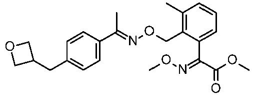 | 87 | 57 | | |
| 83 | 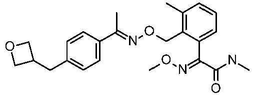 | 97 | 73 | | |
| 84 | 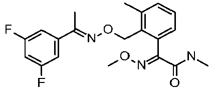 | 0 | 0 | 0 | 0 |
| 85 | 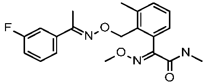 | 1 | 0 | 0 | 0 |
| 86 | 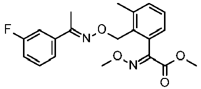 | 12 | 3 | 23 | 3 |
| 87 | 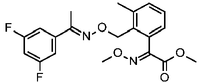 | 8 | 0 | 7 | 0 |
| 93 | 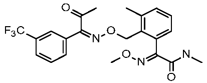 | 83 | 32 | 43 | 37 |
| 94 | 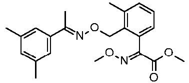 | 22 | 5 | 35 | 2 |
| 95 | 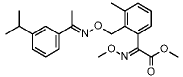 | 11 | 0 | 33 | 4 |
| 96 | 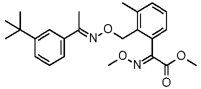 | 6 | 1 | 3 | 0 |
| 97 | 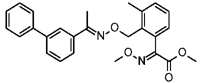 | 4 | 1 | 1 | 0 |
| 98 | 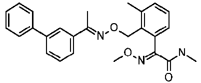 | 3 | 0 | 1 | 0 |
| 99 | 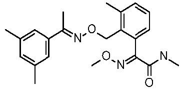 | 43 | 12 | 87 | 33 |
| 100 | 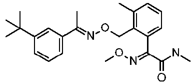 | 18 | 0 | 25 | 4 |
| 101 | 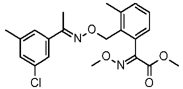 | 70 | 37 | 60 | 17 |
| 102 | 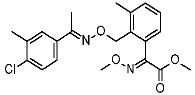 | 60 | 11 | 90 | 43 |
| 103 | 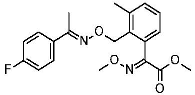 | 40 | 3 | 35 | 4 |
| 104 | 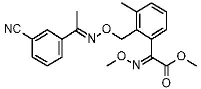 | 22 | 15 | 19 | 11 |
| 105 | 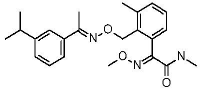 | 18 | 1 | 39 | 13 |
| 106 | 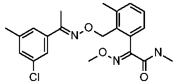 | 5 | 1 | 32 | 9 |
| 107 | 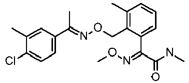 | 1 | 0 | 15 | 1 |
| 108 | 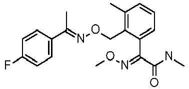 | 1 | 0 | 1 | 0 |
| 109 | 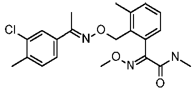 | 8 | 1 | 12 | 0 |
| 110 | 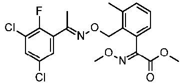 | 20 | 3 | 30 | 1 |
| 111 | 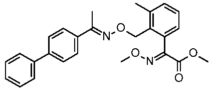 | 28 | 8 | 46 | 10 |
| 112 | 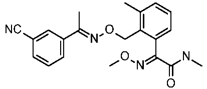 | 13 | 3 | 30 | 9 |
| 113 | 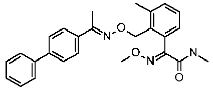 | 25 | 26 | 41 | 25 |
| 114 | 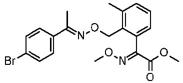 | 38 | 5 | 52 | 15 |
| 115 | 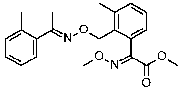 | 83 | 63 | 92 | 75 |
| 116 | 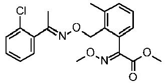 | 85 | 57 | 85 | 67 |
| 117 | 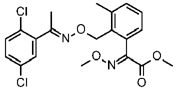 | 85 | 10 | 88 | 12 |
| 118 | 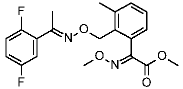 | 10 | 0 | 32 | 0 |
| 119 | 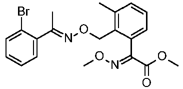 | 93 | 63 | 92 | 63 |
| 120 | 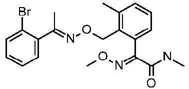 | 43 | 4 | 90 | 8 |
| 121 | 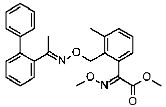 | 100 | 98 | 98 | 92 |
| 122 | 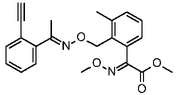 | 100 | 73 | 93 | 90 |
| 123 | 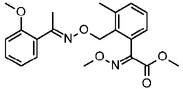 | 100 | 82 | 90 | 78 |
| 124 | 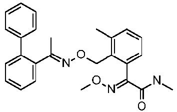 | 98 | 87 | 92 | 75 |
| 125 | 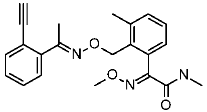 | 72 | 9 | 90 | 44 |
| 126 | 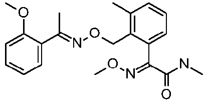 | 87 | 34 | 95 | 70 |
| 127 | 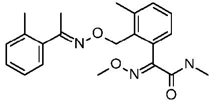 | 90 | 44 | 93 | 62 |
| 128 | 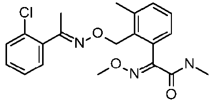 | 32 | 2 | 77 | 2 |
| 129 | 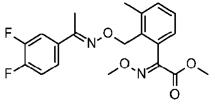 | 28 | 1 | 24 | 2 |
| 130 | 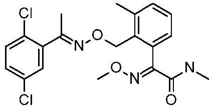 | 20 | 1 | 40 | 1 |
| 131 | 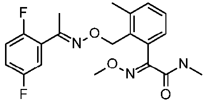 | 0 | 0 | 2 | 0 |
| 132 | 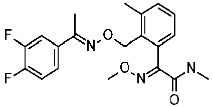 | 1 | 0 | 6 | 0 |
| 133 | 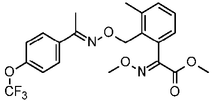 | 1 | 0 | 2 | 0 |
| 134 | 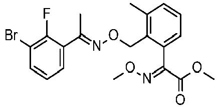 | 37 | 10 | 22 | 7 |
| 135 | 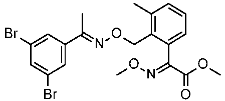 | 15 | 1 | 16 | 2 |
| 136 | 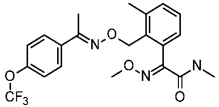 | 1 | 0 | 3 | 2 |
| 137 | 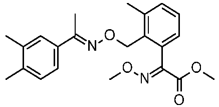 | 19 | 2 | 50 | 5 |
| 138 | 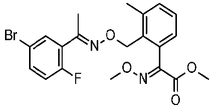 | 47 | 2 | 35 | 1 |
| 139 | 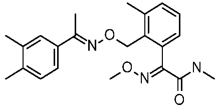 | 47 | 2 | 72 | 24 |
| 140 | 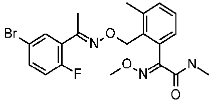 | 8 | 0 | 13 | 4 |
| 141 | 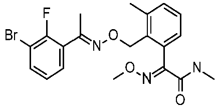 | 2 | 0 | 2 | 0 |
| 142 | 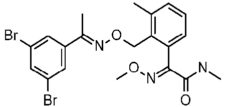 | 19 | 5 | 26 | 5 |
| 143 | 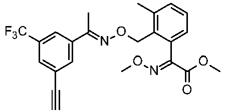 | 4 | 0 | 15 | 0 |
| 144 | 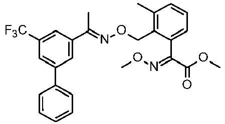 | 87 | 80 | 92 | 90 |
| 145 | 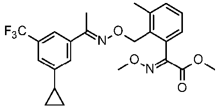 | 28 | 8 | 37 | 16 |
| 146 | 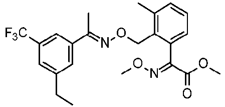 | 73 | 12 | 77 | 48 |
| 147 | 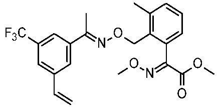 | 73 | 18 | 95 | 18 |
| 148 | 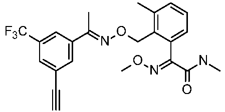 | 9 | 2 | 13 | 7 |
| 149 | 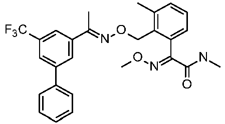 | 83 | 45 | 87 | 31 |
| 150 | 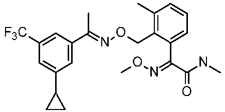 | 56 | 8 | 70 | 29 |
| 151 | 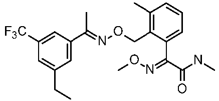 | 37 | 3 | 53 | 6 |
| 152 | 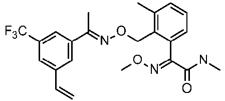 | 24 | 1 | 38 | 7 |
| 153 | 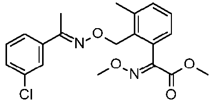 | 12 | 3 | 22 | 1 |
| 154 | 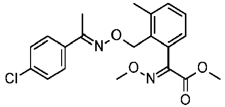 | 30 | 13 | 63 | 9 |
| 155 | 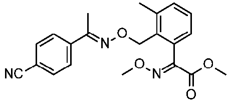 | 97 | 30 | 93 | 20 |
| 156 | 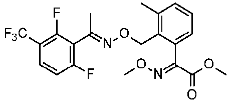 | 27 | 0 | 47 | 3 |
| 157 | 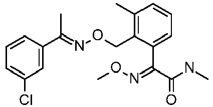 | 1 | 0 | 1 | 0 |
| 158 | 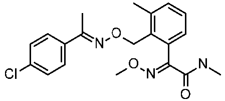 | 0 | 0 | 2 | 0 |
| 159 | 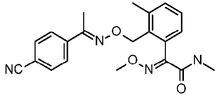 | 28 | 2 | 28 | 1 |
| 160 | 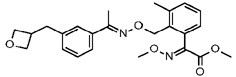 | 100 | 97 | 87 | 90 |
| 161 | 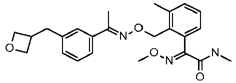 | 100 | 100 | 100 | 90 |
| 162 | 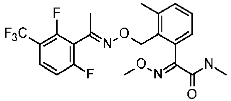 | 0 | 0 | 1 | 0 |
| 163 | 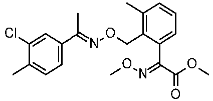 | 20 | 1 | 47 | 9 |
| 164 | 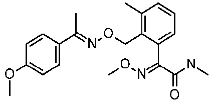 | 5 | 0 | 27 | 0 |
| 165 | 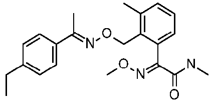 | 0 | 0 | 17 | 0 |
| 166 | 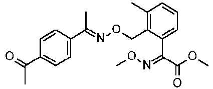 | 100 | 83 | 90 | 43 |
| 167 | 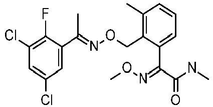 | 2 | 0 | 9 | 0 |
| 168 | 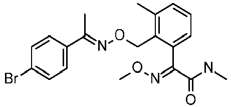 | 2 | 0 | 5 | 0 |
| 169 | 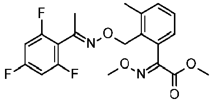 | 77 | 0 | 77 | 0 |
| 170 | 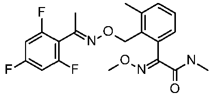 | 9 | 0 | 4 | 0 |
| 171 | 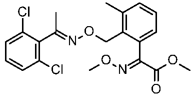 | 100 | 100 | 80 | 50 |
| 172 | 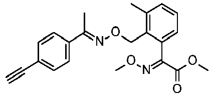 | 35 | 1 | 83 | 12 |
| 173 | 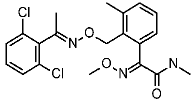 | 100 | 53 | 97 | 17 |
| 174 | 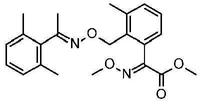 | 100 | 100 | 80 | 70 |
| 175 | 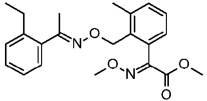 | 100 | 97 | 100 | 93 |
| 176 | 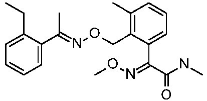 | 100 | 100 | 100 | 90 |
| 177 | 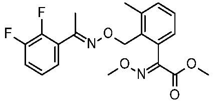 | 100 | 21 | 87 | 47 |
| 178 | 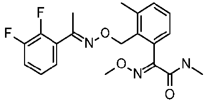 | 6 | 0 | 2 | 0 |
| 179 | 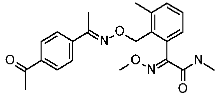 | 100 | 47 | 90 | 28 |
| 180 | 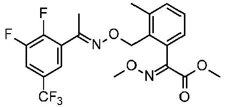 | 40 | 7 | 5 | 0 |
| 181 | 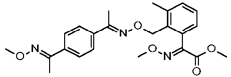 | 22 | 11 | 33 | 5 |
| 182 | 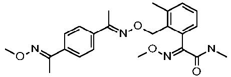 | 6 | 0 | 13 | 0 |
| 183 | 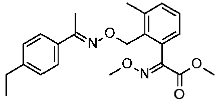 | 16 | 0 | 38 | 2 |
| 184 | 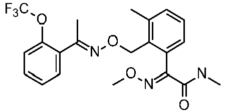 | 42 | 4 | 16 | 1 |
| 185 | 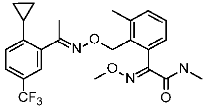 | 100 | 67 | 90 | 77 |
| 186 | 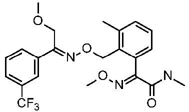 | 1 | 0 | 2 | 0 |
| 187 | 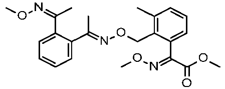 | 100 | 100 | 90 | 90 |
| 188 | 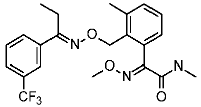 | 1 | 0 | 11 | 0 |
| 189 | 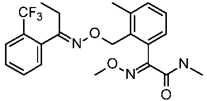 | 82 | 28 | 97 | 37 |
| 190 | 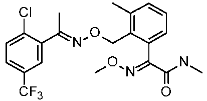 | 20 | 0 | 45 | 3 |
| 191 | 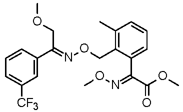 | 77 | 2 | 83 | 22 |
| 193 | 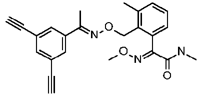 | 15 | 0 | 14 | 0 |
| 196 | 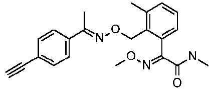 | 1 | 0 | 11 | 5 |
| 197 | 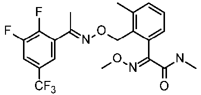 | 0 | 0 | 2 | 0 |
| 198 | 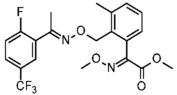 | 4 | 1 | 0 | 0 |
| 199 | 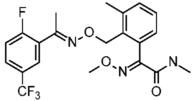 | 0 | 0 | 0 | 0 |
| 200 | 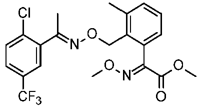 | 93 | 73 | 100 | 77 |
| 202 | 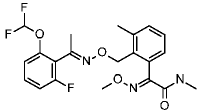 | 90 | 22 | 100 | 47 |
| 203 | 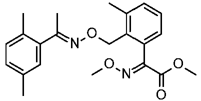 | 87 | 32 | 93 | 32 |
| 204 | 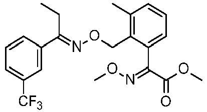 | 50 | 4 | 80 | 5 |
| 206 | 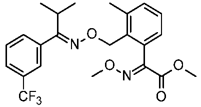 | 100 | 67 | 100 | 90 |
| 207 | 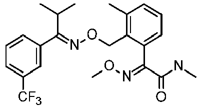 | 40 | 11 | 83 | 5 |
| 211 | 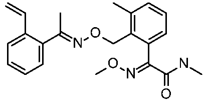 | 100 | 43 | 100 | 77 |
| 213 | 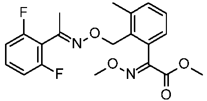 | 50 | 3 | 40 | 11 |
| 214 | 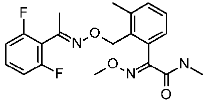 | 14 | 0 | 28 | 4 |
| 215 | 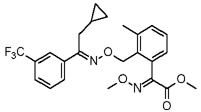 | 87 | 37 | 87 | 33 |
| 216 | 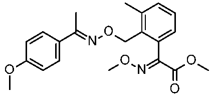 | 77 | 13 | 80 | 29 |
| 217 | 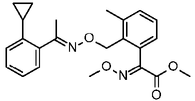 | 97 | 53 | 93 | 100 |
| 218 | 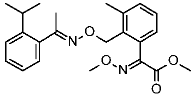 | 100 | 87 | 100 | 100 |
| 219 | 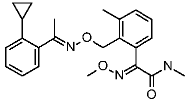 | 60 | 8 | 87 | 43 |
| 220 | 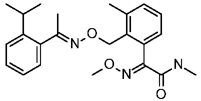 | 90 | 30 | 100 | 77 |
| 221 | 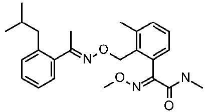 | 100 | 63 | 100 | 100 |
| 222 | 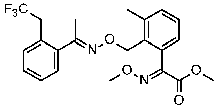 | 100 | 57 | 100 | 93 |
| 223 | 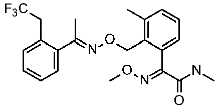 | 4 | 0 | 28 | 0 |
| 224 | 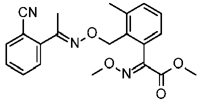 | 97 | 100 | 100 | 100 |
| 225 | 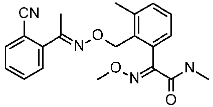 | 83 | 27 | 100 | 97 |
| 226 | 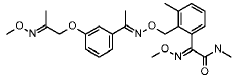 | 28 | 3 | 57 | 7 |
| 227 | 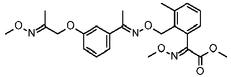 | 100 | 47 | 100 | 57 |
| 228 | 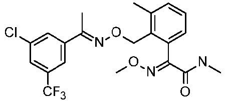 | 5 | 0 | 22 | 2 |
| 229 | 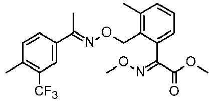 | 27 | 2 | 77 | 6 |
| 230 | 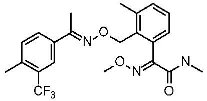 | 22 | 1 | 73 | 4 |
| 231 | 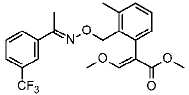 | 0 | 0 | 2 | 0 |
| 232 | 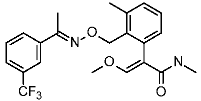 | 100 | 73 | 100 | 83 |
| 233 | 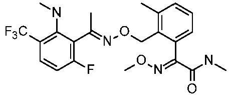 | 100 | 57 | 87 | 27 |
| 234 | 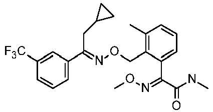 | 53 | 18 | 53 | 15 |
| 235 | 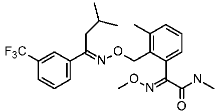 | 100 | 73 | 87 | 77 |
| 236 | 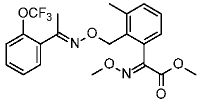 | 97 | 77 | 97 | 100 |
| 238 | 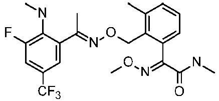 | 100 | 53 | 100 | 77 |
| 240 | 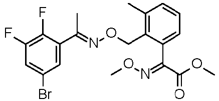 | 70 | 8 | 73 | 3 |
| 241 | 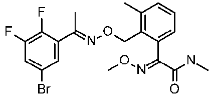 | 3 | 0 | 33 | 12 |
| 242 | 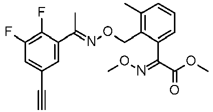 | 87 | 10 | 87 | 20 |
| 243 | 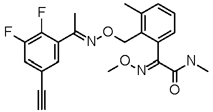 | 4 | 0 | 37 | 1 |
| 244 | 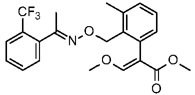 | 14 | 0 | 15 | 2 |
| 245 | 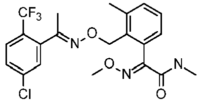 | 28 | 8 | 13 | 2 |
| 246 | 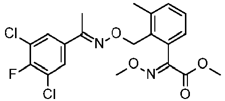 | 40 | 15 | 77 | 7 |
| 247 | 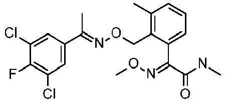 | 16 | 2 | 33 | 8 |
| 248 | 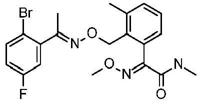 | 33 | 2 | 30 | 2 |
| 249 | 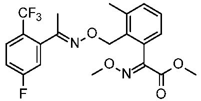 | 87 | 37 | 90 | 43 |
| 250 | 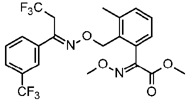 | 90 | 90 | 90 | 80 |
| 251 | 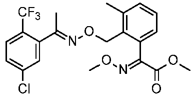 | 80 | 80 | 80 | 80 |
| 252 | 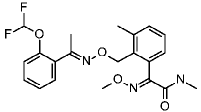 | 50 | 3 | 87 | 32 |
| 253 | 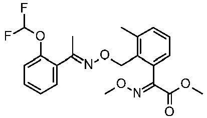 | 100 | 87 | 100 | 57 |
| 254 | 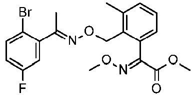 | 100 | 77 | 100 | 100 |
| 255 | 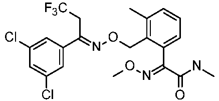 | 97 | 83 | 100 | 87 |
| 256 | 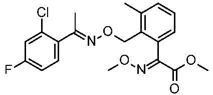 | 50 | 18 | 70 | 20 |
| 257 | 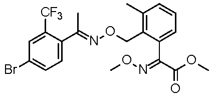 | 93 | 35 | 100 | 57 |
| 258 | 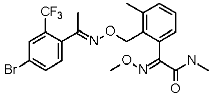 | 32 | 7 | 73 | 8 |
| 259 | 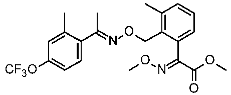 | 80 | 17 | 93 | 40 |
| 260 | 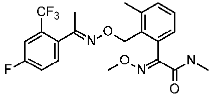 | 22 | 0 | 22 | 0 |
| 261 | 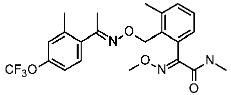 | 70 | 9 | 87 | 32 |
| 262 | 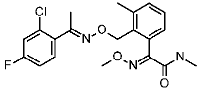 | 63 | 1 | 47 | 4 |
| 263 | 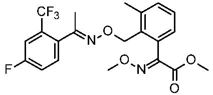 | 80 | 15 | 73 | 22 |
| 266 | 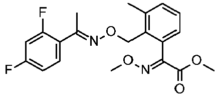 | 67 | 18 | 90 | 43 |
| 267 | 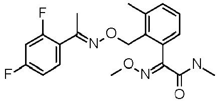 | 2 | 0 | 10 | 4 |
| 268 | 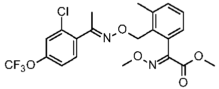 | 63 | 6 | 60 | 17 |
| 269 | 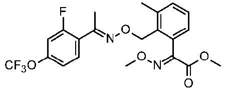 | 5 | 0 | 18 | 0 |
| 270 | 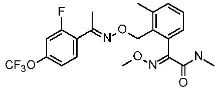 | 3 | 0 | 0 | 1 |
| 271 | 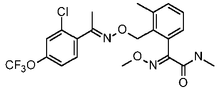 | 5 | 1 | | |
| 272 | 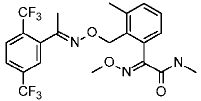 | 60 | 8 | | |
| 273 | 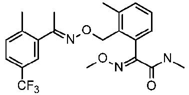 | 60 | 3 | | |
| 277 | 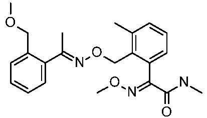 | 100 | 60 | 100 | 100 |
| 278 | 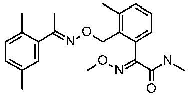 | 90 | 60 | 90 | 93 |
| 282 | 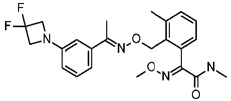 | | 15 | | 18 |
| 283 | 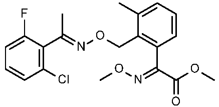 | 83 | 30 | 87 | 22 |
| 284 | 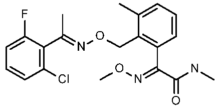 | 63 | 44 | 62 | 34 |
| 285 | 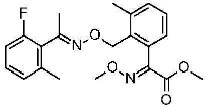 | 87 | 50 | 90 | 35 |
| 286 | 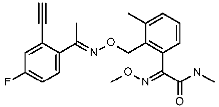 | 67 | 15 | 97 | 27 |
| 288 | 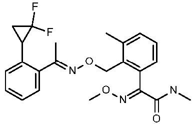 | 87 | 30 | 97 | 20 |
| 290 | 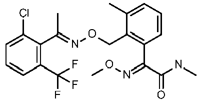 | 92 | 14 | 83 | 21 |
| 294 | 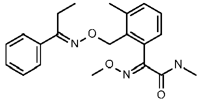 | 1 | 1 | 2 | 0 |
| 295 | 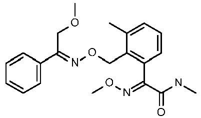 | 2 | 0 | 11 | 0 |
| 296 | 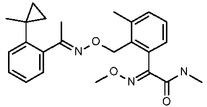 | 100 | 40 | 100 | 83 |
| 297 | 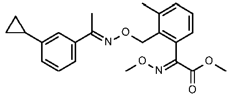 | 90 | 37 | 87 | 37 |
| 298 | 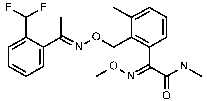 | 63 | 7 | 97 | 37 |
| 299 | 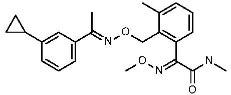 | 53 | 13 | 53 | 22 |
| 302 | 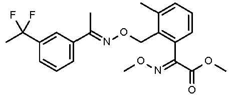 | 47 | 4 | 47 | 3 |
| 303 | 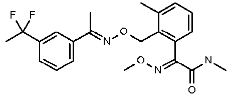 | 2 | 0 | 12 | 0 |
| 304 | 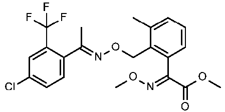 | 53 | 28 | 100 | 37 |
| 312 | 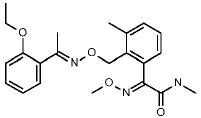 | 100 | 53 | | |
| 314 | 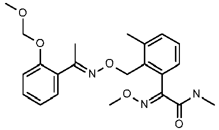 | 100 | 40 | | |
| 315 | 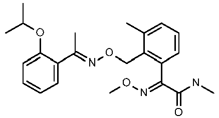 | 100 | 60 | | |
| 321 | 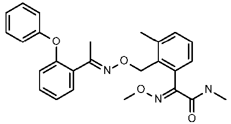 | 100 | 40 | | |
| 324 | 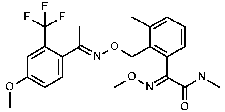 | 2 | 1 | 12 | 0 |
| 325 | 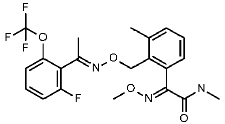 | 24 | 4 | 22 | 1 |
| 326 | 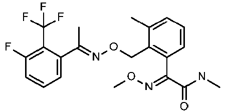 | 25 | 0 | 30 | 1 |
| 327 | 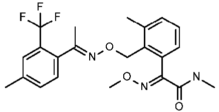 | 23 | 3 | 48 | 4 |
| 328 | 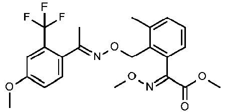 | 33 | 8 | 23 | 5 |
| 329 | 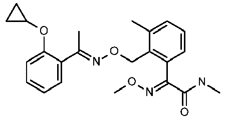 | 100 | 53 | 100 | 73 |
| 330 | 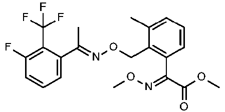 | 83 | 24 | 93 | 17 |
| 335 | 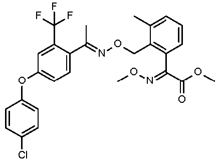 | 77 | 53 | 80 | 25 |
| 336 | 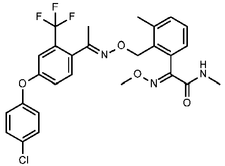 | 63 | 20 | 50 | 17 |
| 337 | 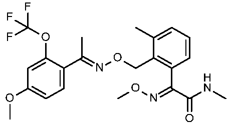 | 9 | 0 | 13 | 1 |
| 340 | 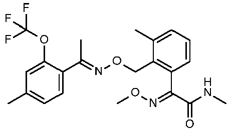 | 23 | 4 | 42 | 7 |
| 344 | 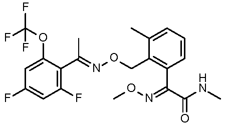 | 6 | 0 | 16 | 0 |
| 345 | 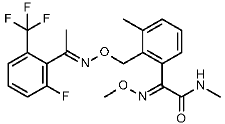 | 22 | 1 | 32 | 1 |
| 346 | 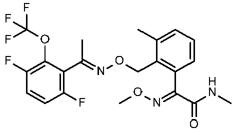 | 4 | 0 | 5 | 0 |
| 349 | 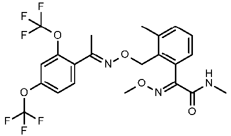 | 97 | 50 | 97 | 35 |
| 354 | 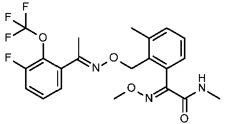 | 17 | 2 | 21 | 4 |
| 355 | 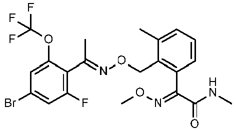 | 34 | 7 | 48 | 4 |
| 357 | 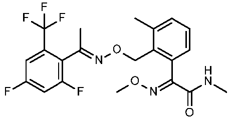 | 13 | 1 | 18 | 0 |
| 358 | 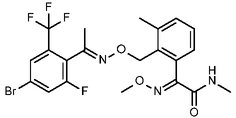 | 77 | 17 | 83 | 18 |
| 359 | 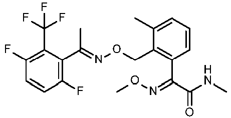 | 100 | 37 | 100 | 43 |
| 360 | 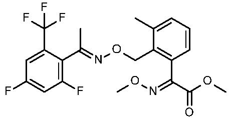 | 53 | 9 | 80 | 5 |
| 361 | 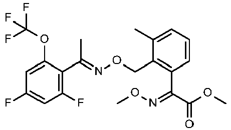 | 80 | 18 | 88 | 31 |
| 363 | 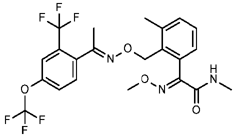 | 29 | 1 | 25 | 2 |
| 365 | 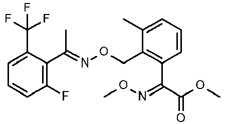 | 77 | 15 | 97 | 43 |
| 366 | 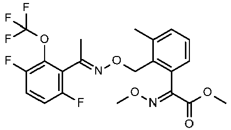 | 53 | 13 | 83 | 12 |
| 367 | 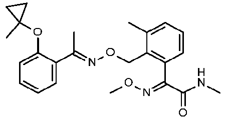 | 63 | 9 | 93 | 30 |
| 368 | 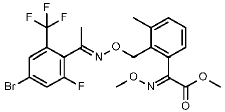 | 83 | 47 | 90 | 73 |
| 372 | 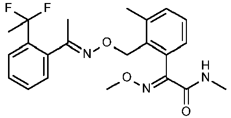 | 85 | 26 | 85 | 16 |
| 373 | 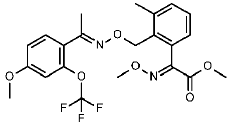 | 77 | 27 | 100 | 38 |
| 375 | 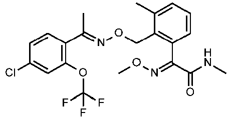 | 47 | 8 | 40 | 6 |
| 378 | 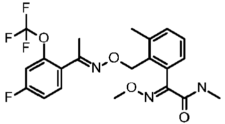 | 18 | 1 | 17 | 1 |
| 380 | 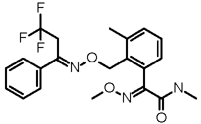 | 53 | 5 | 60 | 12 |
| 387 | 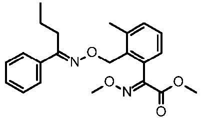 | 60 | 30 | 80 | 47 |
| 388 | 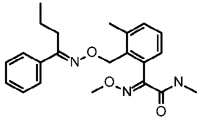 | 1 | 0 | 3 | 0 |
| 389 | 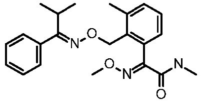 | 28 | 4 | 43 | 3 |
| 390 | 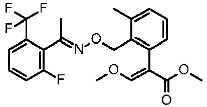 | 22 | 0 | 18 | 2 |
| 393 | 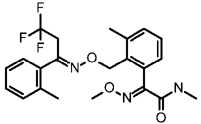 | 93 | 55 | 93 | 42 |
| 394 | 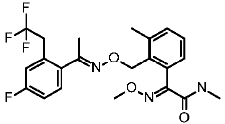 | 9 | 3 | 12 | 2 |
| 395 | 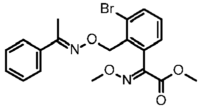 | 43 | 4 | 67 | 18 |
| 396 | 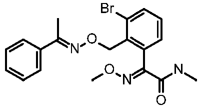 | 3 | 0 | 4 | 0 |
| 399 | 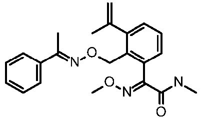 | 67 | 8 | 90 | 15 |
| 400 | 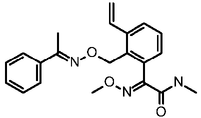 | 2 | 0 | 8 | 0 |
| 401 | 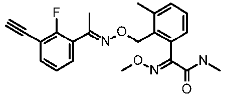 | 17 | 5 | 32 | 4 |
| 405 | 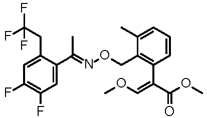 | 97 | 27 | 70 | 27 |
| 406 | 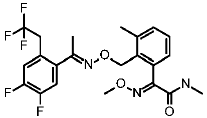 | 97 | 30 | 67 | 23 |
| 407 | 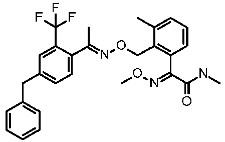 | 12 | 6 | 17 | 4 |
| 408 | 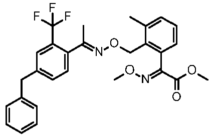 | 30 | 12 | 33 | 13 |
| 409 | 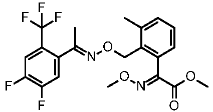 | 77 | 40 | 83 | 73 |
| 410 | 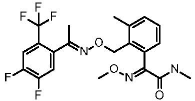 | 9 | 0 | 35 | 1 |
| 412 | 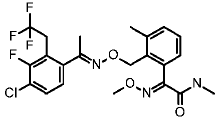 | 47 | 6 | 40 | 6 |
| 413 | 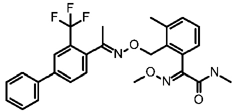 | 40 | 15 | 33 | 15 |
| 414 | 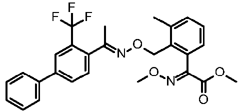 | 53 | 9 | 53 | 15 |
| 415 | 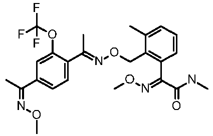 | 47 | 5 | 67 | 11 |
| 416 | 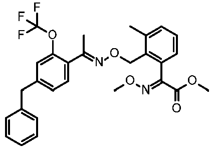 | 57 | 27 | 67 | 25 |
| 417 | 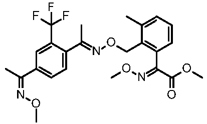 | 35 | 18 | 63 | 22 |
| 418 | 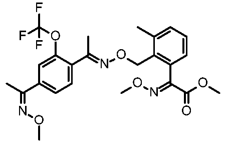 | 70 | 33 | 73 | 57 |
| 419 | 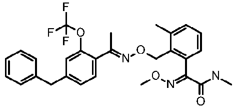 | 40 | 18 | 60 | 12 |
| 420 | 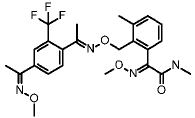 | 8 | 0 | 12 | 1 |
| 421 | 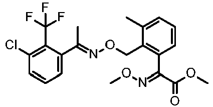 | 100 | 33 | 87 | 57 |
| 422 | 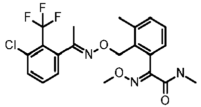 | 30 | 0 | 32 | 2 |
| 423 | 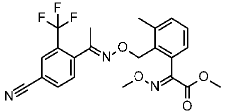 | 100 | 57 | 93 | 53 |
| 424 | 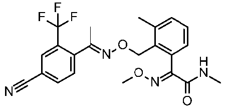 | 100 | 27 | 97 | 50 |
| 425 | 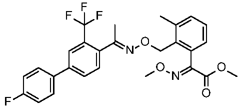 | 27 | 28 | 53 | 40 |
| 426 | 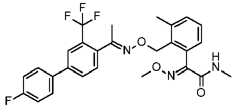 | 7 | 1 | 27 | 10 |
| 427 | 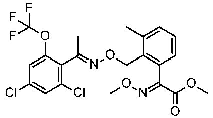 | 100 | 90 | 60 | 47 |
| 428 | 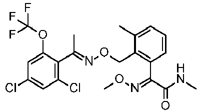 | 70 | 11 | 83 | 20 |
| 429 | 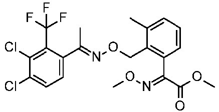 | 83 | 50 | 67 | 43 |
| 430 | 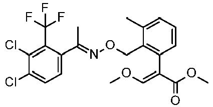 | 22 | 6 | 37 | 17 |
| 431 | 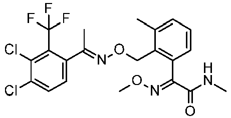 | 32 | 7 | 40 | 12 |
| 432 | 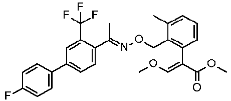 | 12 | 0 | 13 | 3 |
| 433 | 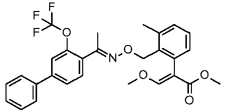 | 83 | 67 | 80 | 57 |
| 435 | 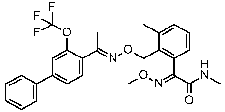 | 93 | 57 | 87 | 60 |
| 436 | 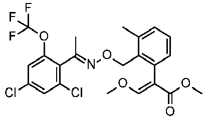 | 70 | 15 | 73 | 27 |
| 437 | 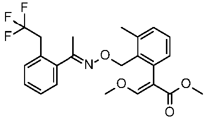 | 2 | 0 | 8 | 0 |
| 440 | 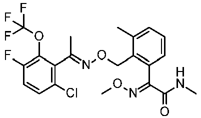 | 93 | 23 | 73 | 23 |
| 441 | 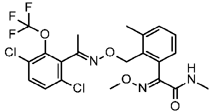 | 100 | 43 | 97 | 50 |
| 442 | 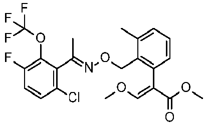 | 100 | 93 | 80 | 77 |
| 444 | 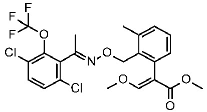 | 100 | 47 | 83 | 53 |
| 445 | 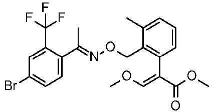 | 15 | 1 | 30 | 2 |
| 446 | 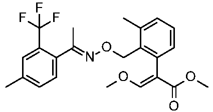 | 2 | 0 | 6 | 0 |
| 447 | 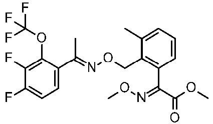 | 7 | 0 | 33 | 1 |
| 449 | 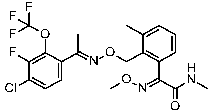 | 33 | 10 | 57 | 9 |
| 450 | 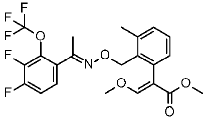 | 3 | 1 | 4 | 1 |
| 451 | 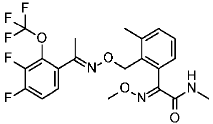 | 1 | 0 | 2 | 0 |
| 452 | 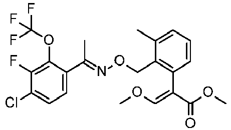 | 60 | 6 | 70 | 14 |
| 458 | 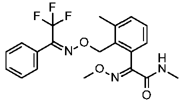 | 93 | 57 | 83 | 50 |
| 461 | 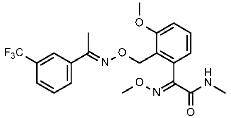 | 26 | 2 | 52 | 6 |
| 462 | 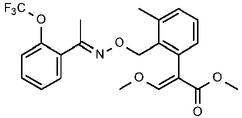 | 37 | 6 | 55 | 10 |
| 463 | 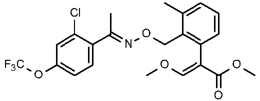 | 6 | 0 | 3 | 0 |
| 464 | 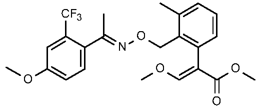 | 1 | 0 | 8 | 0 |

### Comparative trials

**Table C1:**

| | | **PHAKPA (F129L) Disease level (%)** | | | |
|---|---|---|---|---|---|
| **Compound** | **Structure** | **P2 at 4 ppm** | **P2 at 16 ppm** | **P6 at 4 ppm** | **P6 at 16 ppm** |
| Trifloxystrobin as comparative example | 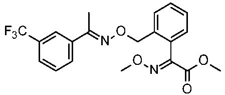 | 71 | 17 | 79 | 33 |
| Ex. 9 | 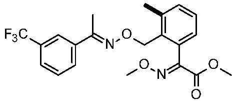 | **35** | **23** | **41** | **4** |

**Table C2:**

| | | **PHAKPA (F129L) Disease level (%)** | |
|---|---|---|---|
| **Compound** | **Structure** | **P2 at 4 ppm** | **P6 at 4 ppm** |
| Comparative example | 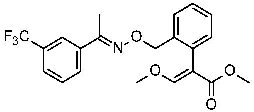 | 6 | 30 |
| Ex. 231 | 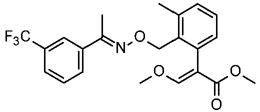 | **0** | **2** |
| Comparative example | 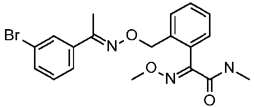 | 27 | 70 |
| Ex. 58 | 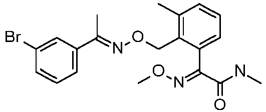 | **0** | **4** |
| Comparative example | 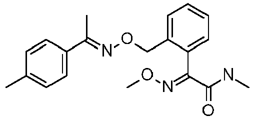 | 100 | 100 |
| Ex. 6 | 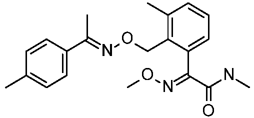 | **0** | **23** |
| Comparative example | 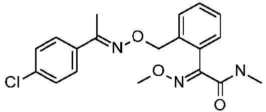 | 40 | 80 |
| Ex. 158 | 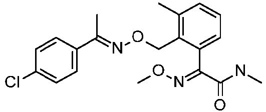 | **1** | **4** |
| Comparative example | 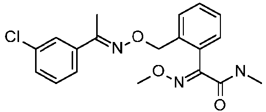 | 43 | 80 |
| Ex. 157 | 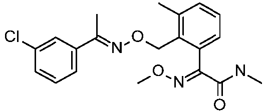 | **0** | **2** |
| Comparative example | 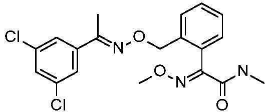 | 100 | 97 |
| Ex. 4 | 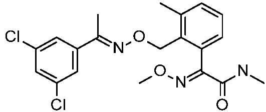 | **2** | **17** |
| Comparative example | 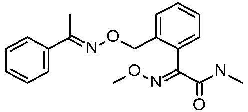 | 87 | 100 |
| Ex. 31 | 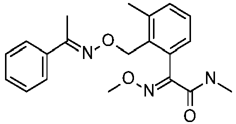 | **0** | **12** |
| Comparative example | 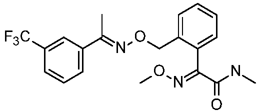 | 12 | 38 |
| Ex. 8 | 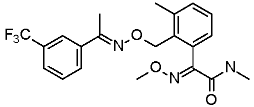 | **1** | **13** |
| Comparative example | 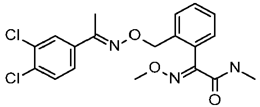 | 43 | 77 |
| Ex. 41 | 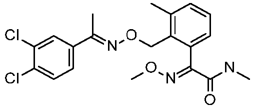 | **4** | **35** |
| Comparative example | 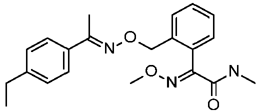 | 35 | 83 |
| Ex. 165 | 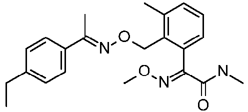 | **0** | **27** |
| Comparative example | 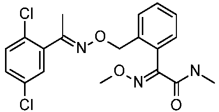 | 87 | 97 |
| Ex. 130 | 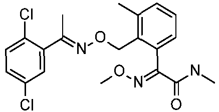 | **33** | **67** |
| Comparative example | 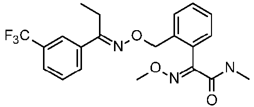 | 60 | 70 |
| Ex. 188 | 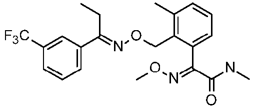 | **2** | **30** |
| Comparative example | 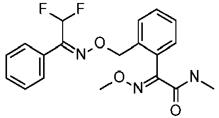 | 43 | 90 |
| Ex. 73 | 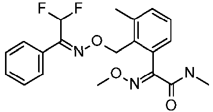 | **1** | **37** |
| Untreated | | 100 | 99 |

**Table C3:**

| | | **PHAKPA (F129L) Disease level (%)** | |
|---|---|---|---|
| **Compound** | **Structure** | **P2 at 16 ppm** | **P6 at 16 ppm** |
| Comparative example | 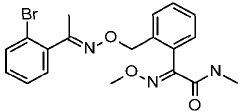 | 23 | 28 |
| Ex. 120 | 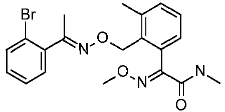 | **6** | **15** |
| Comparative example | 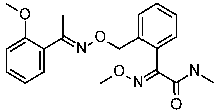 | 87 | 80 |
| Ex. 126 | 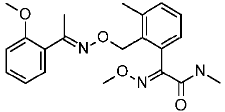 | **32** | **60** |
| Comparative example | 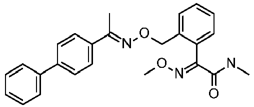 | 37 | 28 |
| Ex. 113 | 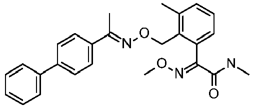 | **17** | **6** |
| Comparative example | 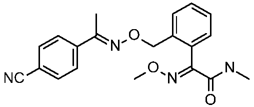 | 37 | 63 |
| Ex. 159 | 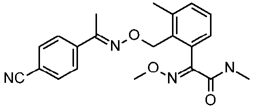 | **0** | **0** |
| Comparative example | 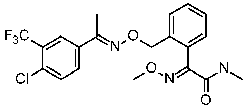 | 11 | 4 |
| Ex. 60 | 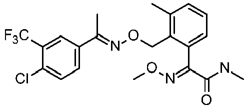 | **0** | **0** |
| Comparative example | 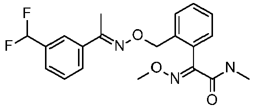 | 16 | 35 |
| Ex. 12 | 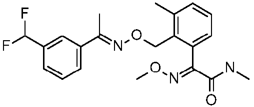 | **3** | **9** |
| Comparative example | 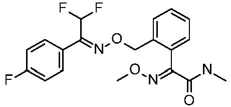 | 15 | 15 |
| Ex. 27 | 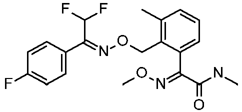 | **0** | **0** |
| Comparative example | 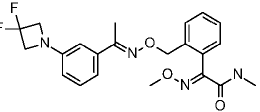 | 70 | 53 |
| Ex. 282 | 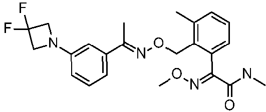 | **15** | **18** |
| Comparative example | 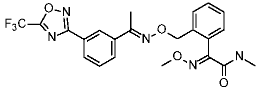 | 23 | 32 |
| Ex. 205 | 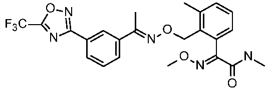 | **1** | **1** |
| Untreated | | 100 | 87 |

**Table C4:**

| | | **PHAKPA (F129L) Disease level (%)** | |
|---|---|---|---|
| **Compound** | **Structure** | **P2 at 16 ppm** | **P6 at 16 ppm** |
| Comparative example | 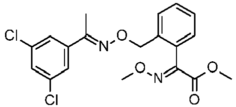 | 27 | 17 |
| Ex. 3 | 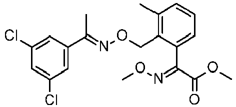 | 2 | 1 |
| Comparative example | 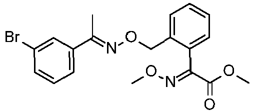 | 80 | 87 |
| Ex. 56 | 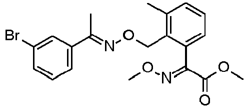 | 32 | 15 |
| Comparative example | 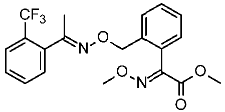 | 87 | 90 |
| Ex. 36 | 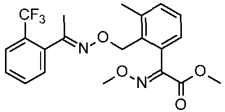 | 47 | 57 |
| Comparative example | 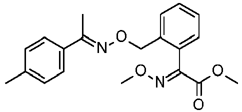 | 25 | 10 |
| Ex. 5 | 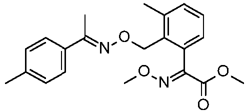 | 1 | 4 |
| Comparative example | 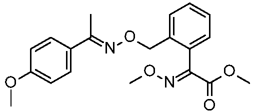 | 67 | 33 |
| Ex. 216 | 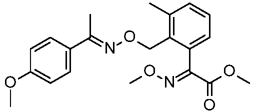 | 20 | 15 |
| Comparative example | 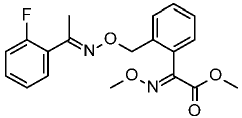 | 83 | 77 |
| Ex. 1 | 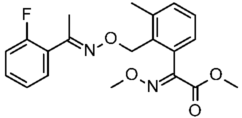 | **28** | **47** |
| Comparative example | 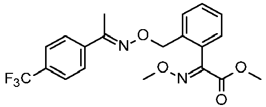 | 43 | 13 |
| Ex. 37 | 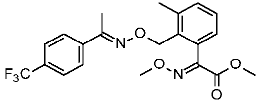 | **0** | **0** |
| Comparative example | 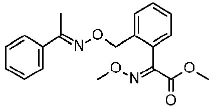 | 87 | 43 |
| Ex. 30 | 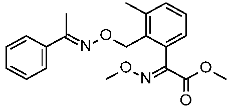 | 2 | 1 |
| Comparative example | 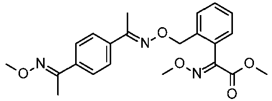 | 57 | 60 |
| Ex. 181 | 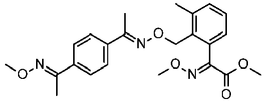 | **12** | **5** |
| Comparative example | 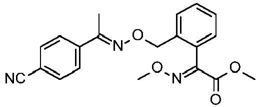 | 87 | 53 |
| Ex. 155 | 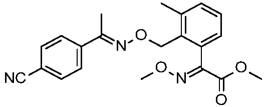 | **23** | **18** |
| Comparative example | 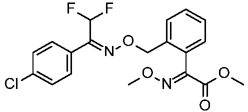 | 100 | 90 |
| Ex. 20 | 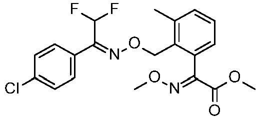 | **30** | **18** |
| Comparative example | 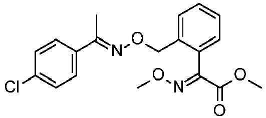 | 63 | 43 |
| Ex. 154 | 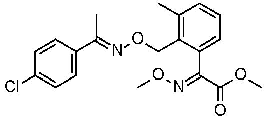 | 25 | 17 |
| Comparative example | 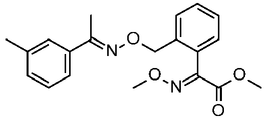 | 93 | 83 |
| Ex. 76 | 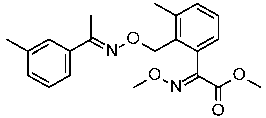 | **1** | **0** |
| Comparative example | 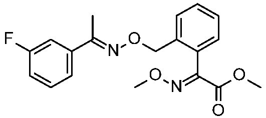 | 90 | 80 |
| Ex. 86 | 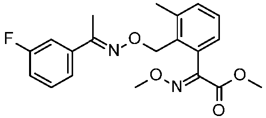 | **6** | **7** |
| Comparative example | 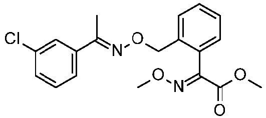 | 73 | 70 |
| Ex. 153 | 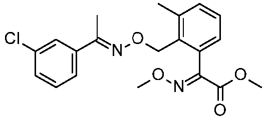 | **5** | **1** |
| Comparative example | 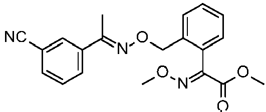 | 80 | 43 |
| Ex. 104 | 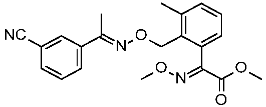 | **37** | **28** |
| Comparative example | 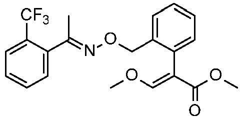 | 11 | 9 |
| Ex. 244 | 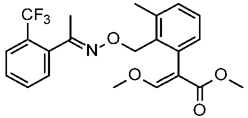 | **0** | **2** |
| Comparative example | 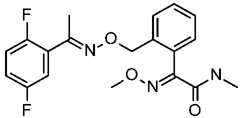 | 1 | 22 |
| Ex. 131 | 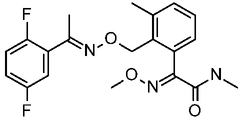 | **0** | **0** |
| Untreated | | >90 | >85 |

The results in Tables C1 to C4 show that the specific substituent at position R³ improves the fungicidal activity against phytopathogenic fungi containing the amino acid substitution F129L in the mitochondrial cytochrome b protein conferring resistance to Qo inhibitors compared to compounds where the position R³ is unsubstituted.

**Table C5:**

| | | **Fungal growth (%)** | | | |
|---|---|---|---|---|---|
| **Concentration applied (ppm)** | | **0.016** | **0.016** | **0.016** | **0.016** |
| **Compound** | **Structure** | **PYRIOR** | **ALTESO wt** | **ALTESO F129L** | **MONGNI** |
| Comparative example from WO 2017/157923 | 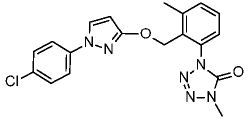 | 87 | 98 | 100 | 97 |
| Ex. 158 | 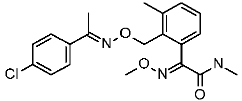 | **38** | **66** | **79** | **71** |

**Table C6a:**

| | | **PHAKPA P1 DL Disease level (%)** | | | | | | | |
|---|---|---|---|---|---|---|---|---|---|
| | | **Qo I-sensitive wt isolate (0 % F129L)** | | | | | | | |
| | | **Test concentration (ppm)** | | | | | | | |
| **Compound** | **Structure** | **0** | **0.3** | **1** | **3** | **10** | **30** | **100** | **300** |
| Comparative example from WO 17/157923 | 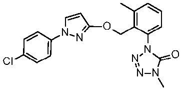 | 93 | 78 | 80 | 77 | 48 | 30 | 18 | 5 |
| Ex. 158 | 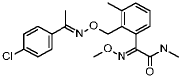 | | **38** | **7** | **2** | **1** | **4** | **5** | **4** |

**Table C6b:**

| | | **PHAKPA P1 DL Disease level (%)** | | | | | | | |
|---|---|---|---|---|---|---|---|---|---|
| | | **Qo I-resistant F129L isolate (100 % F129L)** | | | | | | | |
| | | **Test concentration (ppm)** | | | | | | | |
| **Compound** | **Structure** | **0** | **0.3** | **1** | **3** | **10** | **30** | **100** | **300** |
| Comparative example from WO 17/157923 | 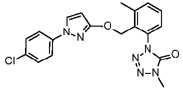 | 93 | 88 | 90 | 95 | 92 | 90 | 65 | 52 |
| Ex. 158 | 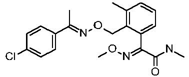 | | **87** | **57** | **8** | **2** | **4** | **4** | **5** |

The results in Tables C5 to C6b show that the compounds to the present invention significantly improve the fungicidal activity against phytopathogenic fungi containing the amino acid substitution F129L in the mitochondrial cytochrome b protein conferring resistance to Qo inhibitors compared to the use of a compound disclosed in WO 2017/157923.

**Table C7a:**

| | | **Fungal growth (%)** | | | |
|---|---|---|---|---|---|
| **Concentration applied (ppm)** | | **0.016** | **0.016** | **0.025** | **4** |
| **Compound** | **Structure** | **PYRIOR** | **ALTESO wt** | **PYRNTE wt** | **CERCSO** |
| Comparative example from WO 98/23156 | 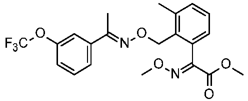 | 100 | 94 | 84 | 33 |
| Ex. 9 | 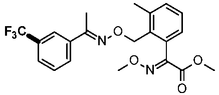 | **38** | **73** | **44** | **11** |

**Table C7b:**

| | | **PHAKPA (F129L)** |
|---|---|---|
| | | **Disease level (%)** |
| **Compound** | **Structure** | **P2 at 4 ppm** |
| Comparative example from WO 98/23156 | 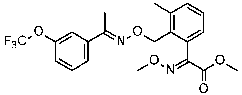 | 17 |
| Ex. 9 | 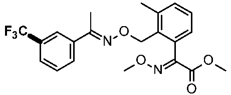 | **6** |
| Untreated | | 92 |

**Table C8a:**

| | | **Fungal growth (%)** | | | |
|---|---|---|---|---|---|
| **Concentration applied (ppm)** | | **0.016** | **0.063** | **0.016** | **4** |
| **Compound** | **Structure** | **PYRIOR** | **COLLLA** | **ALTESO wt** | **ALTESO F129L** |
| Comparative example from WO 98/23156 | 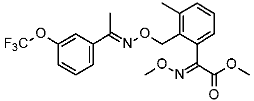 | 100 | 77 | 94 | 87 |
| Ex. 84 | 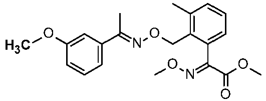 | **48** | **33** | **43** | **39** |

**Table C8b:**

| | | **Fungal growth (%)** | | | |
|---|---|---|---|---|---|
| **Concentration applied (ppm)** | | **0.25** | **0.25** | **0.063** | **0.016** |
| **Compound** | **Structure** | **PYRNTE wt** | **PYRNTE F129L** | **LEPTNO** | **MONGNI** |
| Comparative example from WO 98/23156 | 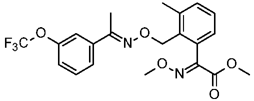 | 87 | 84 | 79 | 86 |
| Ex. 84 | 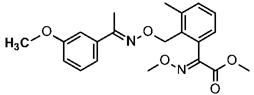 | **39** | **49** | **60** | **32** |

The results in Table C7a to C8b show that the specific substituent R^{a} of the terminal phenyl improves the fungicidal activity against phytopathogenic fungi compared to compounds from the prior art.

**Table C9:**

| | | **Fungal growth (%)** | | |
|---|---|---|---|---|
| **Concentration applied (ppm)** | | **0.016** | **0.063** | **4** |
| **Compound** | **Structure** | **PYRIOR** | **LEPTNO** | **CERCSO** |
| Comparative example from WO 98/23156 | 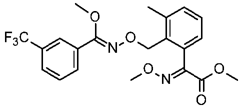 | 58 | 100 | 56 |
| Ex. 9 | 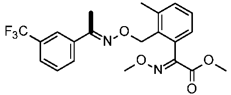 | **38** | **67** | **11** |

**Table C10:**

| | | **Fungal growth (%)** | | | | |
|---|---|---|---|---|---|---|
| **Concentration applied (ppm)** | | **0.016** | **0.063** | **0.016** | **4** | **0.016** |
| **Compound** | **Structure** | **PYRIOR** | **LEPTNO** | **ALTESO F129L** | **CERCSO** | **MONGNI** |
| Comparative example from WO 98/23156 | 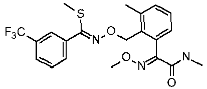 | 49 | 93 | 85 | 66 | 84 |
| Ex. 8 | 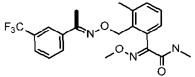 | **13** | **70** | **55** | **27** | 54 |

**Table C11a:**

| | | **Fungal growth (%)** | | | | |
|---|---|---|---|---|---|---|
| **Concentration applied (ppm)** | | **0.016** | **0.25** | **0.063** | **0.016** | **0.016** |
| **Compound** | **Structure** | **PYRIOR** | **SEPTTR** | **LEPTNO** | **ALTESO wt** | **ALTESO F129L** |
| Comparative example from WO 98/23156 | 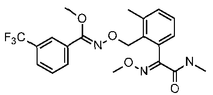 | 39 | 77 | 95 | 100 | 87 |
| Ex. 8 | 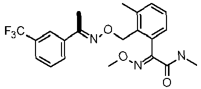 | **13** | **57** | **70** | **56** | **52** |

**Table C11b:**

| | | **Fungal growth (%)** | |
|---|---|---|---|
| **Concentration applied (ppm)** | | **4** | **0.016** |
| **Compound** | **Structure** | **CERCSO** | **MONGNI** |
| Comparative example from WO 98/23156 | 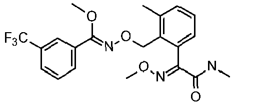 | 60 | 80 |
| Ex. 8 | 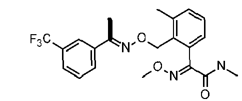 | **27** | **54** |

**Table C12:**

| | | **Fungal growth (%)** | | | | |
|---|---|---|---|---|---|---|
| **Concentration applied (ppm)** | | **0.016** | **0.25** | **0.063** | **0.016** | **0.25** |
| **Compound** | **Structure** | **PYRIOR** | **SEPTTR** | **COLLLA** | **MONGNI** | **PYRTNE F129L** |
| Comparative example from WO 98/23156 | 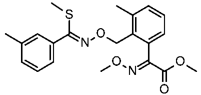 | 87 | 61 | 81 | 69 | |
| Comparative example from WO 98/23156 | 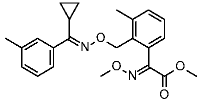 | 82 | 89 | 93 | 84 | 87 |
| Ex. 76 | 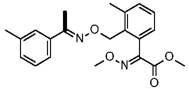 | **43** | **0** | **39** | **35** | **66** |

**Table C13:**

| | | **Fungal growth (%)** | | | | |
|---|---|---|---|---|---|---|
| **Concentration applied (ppm)** | | **0.063** | **0.016** | **0.016** | **0.25** | **4** |
| **Compound** | **Structure** | **LEPTNO** | **ALTESO wt** | **ALTESO F129L** | **PYRNTE wt** | **CERCSO** |
| Comparative example from WO 98/23156 | 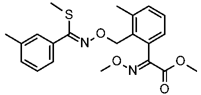 | 85 | 67 | 66 | 59 | 71 |
| Comparative example from WO 98/23156 | 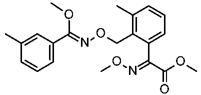 | 65 | 93 | 81 | 53 | 67 |
| Comparative example from WO 98/23156 | 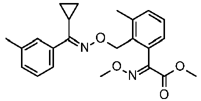 | 100 | 100 | 87 | 78 | 87 |
| Ex. 76 | 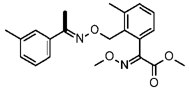 | **39** | **55** | **37** | **39** | **28** |

**Table C14:**

| | | **Fungal growth (%)** | | | | |
|---|---|---|---|---|---|---|
| **Concentration applied (ppm)** | | **0.016** | **0.25** | **0.063** | **0.016** | **0.016** |
| **Compound** | **Structure** | **PYRIOR** | **SEPTTR** | **COLLLA** | **ALTESO wt** | **ALTESO F129L** |
| Comparative example from WO 98/23156 | 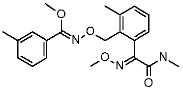 | 80 | 100 | 81 | 93 | 95 |
| Comparative example from WO 98/23156 | 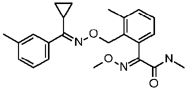 | 81 | 87 | 93 | 89 | 93 |
| Ex. 77 | 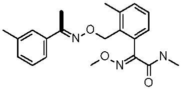 | **20** | **49** | **39** | **73** | **69** |

**Table C15a:**

| | | **Fungal growth (%)** | | | | |
|---|---|---|---|---|---|---|
| **Concentration applied (ppm)** | | **0.016** | **0.25** | **0.063** | **0.016** | **0.016** |
| **Compound** | **Structure** | **PYRIOR** | **SEPTTR** | **COLLLA** | **ALTESO wt** | **ALTESO F129L** |
| Comparative example from WO 98/23156 | 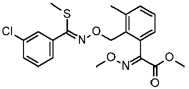 | 88 | 39 | 82 | 94 | 100 |
| Comparative example from WO 98/23156 | 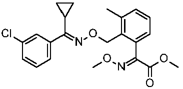 | 83 | 39 | 89 | 81 | 89 |
| Ex. 153 | 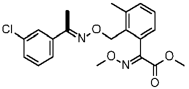 | **50** | **0** | **55** | **71** | **68** |

**Table C15b:**

| | | **Fungal growth (%)** | | | |
|---|---|---|---|---|---|
| **Concentration applied (ppm)** | | **0.063** | **0.25** | **4** | **0.016** |
| **Compound** | **Structure** | **LEPTNO** | **PYRNTE wt** | **CERCSO** | **MONGNI** |
| Comparative example from WO 98/23156 | 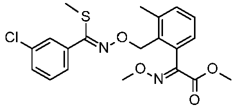 | 88 | 57 | 62 | 95 |
| Comparative example from WO 98/23156 | 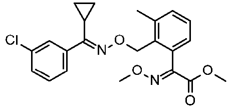 | | 69 | 61 | |
| Ex. 153 | 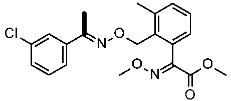 | **55** | **31** | **26** | **75** |

**Table C16a:**

| | | **Fungal growth (%)** | | | | |
|---|---|---|---|---|---|---|
| **Concentration applied (ppm)** | | **0.016** | **0.25** | **0.063** | **0.25** | **0.016** |
| **Compound** | **Structure** | **PYRIOR** | **SEPTTR** | **COLLLA** | **ALTESO wt** | **ALTESO F129L** |
| Comparative example from WO 98/23156 | 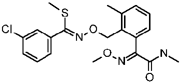 | 100 | 59 | 82 | 43 | 90 |
| Ex. 157 | 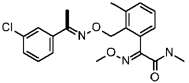 | **15** | **20** | **63** | **27** | **57** |

**Table C16b:**

| | | **Fungal growth (%)** | | | |
|---|---|---|---|---|---|
| **Concentration applied (ppm)** | | **0.25** | **0.25** | **4** | **0.016** |
| **Compound** | **Structure** | **PYRNTE wt** | **PYRNTE F129L** | **CERCSO** | **MONGNI** |
| Comparative example from WO 98/23156 | 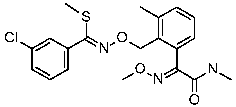 | 76 | 80 | 78 | 100 |
| Ex. 157 | 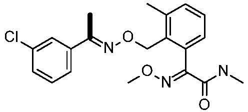 | **54** | **58** | **36** | **56** |

**Table C17:**

| | | **PHAKPA (F129L)** | |
|---|---|---|---|
| | | **Disease level (%)** | |
| **Compound** | **Structure** | **P2 at 4 ppm** | **P6 at 16 ppm** |
| Comparative example from WO 98/23156 | 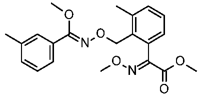 | 83 | 57 |
| Comparative example from WO 98/23156 | 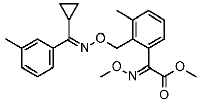 | 80 | 37 |
| Comparative example from WO 98/23156 | 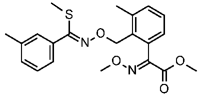 | 60 | 30 |
| Ex. 76 | 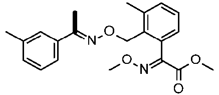 | **35** | **4** |
| Comparative example from WO 98/23156 | 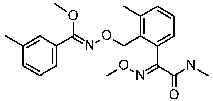 | | 45 |
| Comparative example from WO 98/23156 | 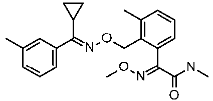 | 67 | 67 |
| Ex. 77 | 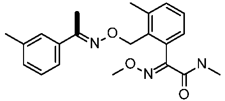 | **37** | **20** |
| Comparative example from WO 98/23156 | 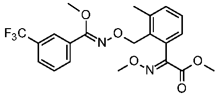 | | 23 |
| Ex. 9 | 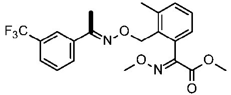 | | **1** |
| Comparative example from WO 98/23156 | 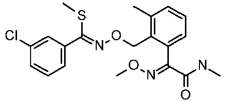 | 20 | 9 |
| Ex. 157 | 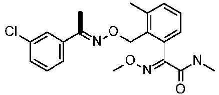 | **1** | **1** |
| Comparative example from WO 98/23156 | 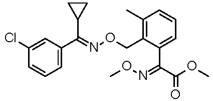 | 83 | 87 |
| Comparative example from WO 98/23156 | 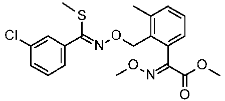 | 47 | 18 |
| Ex. 153 | 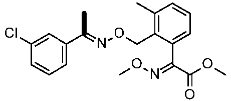 | **19** | **5** |
| Untreated | | 92 | 75 |

**Table C18:**

| | | **PHAKPA (F129L)** | |
|---|---|---|---|
| | | **Disease level (%)** | |
| **Compound** | **Structure** | **P2 at 1 ppm** | **P6 at 4 ppm** |
| Comparative example from WO 98/23156 | 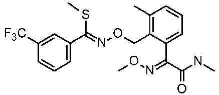 | 32 | 43 |
| Ex. 8 | 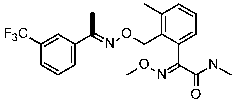 | **6** | **1** |
| Untreated | | 92 | 75 |

The results in Tables C9 to C18 show that the specific substituent R⁴ improves the fungicidal activity against phytopathogenic fungi compared to compounds from the prior art.

## Claims

1. Non-therapeutic use of compounds of formula I 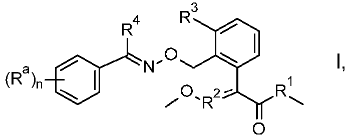 wherein
R¹ is selected from O and NH;
R² is selected from CH and N;
R³ is selected from halogen, C₁-C₄-alkyl, C₂-C₄-alkenyl, C₁-C₂-monohaloalkyl, C₁-C₂-dihaloalkyl, monohalo-ethenyl, dihalo-ethenyl, C₃-C₆-cycloalkyl and -O-C₁-C₄-alkyl;
R⁴ is selected from C₁-C₄-alkyl, C₂-C₄-alkenyl, -C(=O)-C₁-C₂-alkyl, C₁-C₄-haloalkyl, C₂-C₄-haloalkenyl, -(C₁-C₂-alkyl)-O-(C₁-C₂-alkyl) and -CH₂-cyclopropyl;
R^{a} is selected from halogen, CN, -NR⁵R⁶, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via an oxygen atom or via a C₁-C₂-alkylene linker,
and wherein the aliphatic and cyclic moieties of R^{a} are unsubstituted or carry 1, 2, 3, 4 or up to the maximum number of identical or different groups R^{b}:
R^{b} is selected from halogen, CN, NH₂, NO₂, C₁-C₄-alkyl, C₁-C₄-haloalkyl, -O-C₁-C₄-alkyl, and -O-C₁-C₄-haloalkyl;
R⁵, R⁶ are independently of each other selected from the group consisting of H, C₁-C₆-alkyl, C₁-C₆-haloalkyl and C₂-C₄-alkynyl;
n is an integer selected from 0, 1, 2, 3, 4 and 5;
and in form or stereoisomers and tautomers thereof, and the N-oxides and the agriculturally acceptable salts thereof, for combating phytopathogenic fungi containing an amino acid substitution F129L in the mitochondrial cytochrome b protein conferring resistance to Qo inhibitors.

2. The use according to claim 1, wherein in formula I R¹ is O; and R² is CH.

3. The use according to claim 1 or claim 2, wherein in formula I R³ is selected from C₁-C₂-alkyl, C₁-C₂-monohaloalkyl, C₁-C₂-dihaloalkyl, C₃-C₄-cycloalkyl and -O-C₁-C₂-alkyl.

4. The use according to any of claims 1 to 3, wherein in formula I R⁴ is selected from C₁-C₄-alkyl, -C(=O)-C₁-C₂-alkyl, C₁-C₄-haloalkyl and -(C₁-C₂-alkyl)-O-(C₁-C₂-alkyl).

5. The use according to any one of claims 1 to 4, wherein in formula I R^{a} is selected from C₁-C₃-alkyl, C₂-C₃-alkenyl, C₂-C₃-alkynyl, -O-C₁-C₃-alkyl, -C(=N-O-C₁-C₂-alkyl)-C₁-C₂-alkyl, -O-CH₂-C(=N-O-C₁-C₂-alkyl)-C₁-C₂-alkyl, C₃-C₄-cycloalkyl, -C₁-C₂-alkyl-C₃-C₄-cycloalkyl, - O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, wherein said phenyl and heteroaryl are bound directly or via an oxygen atom or via a methylene linker, and wherein the aliphatic and cyclic moieties of R^{a} are unsubstituted or carry 1, 2 or 3 of identical or different groups R^{b} which independently of one another are selected from halogen, CN, methyl and C₁-haloalkyl.

6. The use according to any one of claims 1 to 5, wherein the phytopathogenic fungi are soybean rust (*Phakopsora pachyrhizi* and/or *P*. *meibomiae*).

7. A method for combating phytopathogenic fungi containing an amino acid substitution F129L in the mitochondrial cytochrome b protein conferring resistance to Qo inhibitors, comprising treating curatively and/or preventively the plants or the plant propagation material of said plants that are at risk of being diseased from the said phytopathogenic fungi with an effective amount of at least one compound of formula I as defined in any of claims 1 to 5 or a composition comprising it; and/or a non-therapeutic method for combating phytopathogenic fungi containing an amino acid substitution F129L in the mitochondrial cytochrome b protein conferring resistance to Qo inhibitors, comprising applying to the said phytopathogenic fungi an effective amount of at least one compound of formula I as defined in any of claims 1 to 5 or a composition comprising it.

8. Compounds of formula I 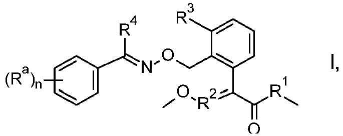 wherein
R¹ is selected from O and NH;
R² is selected from CH and N;
R³ is selected from C₁-C₄-alkyl, C₂-C₄-alkenyl, C₁-C₂-monohaloalkyl, C₁-C₂-dihaloalkyl, monohalo-ethenyl, dihalo-ethenyl, C₃-C₆-cycloalkyl and -O-C₁-C₄-alkyl;
R⁴ is selected from C₁-C₄-alkyl, C₂-C₄-alkenyl, C₁-C₄-haloalkyl, C₂-C₄-haloalkenyl, -(C₁-C₂-alkyl)-O-(C₁-C₂-alkyl) and -(C₁-C₂-alkyl)-O-(C₁-C₂-haloalkyl);
R^{a} is selected from CN, NH-C₁-C₄-alkyl, N(C₁-C₄-alkyl)₂, C₁-C₄-alkyl, C₂-C₄-alkenyl, C₂-C₄-alkynyl, -O-C₁-C₄-alkyl, -C(=O)-C₁-C₄-alkyl,-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S,
wherein said phenyl, heterocycloalkyl, heterocycloalkenyl and heteroaryl are bound directly or via an oxygen atom or via a C₁-C₂-alkylene linker,
and wherein the aliphatic and cyclic moieties of R^{a} are unsubstituted or carry 1, 2, 3, 4 or up to the maximum number of identical or different groups R^{b}:
R^{b} is selected from CN, NH₂, NO₂, C₁-C₄-alkyl and -O-C₁-C₄-alkyl;
n is an integer selected from 0, 1, 2, 3, 4 and 5;
and in form or stereoisomers and tautomers thereof, and the N-oxides and the agriculturally acceptable salts thereof.

9. The compounds according to claim 8, wherein R¹ is selected from O and NH; and R² is selected from CH and N, provided that R² is N in case R¹ is NH.

10. The compound according to any one of the claims 8 to 9, wherein R³ is selected from C₁-C₂-alkyl, C₁-C₂-monohaloalkyl, C₁-C₂-dihaloalkyl, C₃-C₄-cycloalkyl and -O-C₁-C₂-alkyl.

11. The compound according to any one of the claims 8 to 10, wherein R⁴ is selected from C₁-C₄-alkyl, C₁-C₄-haloalkyl and -(C₁-C₂-alkyl)-O-(C₁-C₂-alkyl).

12. The compounds according to any one of the claims 8 to 11, wherein n is 1, 2 or 3.

13. The compounds according to any one of the claims 8 to 12, wherein R^{a} is selected from CN, NH-C₁-C₂-alkyl, N(C₁-C₂-alkyl)₂, C₁-C₄-alkyl, C₂-C₄-alkenyl, C₂-C₄-alkynyl, -O-C₁-C₄-alkyl, -C(=O)-C₁-C₂-alkyl, -C=(N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S, wherein said phenyl, heterocycloalkyl and heteroaryl are bound directly or via an oxygen atom or via a methylene linker, and wherein said phenyl is unsubstituted or carries 1, 2 or 3 identical or different groups R^{b} selected from CN, NH₂, NO₂, C₁-C₄-alkyl and -O-C₁-C₄-alkyl.

14. Agrochemical compositions comprising an auxiliary and at least one compound of formula I, as defined in any of claims 8 to 13 or in the form of a stereoisomer or an agriculturally acceptable salt or a tautomer or N-oxide thereof.

15. A method for combating phytopathogenic fungi comprising treating curatively and/or preventively the plants or the plant propagation material of said plants that are at risk of being diseased from the said phytopathogenic fungi with at least one compound of formula I as defined in any of the claims 8 to 13 or an agrochemical composition as defined in claim 14; and/or a non-therapeutic method for combating phytopathogenic fungi comprising applying to the said phytopathogenic fungi at least one compound of formula I as defined in any of the claims 8 to 13 or an agrochemical composition as defined in claim 14.

## Patentansprüche

1. Nichttherapeutische Verwendung von Verbindungen der Formel I 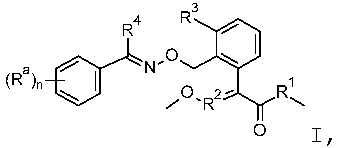 wobei
R¹ aus O und NH ausgewählt ist;
R² aus CH und N ausgewählt ist;
R³ aus Halogen, C₁-C₄-Alkyl, C₂-C₄-Alkenyl, C₁-C₂-Monohalogenalkyl, C₁-C₂-Dihalogenalkyl, Monohalogenethenyl, Dihalogenethenyl, C₃-C₆-Cycloalkyl und -O-C₁-C₄-Alkyl ausgewählt ist;
R⁴ aus C₁-C₄-Alkyl, C₂-C₄-Alkenyl, -C(=O)-C₁-C₂-Alkyl, C₁-C₄-Halogenalkyl, C₂-C₄-Halogenalkenyl, -(C₁-C₂-Alkyl)-O-(C₁-C₂-alkyl) und -CH₂-Cyclopropyl ausgewählt ist;
R^{a} aus Halogen, CN, -NR⁵R⁶, C₁-C₄-Alkyl, C₂-C₄-Alkenyl, C₂-C₄-Alkinyl, -O-C₁-C₄-Alkyl, -C(=N-O-C₁-C₄-Alkyl)-C₁-C₄-alkyl, -C(=O)-C₁-C₄-Alkyl, -O-CH₂-C(=N-O-C₁-C₄-Alkyl)-C₁-C₄-alkyl, C₃-C₆-Cycloalkyl, C₃-C₆-Cycloalkenyl, -C₁-C₂-Alkyl-C₃-C₆-cycloalkyl, -O-C₃-C₆-Cycloalkyl, Phenyl, 3-bis 6-gliedrigem Heterocycloalkyl, 3- bis 6-gliedrigem Heterocycloalkenyl und 5- oder 6-gliedrigem Heteroaryl ausgewählt ist;
wobei das Heterocycloalkyl, Heterocycloalkenyl und Heteroaryl neben Kohlenstoffatomen 1, 2 oder 3 Heteroatome, die aus N, O und S ausgewählt sind, enthalten,
wobei das Phenyl, Heterocycloalkyl, Heterocycloalkenyl und Heteroaryl direkt oder über ein Sauerstoffatom oder über einen C₁-C₂-Alkylen-Linker gebunden sind,
und wobei die aliphatischen und cyclischen Gruppierungen von R^{a} unsubstituiert sind oder 1, 2, 3, 4 oder bis zur maximalen Anzahl gleiche oder verschiedene Gruppen R^{b} tragen:
R^{b} aus Halogen, CN, NH₂, NO₂, C₁-C₄-Alkyl, C₁-C₄-Halogenalkyl, -O-C₁-C₄-Alkyl und -O-C₁-C₄-Halogenalkyl ausgewählt ist;
R⁵, unabhängig voneinander aus der Gruppe bestehend aus H, C₁-C₆-Alkyl, C₁-C₆-Halogenalkyl und C₂-C₄-Alkinyl ausgewählt sind;
n für eine ganze Zahl steht, die aus 0, 1, 2, 3, 4 und 5 ausgewählt ist;
und in Form von Stereoisomeren und Tautomeren davon und den N-Oxiden und den landwirtschaftlich unbedenklichen Salzen davon zur Bekämpfung phytopathogener Pilze, die eine Aminosäuresubstitution F129L im mitochondrialen Cytochrom-b-Protein enthalten, die Resistenz gegen Qo-Inhibitoren verleiht.

2. Verwendung nach Anspruch 1, wobei in Formel I R¹ für O steht und R² für CH steht.

3. Verwendung nach Anspruch 1 oder Anspruch 2, wobei in Formel I R³ aus C₁-C₂-Alkyl, C₁-C₂-Monohalogenalkyl, C₁-C₂-Dihalogenalkyl, C₃-C₄-Cycloalkyl und -O-C₁-C₂-Alkyl ausgewählt ist.

4. Verwendung nach einem der Ansprüche 1 bis 3, wobei in Formel I R⁴ aus C₁-C₄-Alkyl, -C(=O)-C₁-C₂-Alkyl, C₁-C₄-Halogenalkyl und -(C₁-C₂-Alkyl)-O-(C₁-C₂-alkyl) ausgewählt ist.

5. Verwendung nach einem der Ansprüche 1 bis 4, wobei in Formel I R^{a} aus C₁-C₃-Alkyl, C₂-C₃-Alkenyl, C₂-C₃-Alkinyl, -O-C₁-C₃-Alkyl, -C(=N-O-C₁-C₂-Alkyl)-C₁-C₂-alkyl, -O-CH₂-C(=N-O-C₁-C₂-Alkyl)-C₁-C₂-alkyl, C₃-C₄-Cycloalkyl, -C₁-C₂-Alkyl-C₃-C₄-cycloalkyl, -O-C₃-C₄-Cycloalkyl, Phenyl, 3- bis 5-gliedrigem Heterocycloalkyl und 5- oder 6-gliedrigem Heteroaryl ausgewählt ist, wobei das Heterocycloalkyl und das Heteroaryl neben Kohlenstoffatomen 1 oder 2 Heteroatome, die aus N, O und S ausgewählt sind, enthalten, wobei das Phenyl und Heteroaryl direkt oder über ein Sauerstoffatom oder über einen Methylen-Linker gebunden sind, und wobei die aliphatischen und cyclischen Gruppierungen von R^{a} unsubstituiert sind oder 1, 2 oder 3 gleiche oder verschiedene Gruppen R^{b}, die unabhängig voneinander aus Halogen, CN, Methyl und C₁-Halogenalkyl ausgewählt sind, tragen.

6. Verwendung nach einem der Ansprüche 1 bis 5, wobei es sich bei den phytopathogenen Pilzen um Sojabohnenrost (*Phakopsora pachyrhizi* und/oder *P*. *meibomiae*) handelt.

7. Verfahren zur Bekämpfung phytopathogener Pilze, die eine Aminosäuresubstitution F129L im mitochondrialen Cytochrom-b-Protein enthalten, die Resistenz gegen Qo-Inhibitoren verleiht, umfassend das kumulative oder präventive Behandeln der Pflanzen oder des Pflanzenfortpflanzungsmaterials der Pflanzen, bei denen ein Risiko einer Erkrankung durch die phytopathogenen Pilze besteht, mit einer wirksamen Menge mindestens einer Verbindung der Formel I gemäß einem der Ansprüche 1 bis 5 oder einer diese umfassenden Zusammensetzung; und/oder nichttherapeutisches Verfahren zur Bekämpfung phytopathogener Pilze, die eine Aminosäuresubstitution F129L im mitochondrialen Cytochrom-b-Protein enthalten, die Resistenz gegen Qo-Inhibitoren verleiht, umfassend das Aufbringen einer wirksamen Menge mindestens einer Verbindung der Formel I gemäß einem der Ansprüche 1 bis 5 oder einer dieser umfassenden Zusammensetzung auf die phytopathogenen Pilze.

8. Verbindungen der Formel I 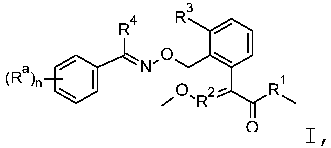 wobei
R¹ aus O und NH ausgewählt ist;
R² aus CH und N ausgewählt ist;
R³ aus C₁-C₄-Alkyl, C₂-C₄-Alkenyl, C₁-C₂-Monohalogenalkyl, C₁-C₂-Dihalogenalkyl, Monohalogenethenyl, Dihalogenethenyl, C₃-C₆-Cycloalkyl und -O-C₁-C₄-Alkyl ausgewählt ist;
R⁴ aus C₁-C₄-Alkyl, C₂-C₄-Alkenyl, C₁-C₄-Halogenalkyl, C₂-C₄-Halogenalkenyl, -(C₁-C₂-Alkyl)-O-(C₁-C₂-alkyl) und - (C₁-C₂-Alkyl)-O-(C₁-C₂-halogenalkyl) ausgewählt ist;
R^{a} aus CN, NH-C₁-C₄-Alkyl, N(C₁-C₄-Alkyl)₂, C₁-C₄-Alkyl, C₂-C₄-Alkenyl, C₂-C₄-Alkinyl, -O-C₁-C₄-Alkyl, -C(=O)-C₁-C₄-Alkyl, -C(=N-O-C₁-C₄-Alkyl)-C₁-C₄-alkyl, C₃-C₆-Cycloalkyl, C₃-C₆-Cycloalkenyl, -C₁-C₂-Alkyl-C₃-C₆-cycloalkyl, -O-C₃-C₆-Cycloalkyl, Phenyl, 3- bis 6-gliedrigem Heterocycloalkyl, 3- 6-gliedrigem Heterocycloalkenyl und 5- oder 6-gliedrigem Heteroaryl ausgewählt ist,
wobei das Heterocycloalkyl, Heterocycloalkenyl und Heteroaryl neben Kohlenstoffatomen 1, 2 oder 3 Heteroatome, die aus N, O und S ausgewählt sind, enthalten,
wobei das Phenyl, Heterocycloalkyl, Heterocycloalkenyl und Heteroaryl direkt oder über ein Sauerstoffatom oder über einen C₁-C₂-Alkylen-Linker gebunden sind,
und wobei die aliphatischen und cyclischen Gruppierungen von R^{a} unsubstituiert sind oder 1, 2, 3, 4 oder bis zur maximalen Anzahl gleiche oder verschiedene Gruppen R^{b} tragen:
R^{b} aus CN, NH₂, NO₂, C₁-C₄-Alkyl und -O-C₁-C₄-Alkyl ausgewählt ist;
n für eine ganze Zahl steht, die aus 0, 1, 2, 3, 4 und 5 ausgewählt ist;
und in Form von Stereoisomeren und Tautomeren davon, und die N-Oxide und die landwirtschaftlich unbedenklichen Salze davon.

9. Verbindungen nach Anspruch 8, wobei R¹ aus O und NH ausgewählt ist und R² aus CH und N ausgewählt ist, mit der Maßgabe, dass R² für N steht, wenn R¹ für NH steht.

10. Verbindung nach einem der Ansprüche 8 bis 9, wobei R³ aus C₁-C₂-Alkyl, C₁-C₂-Monohalogenalkyl, C₁-C₂-Dihalogenalkyl, C₃-C₄-Cycloalkyl und -O-C₁-C₂-Alkyl ausgewählt ist.

11. Verbindung nach einem der Ansprüche 8 bis 10, wobei R⁴ aus C₁-C₄-Alkyl, C₁-C₄-Halogenalkyl und - (C₁-C₂-Alkyl) - O- (C₁-C₂-alkyl) ausgewählt ist.

12. Verbindungen nach einem der Ansprüche 8 bis 11, wobei n für 1, 2 oder 3 steht.

13. Verbindungen nach einem der Ansprüche 8 bis 12, wobei R^{a} aus CN, NH-C₁-C₂-Alkyl, N(C₁-C₂-Alkyl)₂, C₁-C₄-Alkyl, C₂-C₄-Alkenyl, C₂-C₄-Alkinyl, -O-C₁-C₄-Alkyl, - C(=O)-C₁-C₂-Alkyl, -C=(N-O-CH₃)-CH₃, C₃-C₄-Cycloalkyl, -O-C₃-C₄-Cycloalkyl, Phenyl, 3- bis 5-gliedrigem Heterocycloalkyl und 5- oder 6-gliedrigem Heteroaryl ausgewählt ist,
wobei das Heterocycloalkyl und Heteroaryl neben Kohlenstoffatomen 1 oder 2 Heteroatome, die aus N, O und S ausgewählt sind, enthalten, wobei das Phenyl, Heterocycloalkyl und Heteroaryl direkt oder über ein Sauerstoffatom oder über einen Methylen-Linker gebunden sind und wobei das Phenyl unsubstituiert ist oder 1, 2 oder 3 gleiche oder verschiedene Gruppen R^{b}, die aus CN, NH₂, NO₂, C₁-C₄-Alkyl und -O-C₁-C₄-Alkyl ausgewählt sind, trägt.

14. Agrochemische Zusammensetzungen, umfassend einen Hilfsstoff und mindestens eine Verbindung der Formel I gemäß einem der Ansprüche 8 bis 13 oder in Form eines Stereoisomers davon oder ein landwirtschaftlich unbedenkliches Salz oder ein Tautomer oder N-Oxid davon.

15. Verfahren zur Bekämpfung phytopathogener Pilze, umfassend das kumulative oder präventive Behandeln der Pflanzen oder des Pflanzenfortpflanzungsmaterials der Pflanzen, bei denen ein Risiko einer Erkrankung durch die phytopathogenen Pilze besteht, mit mindestens einer Verbindung der Formel I gemäß einem der Ansprüche 8 bis 13 oder einer agrochemischen Zusammensetzung gemäß Anspruch 14; und/oder nichttherapeutisches Verfahren zur Bekämpfung phytopathogener Pilze, umfassend das Aufbringen mindestens einer Verbindung der Formel I gemäß einem der Ansprüche 8 bis 13 oder einer agrochemischen Zusammensetzung gemäß Anspruch 14 auf die phytopathogenen Pilze.

## Revendications

1. Utilisation non thérapeutique de composés de formule I 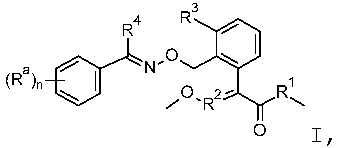
R¹ étant choisi entre O et NH ;
R² étant choisi entre CH et N ;
R³ étant choisi parmi halogène, alkyle en C₁-C₄, alcényle en C₂-C₄, monohalogénoalkyle en C₁-C₂, dihalogénoalkyle en C₁-C₂, monohalogénoéthényle, dihalogénoéthényle, cycloalkyle en C₃-C₆ et -O-alkyle en C₁-C₄ ;
R⁴ étant choisi parmi alkyle en C₁-C₄, alcényle en C₂-C₄, -C(=O)-alkyle en C₁-C₂, halogénoalkyle en C₁-C₄, halogénoalcényle en C₂-C₄, -(alkyl en C₁-C₂)-O-(alkyle en C₁-C₂) et -CH₂-cyclopropyle ;
R^{a} étant choisi parmi halogène, CN, -NR⁵R⁶, alkyle en C₁-C₄, alcényle en C₂-C₄, alcynyle en C₂-C₄, -O-alkyle en C₁-C₄, -C(=N-O-alkyl en C₁-C₄)-alkyle en C₁-C₄, -C (=O) - alkyle en C₁-C₄, -O-CH₂-C(=N-O-alkyl en C₁-C₄)-alkyle en C₁-C₄, cycloalkyle en C₃-C₆, cycloalcényle en C₃-C₆, -alkyl en C₁-C₂-cycloalkyle en C₃-C₆, -O-cycloalkyle en C₃-C₆, phényle, hétérocycloalkyle à 3 à 6 chaînons, hétérocycloalcényle à 3 à 6 chaînons et hétéroaryle à 5 ou 6 chaînons,
lesdits hétérocycloalkyle, hétérocycloalcényle et hétéroaryle contenant, outre des atomes de carbone, 1, 2 ou 3 hétéroatomes choisis parmi N, O et S,
lesdits phényle, hétérocycloalkyle, hétérocycloalcényle et hétéroaryle étant liés directement ou via un atome d'oxygène ou via un lieur alkylène en C₁-C₂
et les fractions aliphatiques et cycliques de R^{a} étant non substituées ou portant 1, 2, 3, 4 ou jusqu'au nombre maximal de groupes R^{b} identiques ou différents :
R^{b} étant choisi parmi halogène, CN, NH₂, NO₂, alkyle en C₁-C₄, halogénoalkyle en C₁-C₄, -O-alkyle en C₁-C₄ et -O-halogénoalkyle en C₁-C₄ ;
R⁵, étant indépendamment l'un de l'autre choisis dans le groupe constitué par H, alkyle en C₁-C₆, halogénoalkyle en C₁-C₆ et alcynyle en C₂-C₄ ;
n étant un entier choisi parmi 0, 1, 2, 3, 4 et 5 ;
et sous forme de stéréoisomères et tautomères de ceux-ci et des N-oxydes et des sels acceptables sur le plan agricole de ceux-ci, pour la lutte contre des champignons phytopathogènes contenant une substitution d'acide aminé F129L dans la protéine mitochondriale cytochrome b conférant une résistance aux inhibiteurs de Qo.

2. Utilisation selon la revendication 1, R¹ dans la formule I étant O ; et R² étant CH.

3. Utilisation selon la revendication 1 ou la revendication 2, R³ dans la formule I étant choisi parmi alkyle en C₁-C₂, monohalogénoalkyle en C₁-C₂, dihalogénoalkyle en C₁-C₂, cycloalkyle en C₃-C₄ et -O-alkyle en C₁-C₂.

4. Utilisation selon l'une quelconque des revendications 1 à 3, R⁴ dans la formule I étant choisi parmi alkyle en C₁-C₄, -C(=O)-alkyle en C₁-C₂, halogénoalkyle en C₁-C₄ et -(alkyl en C₁-C₂)-O-(alkyle en C₁-C₂).

5. Utilisation selon l'une quelconque des revendications 1 à 4, R^{a} dans la formule I étant choisi parmi alkyle en C₁-C₃, alcényle en C₂-C₃, alcynyle en C₂-C₃, -O-alkyle en C₁-C₃, -C(=N-O-alkyl en C₁-C₂)-alkyle en C₁-C₂, -O-CH₂-C(=N-O-alkyl en C₁-C₂)-alkyle en C₁-C₂, cycloalkyle en C₃-C₄, -(alkyl en C₁-C₂)-cycloalkyle en C₃-C₄, -O-cycloalkyle en C₃-C₄, phényle, hétérocycloalkyle à 3 à 5 chaînons et hétéroaryle à 5 ou 6 chaînons, lesdits hétérocycloalkyle et hétéroaryle contenant, outre des atomes de carbone, 1 ou 2 hétéroatomes choisis parmi N, O et S, lesdits phényle et hétéroaryle étant liés directement ou via un atome d'oxygène ou via un lieur méthylène et les fractions aliphatiques et cycliques de R^{a} étant non substituées ou portant 1, 2 ou 3 groupes R^{b} identiques ou différents qui, indépendamment les uns des autres, sont choisis parmi halogène, CN, méthyle et halogénoalkyle en C₁.

6. Utilisation selon l'une quelconque des revendications 1 à 5, les champignons phytopathogènes étant la rouille du soja *(Phakopsora pachyrhizi* et/ou *P*. *meibomiae*)*.*

7. Procédé de lutte contre des champignons phytopathogènes contenant une substitution d'acide aminé F129L dans la protéine mitochondriale cytochrome b conférant une résistance aux inhibiteurs de Qo, comprenant le traitement curatif et/ou préventif des plantes ou du matériel de propagation de plante desdites plantes qui présentent un risque d'être infectées par lesdits champignons phytopathogènes avec une quantité efficace d'au moins un composé de formule I tel que défini dans l'une quelconque des revendications 1 à 5 ou d'une composition le comprenant ; et/ou procédé non thérapeutique de lutte contre des champignons phytopathogènes contenant une substitution d'acide aminé F129L dans la protéine mitochondriale cytochrome b conférant une résistance aux inhibiteurs de Qo, comprenant l'application sur lesdits champignons phytopathogènes d'une quantité efficace d'au moins un composé de formule I tel que défini dans l'une quelconque des revendications 1 à 5 ou d'une composition le comprenant.

8. Composés de formule I 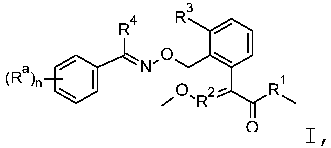
R¹ étant choisi entre O et NH ;
R² étant choisi entre CH et N ;
R³ étant choisi parmi alkyle en C₁-C₄, alcényle en C₂-C₄, monohalogénoalkyle en C₁-C₂, dihalogénoalkyle en C₁-C₂, monohalogénoéthényle, dihalogénoéthényle, cycloalkyle en C₃-C₆ et -O-alkyle en C₁-C₄ ;
R⁴ étant choisi parmi alkyle en C₁-C₄, alcényle en C₂-C₄, halogénoalkyle en C₁-C₄, halogénoalcényle en C₂-C₄, - (alkyl en C₁-C₂)-O-(alkyle en C₁-C₂) et -(alkyl en C₁-C₂)-O- (halogénoalkyle en C₁-C₂) ;
R^{a} étant choisi parmi CN, NH-alkyle en C₁-C₄, N(alkyle en C₁-C₄)₂, alkyle en C₁-C₄, alcényle en C₂-C₄, alcynyle en C₂-C₄, -O-alkyle en C₁-C₄, -C(=O)-alkyle en C₁-C₄, -C(=N-O-alkyl en C₁-C₄)-alkyle en C₁-C₄, cycloalkyle en C₃-C₆, cycloalcényle en C₃-C₆, -alkyl en C₁-C₂-cycloalkyle en C₃-C₆, -O-cycloalkyle en C₃-C₆, phényle, hétérocycloalkyle à 3 à 6 chaînons, hétérocycloalcényle à 3 à 6 chaînons et hétéroaryle à 5 ou 6 chaînons,
lesdits hétérocycloalkyle, hétérocycloalcényle et hétéroaryle contenant, outre des atomes de carbone, 1, 2 ou 3 hétéroatomes choisis parmi N, O et S,
lesdits phényle, hétérocycloalkyle, hétérocycloalcényle et hétéroaryle étant liés directement ou via un atome d'oxygène ou via un lieur alkylène en C₁-C₂
et les fractions aliphatiques et cycliques de R^{a} étant non substituées ou portant 1, 2, 3, 4 ou jusqu'au nombre maximal de groupes R^{b} identiques ou différents :
R^{b} étant choisi parmi CN, NH₂, NO₂, alkyle en C₁-C₄ et -O-alkyle en C₁-C₄ ;
n étant un entier choisi parmi 0, 1, 2, 3, 4 et 5 ;
et sous forme de stéréoisomères et tautomères de ceux-ci, et N-oxydes et sels acceptables sur le plan agricole de ceux-ci.

9. Composés selon la revendication 8, R¹ étant choisi entre O et NH ; et R² étant choisi entre CH et N, à condition que R² soit N dans le cas où R¹ est NH.

10. Composé selon l'une quelconque des revendications 8 à 9, R³ étant choisi parmi alkyle en C₁-C₂, monohalogénoalkyle en C₁-C₂, dihalogénoalkyle en C₁-C₂, cycloalkyle en C₃-C₄ et -O-alkyle en C₁-C₂.

11. Composé selon l'une quelconque des revendications 8 à 10, R⁴ étant choisi parmi alkyle en C₁-C₄, halogénoalkyle en C₁-C₄ et -(alkyl en C₁-C₂)-O-(alkyle en C₁-C₂).

12. Composés selon l'une quelconque des revendications 8 à 11, n étant 1, 2 ou 3.

13. Composés selon l'une quelconque des revendications 8 à 12, R^{a} étant choisi parmi CN, NH-alkyle en C₁-C₂, N(alkyle en C₁-C₂)₂, alkyle en C₁-C₄, alcényle en C₂-C₄, alcynyle en C₂-C₄, -O-alkyle en C₁-C₄, -C(=O)-alkyle en C₁-C₂, -C=(N-O-CH₃)-CH₃, cycloalkyle en C₃-C₄, -O-cycloalkyle en C₃-C₄, phényle, hétérocycloalkyle à 3 à 5 chaînons et hétéroaryle à 5 ou 6 chaînons,
lesdits hétérocycloalkyle et hétéroaryle contenant, outre des atomes de carbone, 1 ou 2 hétéroatomes choisis parmi N, O et S, lesdits phényle, hétérocycloalkyle et hétéroaryle étant liés directement ou via un atome d'oxygène ou via un lieur méthylène et ledit phényle étant non substitué ou portant 1, 2 ou 3 groupes R^{b} identiques ou différents choisis parmi CN, NH₂, NO₂, alkyle en C₁-C₄ et -O-alkyle en C₁-C₄.

14. Compositions agrochimiques comprenant un auxiliaire et au moins un composé de formule I, tel que défini dans l'une quelconque des revendications 8 à 13 ou sous la forme d'un stéréoisomère ou d'un sel acceptable sur le plan agricole ou d'un tautomère ou N-oxyde de celui-ci.

15. Procédé de lutte contre des champignons phytopathogènes comprenant le traitement curatif et/ou préventif des plantes ou du matériel de propagation de plante desdites plantes qui présentent un risque d'être infectées par lesdits champignons phytopathogènes avec au moins un composé de formule I tel que défini dans l'une quelconque des revendications 8 à 13 ou une composition agrochimique telle que définie dans la revendication 14 ; et/ou procédé non thérapeutique de lutte contre des champignons phytopathogènes comprenant l'application sur lesdits champignons phytopathogènes d'au moins un composé de formule I tel que défini dans l'une quelconque des revendications 8 à 13 ou d'une composition agrochimique telle que définie dans la revendication 14.
